(19) Europäisches Patentamt
European Patent Office
Office européen des brevets

(11) Veröffentlichungsnummer : **0 099 578**
**B1**

(12) # EUROPÄISCHE PATENTSCHRIFT

(45) Veröffentlichungstag der Patentschrift : **12.11.86**

(51) Int. Cl.⁴ : **C 07 H 15/00**, A 61 K 31/70// C07K1/00

(21) Anmeldenummer : **83107112.1**

(22) Anmeldetag : **20.07.83**

(54) **Neue Muramylpeptide und Verfahren zu ihrer Herstellung.**

(30) Priorität : **23.07.82 CH 4527/82**

(43) Veröffentlichungstag der Anmeldung : **01.02.84 Patentblatt 84/05**

(45) Bekanntmachung des Hinweises auf die Patenterteilung : **12.11.86 Patentblatt 86/46**

(84) Benannte Vertragsstaaten : **AT BE CH DE FR GB IT LI LU NL SE**

(56) Entgegenhaltungen :
**EP-A- 0 025 495**
**EP-A- 0 027 258**
**EP-A- 0 056 992**

(73) Patentinhaber : **CIBA-GEIGY AG**
**Klybeckstrasse 141**
**CH-4002 Basel (CH)**

(72) Erfinder : **Hartmann, Albert, Dr.**
**Steingasse 21A**
**D-7889 Grenzach (DE)**
Erfinder : **Wacker, Oskar, Dr.**
**Löwenbergstrasse 60**
**CH-4059 Basel (CH)**
Erfinder : **Baschang, Gerhard, Dr.**
**Bückenweg 7**
**CH-4126 Bettingen (CH)**
Erfinder : **Tarcsay, Lajos, Dr.**
**Muttenzerstrasse 25**
**D-7889 Grenzach-Wyhlen (DE)**

(74) Vertreter : **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 099 578 B1

Jouve, 18, rue St-Denis, 75001 Paris, France

**Beschreibung**

Die Erfindung betrifft Verbindungen der Formel I,

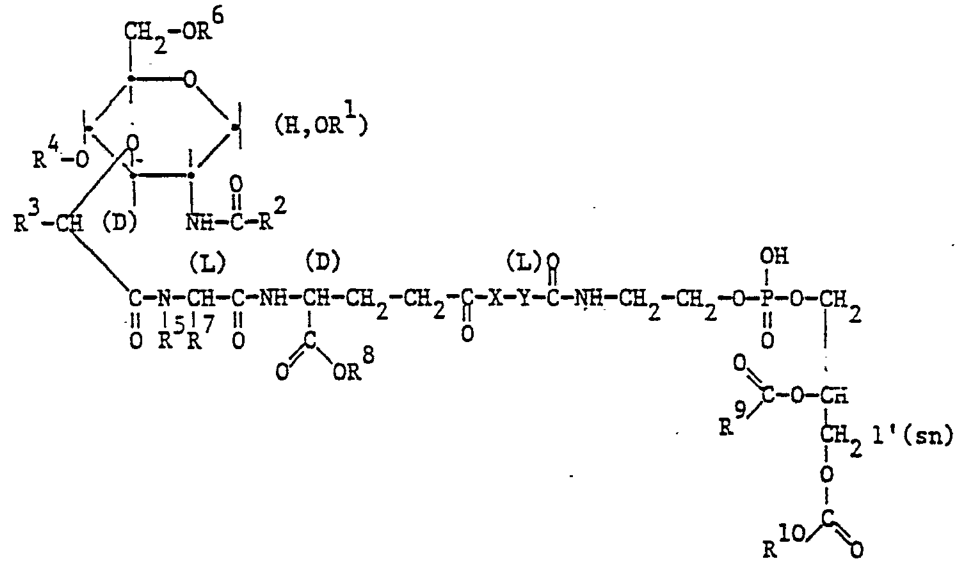

(I)

worin $R^1$, $R^4$ und $R^6$ unabhängig voneinander Wasserstoff oder Niederalkanoyl, $R^2$ $C_{1-4}$-Alkyl, Hydroxymethyl oder Phenyl, $R^3$ Wasserstoff oder Methyl, $R^5$ Wasserstoff oder $C_{1-3}$-Alkyl, $R^7$ unsubstituiertes oder durch Hydroxy, Mercapto oder Methylthio substituiertes $C_{1-3}$-Alkyl, $R^8$ Wasserstoff oder Niederalkyl, X Sauerstoff oder die Gruppe NH, Y $C_{1-4}$-Alkyliden und $R^9$ und $R^{10}$ unabhängig voneinander $C_{11-17}$-Alkyl oder $C_{11-17}$-Alkenyl bedeuten, die Salze dieser Verbindungen, Verfahren zu ihrer Herstellung, pharmazeutische Präparate, die diese Verbindungen enthalten, sowie die Verwendung der Verbindungen der Formel I und ihrer Salze.

Die Verbindungen der Formel I haben im Falle asymmetrischer Substitution am C—$R^3$ die (D)—, am C—$R^7$ die (L)—, am C—C—$OR^8$ die (D)—, im Rest Y die (L)— und am C—1 des D-Glucoserestes die α- und/oder β-Konfiguration, d. h. die Verbindungen der Formel I können als reine Anomere oder als Anomerengemische vorliegen.

Zur Kennzeichnung der Konfiguration am mittleren C-Atom des Glycerinteils wird den IUPAC Regeln entsprechend die stereospezifische Numerierung der C-Atome des Glycerinteils, gekennzeichnet durch das Präfix « sn », verwendet. Das oberste C-Atom in der Fischer Projektion der vertikalen Kohlenstoffkette, wobei die Hydroxygruppe am C—2 nach links steht, erhält die Nummer 1. Bei den erfindungsgemässen Verbindungen ist die Phosphorsäuregruppe an das C-Atom 3 im Sinne der stereospezifischen Numerierung gebunden.

Das vor- und nachstehend verwendete Präfix « Nieder » bezeichnet Reste von und mit 1 bis und mit 7, insbesondere mit 1-4, Kohlenstoffatomen.

Niederalkanoyl ist z. B. Propionyl, Butyryl oder Hexanoyl, in erster Linie Acetyl.

Niederalkyl ist z. B. n-Propyl, n-Butyl, Isobutyl, sek.Nutyl, tert.Butyl, ferner n-Pentyl, n-Hexyl oder n-Heptyl, in erster Linie Methyl, Ethyl oder Isopropyl.

Im einzelnen ist ein Alkylrest $R^2$ insbesondere Methyl, wenn $R^3$ für Methyl steht, oder Ethyl, wenn $R^3$ für Wasserstoff steht, ein Alkylrest $R^5$ insbesondere Methyl, ein Alkylrest $R^7$ insbesondere Methyl, Ethyl oder Isopropyl und ein Alkylrest $R^8$ insbesondere $C_{1-4}$-Alkyl, vornehmlich geradkettiges $C_{1-4}$-Alkyl, in allererster Linie n-Butyl.

Durch Hydroxy, Mercapto oder Methylthio substituiertes $C_{1-3}$-Alkyl $R^7$ ist insbesondere Hydroxymethyl oder 1-Hydroxy-ethyl, Mercaptomethyl bzw. 2-Methylthio-ethyl.

Ein Alkylidenrest Y ist ein bivalenter Rest, in dem beide Bindungen vom gleichen C-Atom ausgehen, vorzugsweise ein 1,1-verknüpfter Rest, wie insbesondere Methylen oder Ethyliden, ferner Propyliden oder Isobutyliden.

Ein $C_{11-17}$-Alkylrest $R^9$ oder $R^{10}$ ist ein geradkettiger oder verzweigter, insbesondere aber geradkettiger Rest, in erster Linie ein geradkettiger Rest mit ungerader Anzahl C-Atome, wie n-Undecyl oder n-Tridecyl, in allererster Linie ein geradkettiger $C_{15-17}$-Alkylrest mit ungerader Anzahl C-Atome, d. h. n-Heptadecyl oder in allererster Linie n-Pentadecyl.

Ein $C_{11-17}$-Alkenylrest $R^9$ oder $R^{10}$ ist insbesondere Hepta-8(Z)-decenyl.

Das über Sauerstoff an das Phosphoratom gebundene Proton ist sauer und kann leicht mit Basen abgespalten und durch ein anderes Kation ersetzt werden. Bei einem pH-Wert von 7 liegen die Verbindungen der Formel I daher vollständig oder zum überwiegenden Teil in Salzform vor. Diese Salze

sowie die Säure-Salz-Gemische gehören ebenfalls zum Gegenstand der Erfindung. Insbesondere betrifft die Erfindung pharmazeutisch verwendbare, nicht toxische Salze der Verbindungen der Formel I. Es sind dies in erster Linie Metall- oder Ammoniumsalze, wie Alkalimetall- und Erdalkalimetall-, z. B. Natrium-, Kalium-, Magnesium- oder Calciumsalze, sowie Ammoniumsalze oder Salze mit geeigneten organischen Aminen, wie Niederalkylaminen, z. B. Triethylamin. Zur Isolierung oder Reinigung können auch pharmazeutisch ungeeignete Salze Verwendung finden. Zur therapeutischen Anwendung gelangen jedoch nur die pharmazeutisch verwendbaren, nicht toxischen Salze, die deshalb bevorzugt werden.

Die erfindungsgemässen Verbindungen, worin $R^1$, $R^4$ und $R^6$ für Wasserstoff stehen, fallen unter den allgemeinen Rahmen der europäischen Patentanmeldung mit der Publikationsnummer 00 25 495 und können ebenso wie die Verbindungen der Formel I, worin $R^1$, $R^4$ und/oder $R^6$ für Niederalkanoyl stehen, als Immunostimulatoren verwendet werden. Gegenüber den in der o. a. Anmeldung vorpublizierten Verbindungen weisen die erfindungsgemässen Verbindungen stark reduzierte unerwünschte Nebenwirkungen auf, insbesondere sind sie viel weniger, d. h. praktisch nicht, pyrogen. Dieser Befund ist von grosser Bedeutung, da die Applikation einer pyrogenen Substanz unter Umständen einen thermischen Schock herbeiführen kann, weshalb eine sichere Anwendbarkeit nur unter ständiger ärztlicher Aufsicht gewährleistet ist und bestimmte Verabreichungsformen, wie die intravenöse Gabe, ausser Betracht bleiben müssen.

Die Prüfung auf Pyrogenität kann nach der in Europäische Pharmakopöe, Band 2, Seiten 56-59 (1971) gegebenen Vorschrift an Kaninchen durchgeführt werden. Hiernach ist z. B. selbst bei subkutaner Applikation einer hohen Dosis, wie 30 mg/kg von N-Acetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (Verbindung I) kein pyrogener Effekt feststellbar.

Demgegenüber ist die gleiche Dosis von N-Acetyl-muramyl-L-alanyl-D-isoglutaminyl-L-alanin-2-(1,2-dipalmitoyl-sn-glycero-3-hydroxyphosphoryloxy)-ethylamid-natriumsalz stark pyrogen.

Die erfindungsgemässen Verbindungen können verwendet werden, wie in der europäischen Patentanmeldung 00 25 495 beschrieben ist.

Aufgrund ihrer ausgezeichneten Verträglichkeit sind sie jedoch besonders zur Prophylaxe und Therapie von Infektionskrankheiten geeignet, wobei eine starke Wirkung nicht nur im Falle von bakteriellen Erregern, sondern überraschenderweise auch gegen virale Erreger eintritt. Der letzte Befund ist bisher nicht beschrieben und von grosser praktischer Bedeutung, da Pharmazeutika zur Heilung virusbedingter Krankheiten bisher nur in völlig unzureichendem Masse zur Verfügung stehen. Bemerkenswert ist vor allem die lange prophylaktische oder therapeutische Wirkungsdauer von vielen Tagen bis zu einigen Wochen nach einmaliger Applikation der erfindungsgemässen Verbindungen und das breite Spektrum viraler Erreger, gegen das die Verbindungen wirksam sind.

Die Muramylpeptide der Formel I können insbesondere zur Prophylaxe und Therapie von Krankheiten verwendet werden, die durch die nachstehend näher bezeichneten Viren hervorgerufen werden [zur Nomenklatur vgl. J. L. Melnick, Prog. med. Virol, *26*, 214-232 (1980) und *28*, 208-221 (1982)] :

DNA-Viren mit kubischer Symmetrie und nacktem Nukleokapsid, DNA-Viren mit umhüllten Virion sowie RNA-Viren mit kubischer und solche mit helikaler Symmetrie des Kapsids.

Bevorzugterweise verwendet man die Verbindungen der Formel I im Falle von DNA-Viren mit umhüllten Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z. B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, Rhinoviren, Cardioviren und Orthomyxoviridae, aber auch im Falle von Betaherpesvirinae, wie insbesondere menschlichen Cytomegaloviren, im Falle von Aphthoviren, in erster Linie Apthoviren von Paarhufern, wie hauptsächlich von Rindern, sowie im Falle von Paramyxoviridae, wie in erster Linie Pneumoviren, z. B. respiratorischen Syncitialviren des Menschen, und wie daneben Morbilliviren oder Paramyxoviren, wie z. B. menschlichen Parainfluenzaviren einschliesslich der Sendaiviren.

Im allererster Linie verwendet man die Verbindungen der Formel I im Falle von Simplexviren, z. B. menschlichen Herpes simplex Viren der Typen 1 und 2, im Falle von menschlichen Encephalomyocarditisviren und im Falle von Influenzaviren, wie hauptsächlich Influenza A und Influenza B Viren, z. B. den in den Beispielen genannten Viren.

Die Muramylpeptide der Formel I können zur Prophylaxe oder Therapie von Virusinfektionen verwendet werden, indem man sie enteral oder parenteral, in erster Linie zusammen mit geeigneten Hilfs- oder Trägerstoffen, appliziert. Bevorzugterweise appliziert man sie auf die Schleimhaut, z. B. intranasal, rektal, vaginal oder auf die Bindehaut des Auges, oder oral. Der antivirale Effekt tritt jedoch auch bei Applikation auf anderen Wegen, z. B. subkutan, intravenös, intramuskulär oder bei Applikation auf die

normale Haut ein.

Welche Applikationsart die geeignetste ist, hängt u. a. von der Art des jeweiligen Virus ab, z. B. ist im Falle respiratorischer Viren in vielen Fällen die intranasale Verabreichung zu bevorzugen. Die Dosierung des Wirkstoffs hängt u. a. von der Warmblüterspezies, der Abwehrlage des Organismus, der Applikationsweise und der Art des Virus ab.

Die erfindungsgemässen Verbindungen haben die weitere sehr interessante Eigenschaft, die Bildung von Metastasen bei einigen Tumoren, insbesondere der Lunge, zu hemmen, wie experimentell im B16-BL6-Melanom-Modell und im Fall von Lewis Lungenkarzinomen gezeigt werden kann, wobei die Applikation in Liposomen besonders vorteilhaft ist.

Die vorliegende Erfindung betrifft insbesondere Verbindungen der Formel I, worin mindestens einer der Reste $R^1$, $R^4$ und $R^6$ für Niederalkanoyl steht.

Hauptsächlich betrifft die Erfindung Verbindungen der Formel I, worin $R^1$, $R^4$ und $R^6$ unabhängig voneinander Wasserstoff oder $C_{1-3}$-Alkanoyl, $R^2$ $C_{1-2}$-Alkyl oder Hydroxymethyl, $R^3$ Wasserstoff oder Methyl, $R^5$ Wasserstoff oder Methyl, $R^7$ $C_{1-3}$-Alkyl oder Hydroxymethyl, $R^8$ Wasserstoff oder $C_{1-4}$-Alkyl, X Sauerstoff oder die Gruppe NH, Y $C_{1-3}$-Alkyliden und $R^9$ und $R^{10}$ unabhängig voneinander geradkettiges $C_{11-17}$-Alkyl oder Descarbonyl-oleoyl bedeuten, und ihre Salze.

In erster Linie betrifft die Erfindung Verbindungen der Formel I, worin $R^1$, $R^4$ und $R^6$ unabhängig voneinander Wasserstoff oder Acetyl, $R^2$ Methyl oder Ethyl, $R^3$ Wasserstoff oder Methyl, $R^5$ Wasserstoff oder Methyl, $R^7$ $C_{1-3}$-Alkyl, $R^8$ Wasserstoff oder $C_{1-4}$-Alkyl, X Sauerstoff oder die Gruppe NH, Y $C_{1-2}$-Alkyliden und $R^9$ und $R^{10}$ unabhängig voneinander geradkettiges $C_{11-17}$-Alkyl mit ungerader Anzahl C-Atome oder Descarbonyl-oleoyl bedeuten, und ihre Salze.

Besonders betrifft die Erfindung die obengenannten Verbindungen der Formel I, worin $R^8$ worin für einen der genannten Alkylreste steht, und/oder worin $R^1$, $R^4$ und $R^6$ für Wasserstoff stehen, und ihre Salze.

Vornehmlich betrifft die Erfindung Verbindungen der Formel I, worin $R^1$, $R^4$ und $R^6$ Wasserstoff, $R^2$ Methyl oder Ethyl, $R^3$ Wasserstoff, wenn $R^2$ für Ethyl steht, oder Methyl, wenn $R^2$ für Methyl steht, $R^5$ Wasserstoff oder Methyl, $R^7$ $C_{1-2}$-Alkyl, $R^8$ geradkettiges $C_{1-4}$-Alkyl, X Sauerstoff oder die Gruppe NH, Y Ethyliden und $R^9$ und $R^{10}$ unabhängig voneinander geradkettiges $C_{15-17}$-Alkyl mit ungerader Anzahl C-Atome bedeuten, und ihre Salze.

In allererster Linie betrifft die Erfindung die obengenannten Verbindungen der Formel I, worin $R^3$ Methyl, $R^5$ Wasserstoff, $R^8$ n-Butyl, X die Gruppe NH und/oder $R^9$ genau wie $R^{10}$ n-Pentadecyl bedeuten, und ihre Salze, sowie die in den Beispielen genannten Verbindungen der Formel I.

Die neuen Verbindungen der Formel I und ihre Salze können nach an sich bekannten Methoden erhalten werden.

So lassen sie sich erhalten, wenn man

a) eine Verbindung der Formel II,

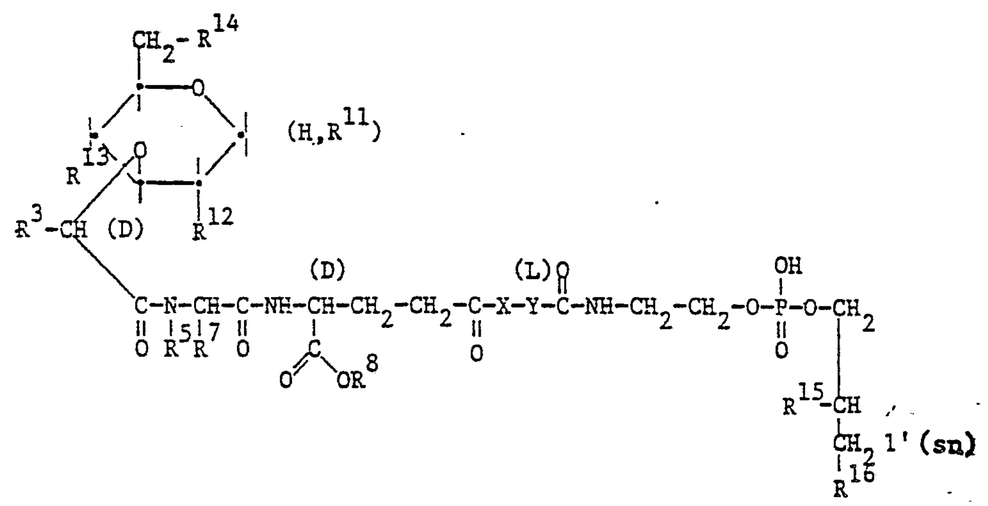

(II)

worin mindestens einer der Reste $R^{11}$, $R^{13}$, $R^{14}$, $R^{15}$ und $R^{16}$ für freies oder in reaktionsfähiger Form vorliegendes Hydroxy steht und/oder worin $R^{12}$ für freies oder in reaktionsfähiger Form vorliegendes Amino steht, und die übrigen Substituenten die obengenannten Bedeutungen haben, mit der Massgabe, dass in einer Verbindung der Formel II vorhandene funktionelle Gruppen mit Ausnahme der an der Reaktion teilnehmenden Gruppe(n) gegebenenfalls in geschützter Form vorliegen, mit einer Carbonsäure oder einem reaktionsfähigen Derivat davon unter Einführung von mindestens einem Acylrest $R^1$,

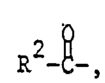

$R^4$, $R^6$,

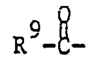

oder

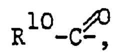

worin die Substituenten die obengenannten Bedeutungen haben, acyliert und gegebenenfalls vorhandene Schutzgruppen abspaltet, oder

b) eine Verbindung der Formel III,

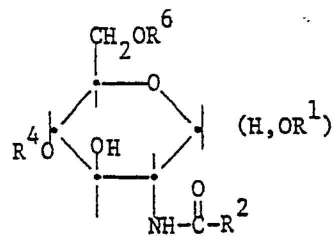

(III)

worin die Susbtituenten die obengenannten Bedeutungen haben und vorhandene funktionelle Gruppen mit Ausnahme der freien Hydroxygruppe in 3-Stellung gegebenenfalls in geschützter Form vorliegen, wobei die 3-Hydroxygruppe gegebenenfalls in reaktionsfähiger Form vorliegt, mit einer Verbindung der Formel IV,

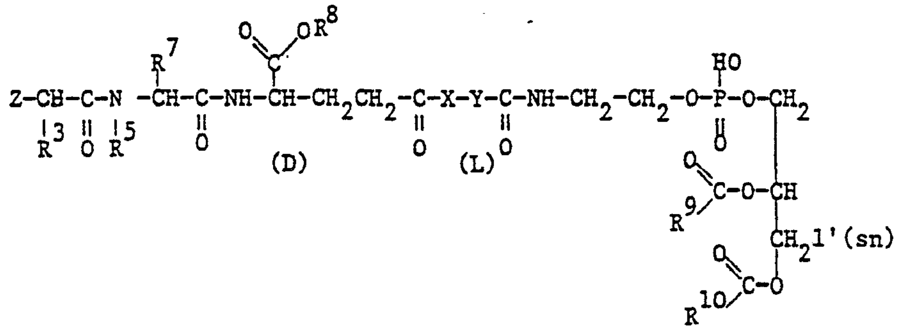

(IV)

worin Z eine gegebenenfalls in reaktionsfähiger Form vorliegende Hydroxygruppe bedeutet und die übrigen Substituenten die obengenannten Bedeutungen haben, mit der Massgabe, dass in einer Verbindung der Formel IV vorhandene funktionelle Gruppen mit Ausnahme von Z gegebenenfalls in geschützter Form vorliegen, umsetzt und vorhandene Schutzgruppen abspaltet, oder

c) eine Verbindung der Formel V,

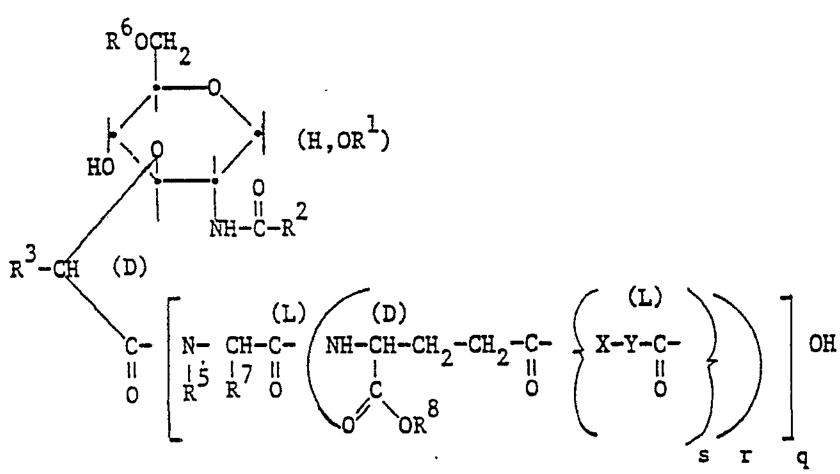

(V)

worin s, r und q unabhängig voneinander für 0 oder 1 stehen und die Substituenten die obengenannten Bedeutungen haben, mit der Massgabe, dass in einer Verbindung der Formel V vorhandene freie funktionelle Gruppen mit Ausnahme der an der Reaktion teilnehmenden Gruppe gegebenenfalls in geschützter Form vorliegen, oder ein reaktionsfähiges Carbonsäurederivat davon, mit einer Verbindung der Formel VI,

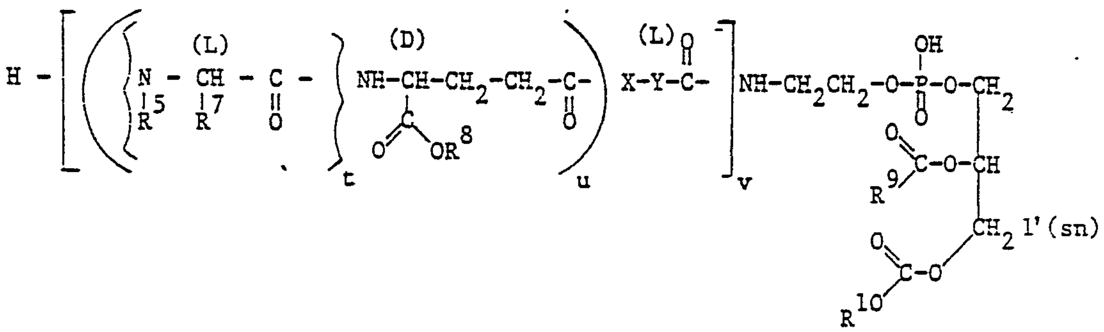

(VI)

worin t, u und v unabhängig voneinander für 0 oder 1 und $R^8$ für Niederalkyl oder eine Carboxylschutzgruppe stehen und die übrigen Substituenten die obengenannten Bedeutungen haben, mit der Massgabe, dass t, u und v für 1 stehen, wenn im Reaktionspartner der Formel V q für 0 steht, oder dass t für 0, u für 1 und v für 1 stehen, wenn q für 1 und r für 0 stehen, oder dass u für 0 und v für 1 stehen, wenn q für 1, r für 1 und s für 0 stehen, oder dass v für 0 steht, wenn q, r und s für 1 stehen, oder einem reaktionsfähigen Derivat davon umsetzt, und gegebenenfalls vorhandene Schutzgruppen abspaltet, oder

d) eine Verbindung der Formel VII,

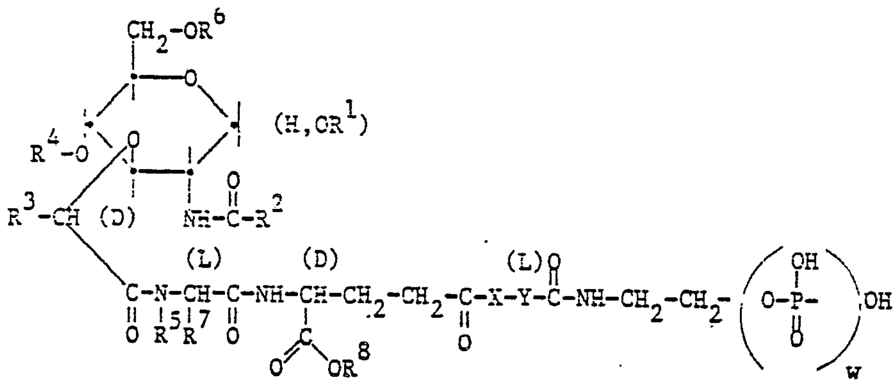

(VII)

worin w für 0 oder 1 steht und die Substituenten die obengenannten Bedeutungen haben, mit der Massgabe, dass freie funktionelle Gruppen mit Ausnahme der an der Reaktion teilnehmenden Gruppe gegebenenfalls in geschützter Form vorliegen, oder ein reaktionsfähiges Phosphorsäurederivat einer solchen Säure der Formel VII, worin w für 1 steht, mit einer Verbindung der Formel VIII

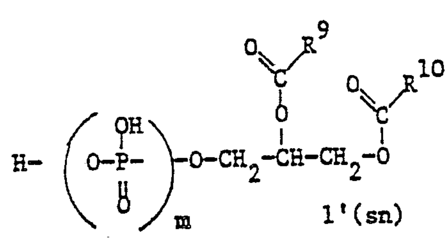

(VIII)

worin die Substituenten die obengenannten Bedeutungen haben und m für 0 oder 1 steht, mit der Massgabe, dass m für 1 steht, wenn im Reaktionspartner der Formel VII w für 0 steht, oder dass m für 0 steht, wenn w für 1 steht, oder mit einem reaktionsfähigen Phosphorsäurederivat einer Säure der Formel VIII, worin m für 1 steht, umsetzt, und vorhandene Schutzgruppen abspaltet, oder

e) das reaktionsfähige Derivat einer Verbindung der Formel VII,

(Siehe Formel VII Seite 7 f.)

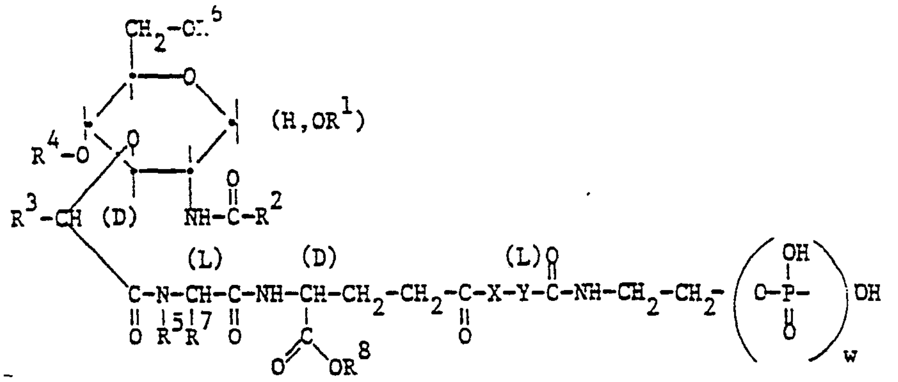

(VII)

worin w für 0 oder 1 steht und die Substituenten die obigen Bedeutungen haben, mit der Massgabe, dass in einer Verbindung der Formel VII vorhandene freie funktionelle Gruppen mit Ausnahme der an der Reaktion teilnehmenden Gruppe, gegebenenfalls durch leicht abspaltbare Schutzgruppen geschützt sind, sowie mit der Massgabe, dass das reaktionsfähige Derivat einer Verbindung der Formel VII, worin w für 0 steht, ein solches mit reaktionsfähiger, veresterter, endständiger Hydroxygruppe ist, und mit der Massgabe, dass das reaktionsfähige Derivat einer Verbindung der Formel VII, worin w für 1 steht, ein Salz ist, mit dem reaktionsfähigen Derivat einer Verbindung der Formel VIII,

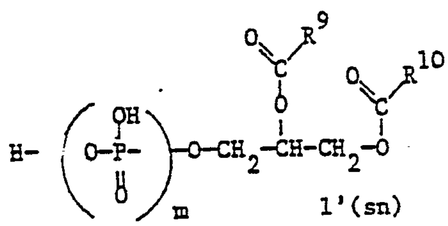

. (VIII)

worin die Substituenten die obengenannten Bedeutungen haben und m für 0 oder 1 steht, mit der Massgabe, dass m für 1 steht, wenn im Reaktionspartner der Formel VII w für 0 steht, wobei das reaktionsfähige Derivat einer Verbindung der Formel VIII ein Salz ist, oder dass m für 0 steht, wenn w für 1 steht, wobei das reaktionsfähige Derivat einer Verbindung der Formel VIII ein solches mit reaktionsfähiger, veresterter, endständiger Hydroxygruppe ist, umsetzt und vorhandene Schutzgruppen abspaltet, oder

f) eine Verbindung der Formel IX,

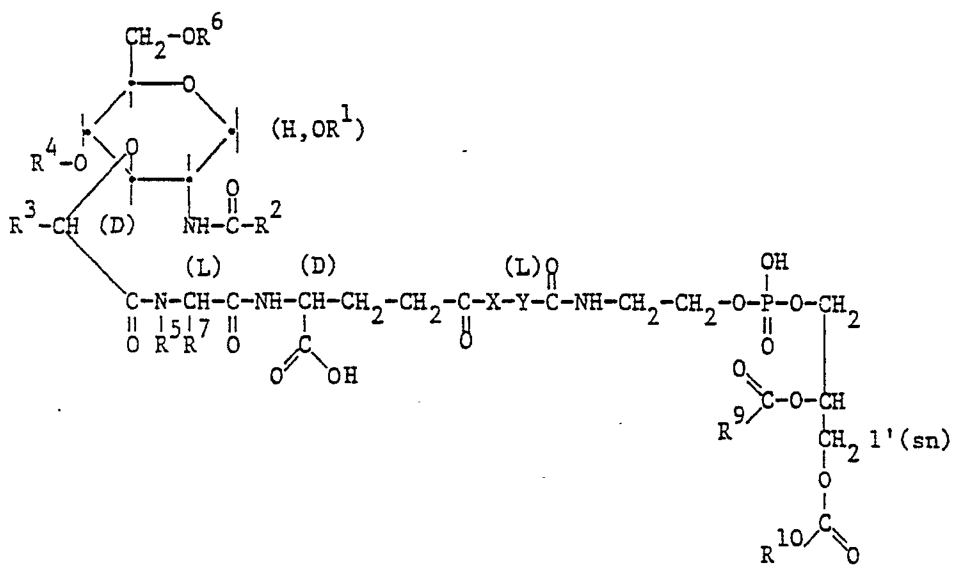

(IX)

worin die Substituenten die obigen Bedeutungen haben, wobei in einer Verbindung der Formel IX vorhandene freie funktionelle Gruppen mit Ausnahme der an der Reaktion teilnehmenden Gruppe gegebenenfalls in geschützter Form vorliegen, oder ein reaktionsfähiges Carbonsäurederivat einer Verbindung der Formel IX, mit einem Niederalkanol $R^8$—OH, worin $R^8$ Niederalkyl bedeutet, oder einem reaktionsfähigen Derivat davon umsetzt und vorhandene Schutzgruppen abspaltet, oder

g) eine Verbindung der Formel X,

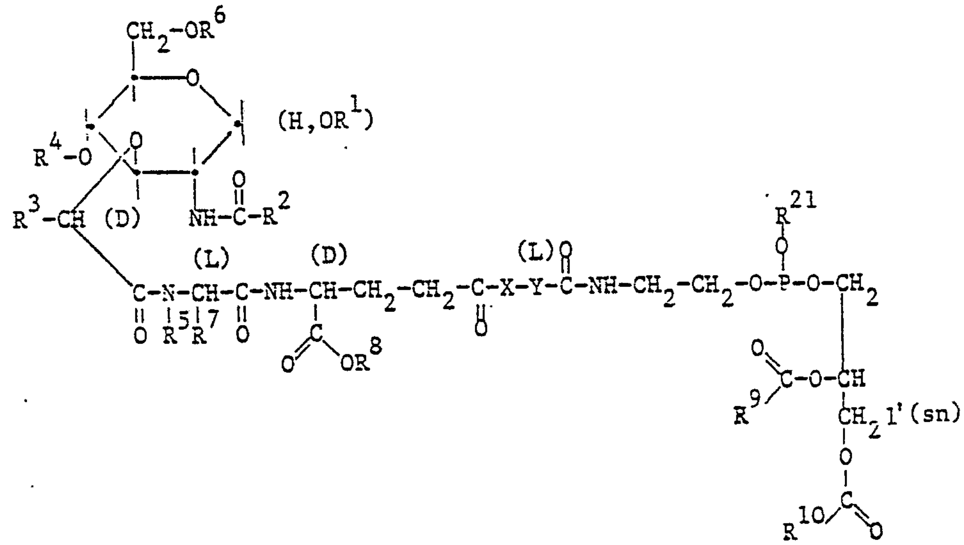

(X)

worin $R^{21}$ für Wasserstoff oder eine Schutzgruppe steht und die übrigen Substituenten die obengenannten Bedeutungen haben, wobei in einer Verbindung der Formel X vorhandene freie funktionelle Gruppen mit Ausnahme der an der Reaktion teilnehmenden Gruppe gegebenenfalls in geschützter Form vorliegen, oder ein Tautomeres dieser Verbindung mit einem Oxidationsmittel oxidiert und vorhandene Schutzgruppen abspaltet, oder

h) in einer Furanoseverbindung der Formel XI,

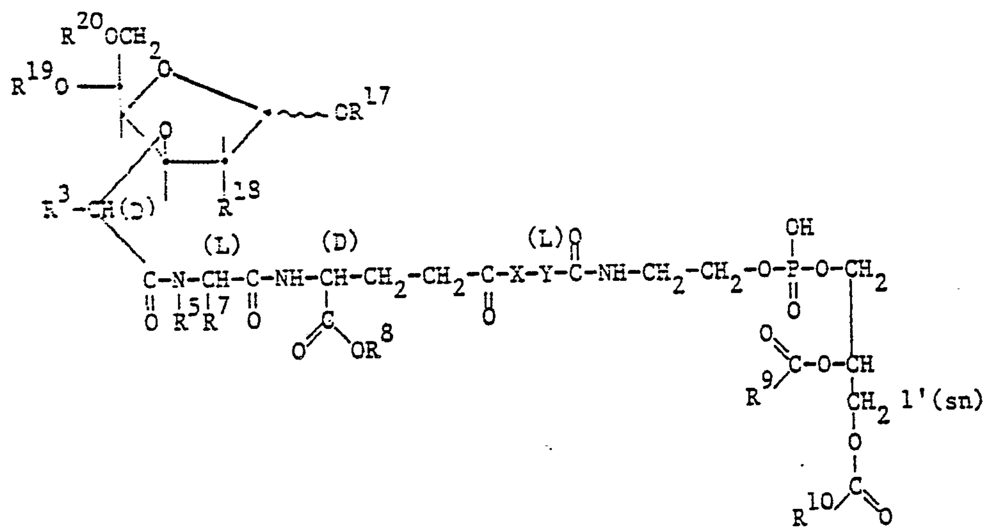

(XI)

worin $R^{17}$ Wasserstoff oder eine leicht abspaltbare Hydroxyschutzgruppe bedeutet und $R^{18}$ die oben für den Rest

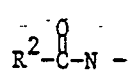

genannte Bedeutung hat, oder $R^{17}$ und $R^{18}$ zusammen eine mit der freien Valenz des C-Atoms an Sauerstoff gebundene, bivalente Schutzgruppe der Formel

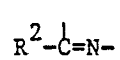

bedeuten, worin $R^2$ die obengenannte Bedeutung hat, und $R^{19}$ und $R^{20}$ Wasserstoff oder eine leicht abspaltbare Hydroxyschutzgruppe bedeuten, oder $R^{19}$ und $R^{20}$ zusammen für eine bivalente Hydroxyschutzgruppe stehen, und die übrigen Substituenten die obengenannten Bedeutungen haben, die Schutzgruppen abspaltet, oder

i) in einer Verbindung der Formel I, worin mindestens einer der Reste $R^1$, $R^2$, $R^4$, $R^6$, $R^7$ oder $R^8$ in geschützter Form vorliegt, die Schutzgruppe(n) abspaltet, oder

j) eine Verbindung der Formel XII,

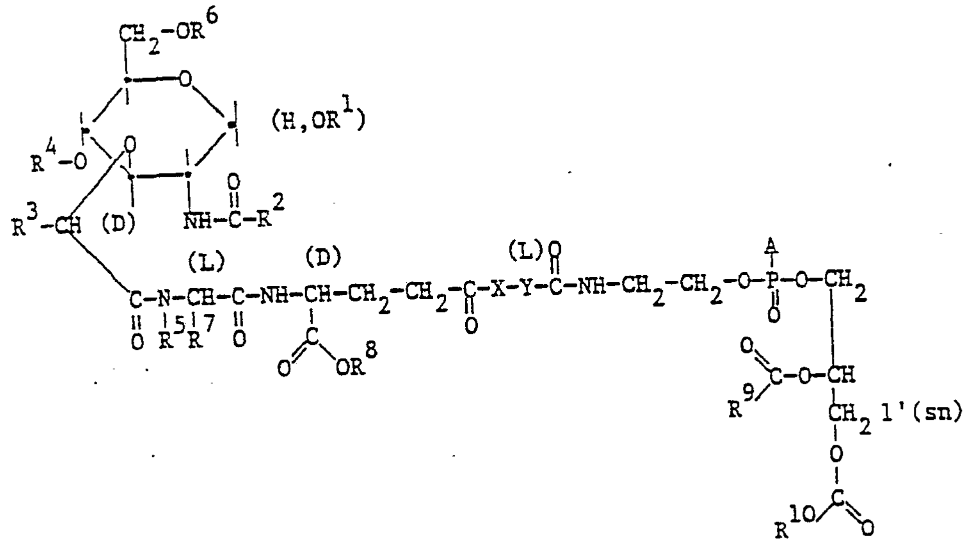

(XII)

worin A ein Halogenatom bedeutet und die übrigen Substituenten die obengenannten Bedeutungen haben, hydrolysiert, und, wenn erwünscht, nach Durchführung eines der Verfahren a-j) eine erhaltene Verbindung der Formel I in ihr Salz oder ein erhaltenes Salz in ein anderes Salz überführt.

Verfahren a (Acylierung)

In reaktionsfähiger Form vorliegendes Hydroxy $R^{11}$, $R^{13}$, $R^{14}$, $R^{15}$ oder $R^{16}$ ist insbesondere Metalloxy oder reaktionsfähiges verestertes Hydroxy.

Metalloxy ist in erster Linie Alkalimetall- oder Erdalkalimetalloxy, hauptsächlich Lithium-, Natrium- oder Kaliumoxy.

Reaktionsfähiges verestertes Hydroxy ist z. B. mit einer starken anorganischen oder organischen Säure verestertes Hydroxy, wie mit einer Mineralsäure, z. B. Halogenwasserstoffsäure, wie Chlorwasserstoff-, Bromwasserstoff- oder Jodwasserstoffsäure, ferner Schwefelsäure, oder Halogenschwefelsäure, z. B. Fluorschwefelsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trifluormethansulfon- oder p-Toluolsulfonsäure verestertes Hydroxy.

In reaktionsfähiger Form vorliegendes Amino ist z. B. durch Reaktion mit einem Phosphit, wie Diethylchlorphosphit, Ethylen-chlor-phosphit, 1,2-Phenylen-chlor-phosphit, Ethyl-dichlor-phosphit oder Tetraethylpyrophosphit aktiviertes Amino. Eine reaktionsfähige Aminogruppe ist z. B. auch ein Carbaminsäurehalogenid oder ein Isocyanat, wobei die Aminogruppe an Halogencarbonyl, z. B. Chlorcarbonyl, gebunden ist bzw. als Isocyanatgruppe vorliegt, wobei im letzteren Falle nur Verbindungen der Formel I zugänglich sind, die am Stickstoffatom der durch die Reaktion gebildeten Amidgruppe eine Wasserstoffatom tragen.

Das reaktionsfähige Derivat einer Carbonsäure ist z. B. ein Salz, z. B. ein Cäsiumsalz, wenn in dem Reaktionspartner der Formel II mindestens eine Hydroxygruppe in reaktionsfähiger veresterter Form vorliegt, oder ein reaktionsfähiger Carbonsäureester, ein reaktionsfähiges Carbonsäureanhydrid oder ein cyclisches Amid. Dabei können reaktionsfähige Säurederivate auch *in situ* gebildet werden.

Aktivierte Ester von Säuren sind insbesondere am Verknüpfungskohlenstoffatom des veresternden Restes ungesättigte Ester, z. B. vom Vinylester-Typ, wie eigentliche Vinylester (die man z. B. durch Umesterung eines entsprechenden Esters mit Vinylacetat erhalten kann ; Methode des aktivierten Vinylesters), Carbamoylvinylester (die man z. B. durch Behandeln der entsprechenden Säure mit einem Isoxazoliumreagens erhalten kann ; 1,2-Oxazolium- oder Woodward-Methode), oder 1-Niederalkoxyvinylester (die man z. B. durch Behandeln der entsprechenden Säure mit einem Niederalkoxyacetylen erhalten kann ; Ethoxyacetylen-Methode), oder Ester vom Amidinotyp, wie N,N'-disubstituierte Amidinoester (die man z. B. durch Behandeln der entsprechenden Säure mit einem geeigneten N,N'-disubstituierten Carbodiimid, z. B. N,N'-Dicyclohexylcarbodiimid, erhalten kann ; Carbodiimid-Methode), oder N,N-disubstituierte Amidinoester (die man z. B. durch Behandeln der entsprechenden Säure mit einem N,N-disubstituierten Cyanamid erhalten kann ; Cyanamid-Methode), geeignete Arylester, insbesondere durch Elektronenanziehende Substituenten geeignet substituierte Phenylester (die man z. B. durch Behandeln der entsprechenden Säure mit einem geeignet substituierten Phenol, z. B. 4-Nitrophenol, 4-Methylsulfonyl-phenol, 2,4,5-Trichlorphenol, 2,3,4,5,6-Pentachlor-phenol oder 4-Phenyldiazo-phenol, in

Gegenwart eines Kondensationsmittels, wie N,N'-Dicyclohexyl-carbodiimid, erhalten kann ; Methode der aktivierten Arylester), Cyanmethylester (die man z. B. durch Behandeln der entsprechenden Säure mit Chloracetonitril in Gegenwart einer Base erhalten kann ; Cyanmethylester-Methode), Thioester, insbesondere gegebenenfalls, z. B. durch Nitro, substituierte Phenyl-thioester (die man z. B. durch Behandeln der entsprechenden Säure mit gegebenenfalls, z. B. durch Nitro, substituierten Thiophenolen, u. a. mit Hilfe der Anhydrid- oder Carbodiimid-Methode, erhalten kann ; Methode der aktivierten Thiolester), oder Amino- oder Amidoester (die man z. B. durch Behandeln der entsprechenden Säure mit einer N-Hydroxyamino- bzw. N-Hydroxy-amido-Verbindung, z. B. N-Hydroxy-succinimid, N-Hydroxy-piperidin, N-Hydroxyphthalimid oder 1-Hydroxy-benztriazol, z. B. nach der Anhydrid- oder Carbodiimid-Methode, erhalten kann ; Methode der aktivierten N-Hydroxy-ester).

Reaktionsfähige Anhydride können symmetrische oder vorzugsweise gemischte Anhydride dieser Säuren sein, so z. B. Anhydride mit anorganischen Säuren, wie Säurehalogenide, insbesondere Säurechloride (die man z. B. durch Behandeln der entsprechenden Säure mit Thionylchlorid, Phosphorpentachlorid oder Oxalylchlorid erhalten kann ; Säurechloridmethode), Azide (die man z. B. aus einem entsprechenden Säureester über das entsprechende Hydrazid und dessen Behandlung mit salpetriger Säure erhalten kann ; Azidmethode), Anhydride mit Kohlensäurehalbderivaten, wie mit entsprechenden Estern, z. B. Kohlensäureniederalkylhalbestern (die man z. B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z. B. 1-Niederalkoxycarbonyl-2-ethoxy-1,2-dihydrochinolin, erhalten kann ; Methode der gemischten O-Alkyl-kohlensäureanhydride), oder Anhydride mit dihalogenierter, insbesondere dichlorierter Phosphorsäure (die man z. B. durch Behandeln der entsprechenden Säure mit Phosphoroxychlorid erhalten kann ; Phosphoroxychloridmethode), oder Anhydride mit organischen Säuren, wie gemischte Anhydride mit organischen Carbonsäuren (die man z. B. durch Behandeln der entsprechenden Säure mit einem gegebenenfalls substituierten Niederalkan- oder Phenylalkancarbonsäurehalogenid, z. B. Phenylessigsäure-, Pivalinsäure- oder Trifluoressigsäurechlorid erhalten kann ; Methode der gemischten Carbonsäureanhydride) oder mit organischen Sulfonsäuren (die man z. B. durch Behandeln eines Salzes, wie eines Alkalimetallsalzes, der entsprechenden Säure, mit einem geeigneten organischen Sulfonsäurehalogenid, wie Niederalkan- oder Aryl-, z. B. Methan- oder p-Toluolsulfonsäurechlorid, erhalten kann ; Methode der gemischten Sulfonsäureanhydride), sowie symmetrische Anhydride (die man z. B. durch Kondensation der entsprechenden Säure in Gegenwart eines Carbodiimids oder von 1-Diethylaminopropin erhalten kann ; Methode der symmetrischen Anhydride).

Geeignete cyclische Amide sind insbesondere Amide mit fünfgliedrigen Diazacyclen aromatischen Charackters, wie Amide mit Imidazolen, z. B. Imidazol (die man z. B. durch Behandeln der entsprechenden Säure mit N,N'-Carbonyldiimidazol ; Imidazolid-Methode), oder Pyrazolen, z. B. 3,5-Dimethyl-pyrazol (die man z. B. über das Säurehydrazid durch Behandeln mit Acetylaceton erhalten kann ; Pyrazolid-Methode).

Schutzgruppen von gegebenenfalls vorhandenen funktionellen Gruppen sind z. B. die untengenannten Schutzgruppen.

Die Reaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z. B., wenn die an der Reaktion teilnehmende Carboxylgruppe als Anhydrid vorliegt, auch ein Säure-bindendes Mittel sein kann, unter Kühlen oder Erwärmen, z. B. in einem Temperaturbereich von etwa — 30 °C bis etwa + 150 °C, in einem geschlossenen Reaktionsgefäss und/oder in der Atmosphäre eines Inertgases, z. B. Stickstoff. Uebliche Kondensationsmittel sind z. B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z. B. 2-Ethyl-5-phenyl-1,2-oxazolium-3'-sulfonat und 2-tert.-Butyl-5-methyl-isoxazolium-perchlorat, oder eine geeignete Acylaminoverbindung, z. B. 2-Ethoxy-1-ethoxycarbonyl-1,2-dihydrochinolin. Uebliche säurebindende Kondensationsmittel sind z. B. Alkalimetallcarbonate oder -hydrogencarbonate, z. B. Natrium- oder Kaliumcarbonat oder -hydrogencarbonat (üblicherweise zusammen mit einem Sulfat), oder organische Basen, wie üblicherweise sterisch gehinderte Triniederalkylamine, z. B. N,N-Diisopropyl-N-ethyl-amin.

Bevorzugterweise wird die Reaktion so durchgeführt, dass man einen aktivierten Carbonsäureester oder ein reaktionsfähiges Carbonsäureanhydrid oder cyclisches Amid mit einer Verbindung der Formel II umsetzt, worin die an der Reaktion teilnehmenden Hydroxy- und/oder Aminogruppen in freier Form vorliegen.

Schutzgruppen, ihre Einführung und Abspaltung sind beispielsweise beschrieben in « Protective Groups in Organic Chemistry », Plenum Press, London, New York 1973, und in « Methoden der organischen Chemie », Houben-Weyl, 4. Auflage, Bd. 15/1, Georg-Thieme-Verlag, Stuttgart 1974. Charakteristisch für Schutzgruppen ist, dass sie leicht, d. h. ohne dass unerwünschte Nebenreaktionen stattfinden, z. B. solvolytisch, reduktiv, photolytisch oder auch unter physiologischen Bedingungen abspaltbar sind.

Hydroxyschutzgruppen sind z. B. Acylreste, wie gegebenenfalls, z. B. durch Halogen, substituiertes

Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z. B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z. B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z. B. Methoxymethyl, 1-Methoxyethyl, 1-Ethoxy-ethyl, 1-Methylthiomethyl, 1-Methylthioethyl oder 1-Ethylthioethyl, oder 2-Oxa- oder 2-Thiacycloalkyl mit 5-6 Ringatomen z. B. Tetrahydrofuryl oder 2-Tetrahydropyranyl oder entsprechende Thiaanaloge, sowie gegebenenfalls substituiertes 1-Phenyl-niederalkyl, wie gegebenenfalls substituiertes Benzyl oder Diphenylmethyl, wobei als Substituenten der Phenylreste z. B. Halogen, wie Chlor, Niederalkoxy, wie Methoxy und/oder Nitro in Frage kommen.

Verfahren b (Verknüpfung von Zuckerteil und Seitenkette)

Eine Verbindung der Formel III, worin die 3-Hydroxy-Gruppe in reaktionsfähiger Form vorliegt, ist z. B. eine Metalloxyverbindung, wie sie z. B. durch Umsetzung einer Verbindung der Formel III mit einer stärkeren Base, wie einem Alkalimetallhydrid oder -amid, erhalten werden kann, oder eine Verbindung, in der die 3-Hydroxy-Gruppe in reaktionsfähiger veresterter Form, z. B. wie oben beschrieben, vorliegt.

Bevorzugterweise wird die Reaktion so durchgeführt, dass man eine 3-Metalloxy-Verbindung der Formel III mit einer Verbindung der Formel IV umsetzt, worin Z für eine der obenbeschriebenen reaktionsfähigen veresterten Hydroxygruppen steht. Daneben kann man auch eine Verbindung der Formel III, worin die 3-Hydroxy-Gruppe in reaktionsfähiger veresterter Form vorliegt, mit einer Verbindung der Formel IV umsetzen, worin Z für wie oben beschriebenes Metalloxy steht.

Verfahren c ($S_N2_t$-Reaktionen)

Reaktionsfähige Carbonsäurederivate einer Verbindung der Formel V sind reaktionsfähige Ester, Anhydride oder Amide analog den bei Verfahren a) genannten.

Wie bei a) erwähnt, können Derivate von Säuren der Formel V auch *in situ* gebildet werden. So kann man z. B. N,N'-disubstituierte Amidinoester *in situ* bilden, indem man das Gemisch des Ausgangsmaterials der Formel VI und der Säure der Formel V in Gegenwart eines geeigneten N,N'-disubstituierten Carbodiimids, z. B. N,N'-Dicyclohexylcarbodiimid, zur Reaktion bringt. Ferner kann man Amino- oder Amidoester von Säuren der Formel V in Gegenwart des zu acylierenden Ausgangsmaterials der Formel VI bilden, indem man das Gemisch der entsprechenden Säure- und Amino-Ausgangsstoffe in Gegenwart eines N,N'-disubstituierten Carbodiimids, z. B. N,N'-Dicyclohexyl-carbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt.

Ein Derivat der Verbindung der Formel VI, worin die an der Reaktion teilnehmende Aminogruppe in reaktionsfähiger Form vorliegt, kann z. B. durch Umsetzung mit einem Phosphit, z. B. einem der bei Verfahren a) genannten, hergestellt werden. Eine reaktionsfähige Form einer Verbindung der Formel VI ist z. B. auch ein Carbaminsäurehalogenid oder ein Isocyanat, wobei die an der Reaktion teilnehmende Aminogruppe in einer Verbindung der Formel VI an Halogencarbonyl, z. B. Chlorocarbonyl, gebunden ist bzw. als Isocyanatogruppe vorliegt, wobei im letzteren Falle nur Verbindungen der Formel I zugänglich sind, die am Stickstoffatom der durch die Reaktion gebildeten Amidgruppe ein Wasserstoffatom tragen.

Bevorzugterweise setzt man ein reaktionsfähiges Carbonsäurederivat einer Verbindung der Formel V mit einer Verbindung der Formel VI um, worin die an der Reaktion teilnehmende Amino- oder Hydroxygruppe in freier Form vorliegt.

Alternativ kann man auch ein Salz, z. B. ein Cäsiumsalz, einer Verbindung der Formel V, worin q und r für 1 und s für 0 stehen, mit einer Verbindung der Formel VI umsetzen, worin X für Sauerstoff, v für 1 und u und t für 0 stehen, wobei die an der Reaktion teilnehmende Hydroxygruppe in reaktionsfähiger veresterter Form vorliegt.

Schliesslich kann man auch eine freie Säure der Formel V mit einer Verbindung der Formel VI umsetzen, worin die an der Reaktion teilnehmende Gruppe in aktivierter Form vorliegt.

Die Acylierung einer Verbindung der Formel VI oder einem reaktionsfähigen Derivat davon mit einer Verbindung der Formel V oder einem reaktionsfähigen Säurederivat davon kann in an sich bekannter Weise durchgeführt werden, wobei die Reaktionsbedingungen in erster Linie von der Art des verwendeten Acylierungsmittels abhängen, üblicherweise in Gegenwart eines geeigneten Lösungs- oder Verdünnungsmittels oder eines Gemisches von solchen, und, falls notwendig, in Gegenwart eines Kondensationsmittels, das z. B. bei Verwendung eines Anhydrids als Acylierungsmittel gegebenenfalls auch ein Säure-bindendes Mittel sein kann, unter Kühlen oder Erwärmen, z. B. in einem Temperaturbereich von etwa —30 °C bis etwa + 150 °C, in einem geschlossenen Reaktionsgefäss und/oder in der Atmosphäre eines Inertgases, z. B. Stickstoff.

Die Ausgangsstoffe der Formeln V und VI sind bekannt oder können nach an sich bekannten Verfahren, z. B. analog den in dieser Anmeldung beschriebenen Verfahren, hergestellt werden.

Verfahren d (Phosphorsäurediester-Bildung)

Ein reaktionsfähiges Phosphorsäurederivat einer Säure der Formel VII oder VIII ist z. B. ein Mono- oder Bisanhydrid mit einer starken Säure, insbesondere einer Mineralsäure, wie vornehmlich einer Halogenwasserstoffsäure, wie hauptsächlich Chlorwasserstoffsäure. Die zweite saure Phosphorsäuregruppe kann als solche, als Anhydrid wie oben geschildert oder in veresterter Form vorliegen, wobei als veresternde Reste solche bevorzugt sind, die nach erfolgter Reaktion zwischen den Verbindungen VII und VIII regioselektiv wieder abgespalten werden können, z. B. die Methylestergruppe, die z. B. durch alkalische Verseifung abgespalten werden kann, oder insbesondere hydrogenolytisch abspaltbare Reste, z. B. Benzyl- oder Phenylesterreste, wobei Benzylesterreste z. B. in Gegenwart von Palladiumkatalysatoren, wie Palladium auf Kohlenstoff, und Phenylestergruppen z. B. in Gegenwart von Platin- oder gemischten Platin-Palladium-Katalysatoren abgespalten werden können.

Die Bildung reaktionsfähiger Phosphorsäurederivate kann auch *in situ* in Gegenwart von Verbindungen erfolgen, die mit Phosphorsäure oder deren Monoestern zumindest intermediär reaktionsfähige Verbindungen mit anhydrid- oder enolesterartigem Charakter einzugehen vermögen, z. B. in Gegenwart von p-Toluolsulfonsäurechlorid, Cyanurchlorid, N-Alkyl-5-phenylisoxazoliumsalzen, Ethoxyacetylen oder bevorzugterweise Trichloracetonitril oder insbesondere einem Carbodiimid, wie hauptsächlich Dicyclohexylcarbodiimid. Beispielsweise kann man einen Phosphorsäuremonoester der Formeln VII oder VIII mit überschüssigem Alkohol der Formeln VII bzw. VIII in Gegenwart der mehrfachen, z. B. fünffachen, molaren Menge von Dicyclohexylcarbodiimid in An- oder Abwesenheit eines tertiären Amins umsetzen.

Wenn in einem Phosphorsäuremonoester beide sauren Gruppen als Anhydrid mit einer Halogenwasserstoffsäure vorliegen, kann man zunächst neben dem Triester auch Phosphorsäurediesterhalogenide erhalten, die anschliessend durch Wasser, wasserabgebende Mittel oder durch Erhitzen mit tertiären Alkoholen, wie tert.-Butanol oder Tetrahydropyranol zu Diestern hydrolysiert werden können.

Wenn man von einem Phosphorsäuremonoester-dihalogenid, z. B. einem Phosphorsäuremonoesterdichlorid, ausgeht, führt man die Umsetzung bevorzugterweise in Anwesenheit eines tertiären Amins, wie Pyridin, Lutidin oder Chinolin durch, wobei eine zusätzliche Aktivierung des Esterchlorids durch Dimethylformamid bewirkt wird.

Eine bevorzugte Ausführungsform des Verfahrens d) ist die Umsetzung eines Phosphorsäuremonoester-dichlorids mit dem entsprechenden Alkohol in Gegenwart eines tertiären Amins, gefolgt von der Hydrolyse des zunächst erhaltenen Phosphorsäurediesterhalogenids.

Verfahren e (nucleophile Substitution mittels Phosphat)

Reaktionsfähige veresterte Hydroxygruppen sind die bei Verfahren a) genannten, bevorzugterweise Chloride, Bromide oder Jodide.

Als Salze von Verbindungen der Formeln VII oder VIII verwendet man im Hinblick auf die beabsichtigte nucleophile Substitutionsreaktion besonders reaktionsfähige Salze, z. B. Salze, wie Silbersalze, die mit der nucleophilen Abgangsgruppe im Reaktionspartner, z. B. einem der obengenannten Halogenidionen, einen schwerlöslichen Niederschlag zu bilden vermögen, oder Salze mit grossem Kation, z. B. Cäsiumsalze, in denen die Nucleophilie des Phosphatrestes erhöht ist. Zur Erhöhung der Nucleophilie des Phosphatrestes kann das Gegenion auch räumlich entfernt werden, z. B. durch Zugabe von Komplexbildnern, wie Kronenethern, z. B. 18-Krone-6. Bei Verwendung von 18-Krone-6 kann man die Reaktion mit einem Kaliumsalz durchführen.

Eine bevorzugte Ausführungsform des Verfahrens e) ist die Umsetzung des Silbersalzes eines Phosphorsäuremonoesters der Formeln VII oder VIII, worin eine der beiden sauren Gruppen durch eine leicht abspaltbare Schutzgruppe, z. B. eine der bei Verfahren d) beschriebenen, geschützt ist, z. B. als Benzyl- oder Phenylester, mit einem reaktionsfähigen veresterten Alkohol der Formeln VII bzw. VIII, worin die OH-Gruppe durch Chlor, Brom oder Jod substituiert ist. Nach erfolgter Umsetzung wird die Schutzgruppe abgespalten, z. B. eine Benzyl- oder Phenylesterschutzgruppe durch Hydrierung wie bei Verfahren d) beschrieben.

Verfahren f (Veresterung)

Reaktionsfähige Carbonsäurederivate einer Verbindung der Formel IX sind reaktionsfähige Ester, Anhydride oder Amide analog den bei Verfahren a) genannten oder Carbonsäuresalze.

Schutzgruppen sind z. B. die obengenannten. Ein reaktionsfähiges Derivat eines Niederalkanols $R^8$—OH ist z. B. ein solches mit, wie oben beschrieben, reaktionsfähiger veresterter Hydroxygruppe, z. B. eine der untengenannten Verbindungen.

Bevorzugterweise führt man die Reaktion so durch, dass man ein Carbonsäuresalz, z. B. ein Cäsiumsalz, einer Verbindung der Formel IX mit dem Niederalkanol $R^8$—OH umsetzt, worin die Hydroxygruppe in reaktionsfähiger veresterter Form vorliegt. Alternativ kann man ein reaktionsfähiges Carbonsäurederivat einer Verbindung der Formel IX mit dem Niederalkanol $R^8$—OH mit freier OH-Gruppe verestern.

Die Veresterung von freiem Carboxyl mit dem gewünschten Alkohol wird vorzugsweise in Gegenwart eines geeigneten Kondensationsmittels durchgeführt. Uebliche Kondensationsmittel sind z. B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z. B. 2-Ethyl-5-phenyl-1,2-oxazolium-3'-sulfonat und 2-tert.-Butyl-5-methyl-isoxazolium-perchlorat, oder eine geeignete Acylaminoverbindung, z. B. 2-Ethoxyl-1-ethoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z. B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Weiterhin kann man eine freie Carbonsäure der Formel IX mit dem reaktionsfähigen Derivat eines Niederalkanols $R^8$—OH umsetzen. Geeignete Derivate dieser Art sind beispielsweise entsprechende Diazoverbindungen, wie gegebenenfalls substituierte Diazoniederalkane, z. B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffes, wie Hexan, Cyclohexan, Benzol oder Toluol, eines halogenierten aliphatischen Kohlenwasserstoffs, z. B. Methylenchlorid, oder eines Ethers, wie eines Diniederalkylethers, z. B. Diethylether, oder eines cyclischen Ethers, z. B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z. B. Stickstoffatmosphäre, zur Anwendung gebracht.

Weitere geeignete Mittel zur Veresterung einer Carboxylgruppe in einer Verbindung der Formel IX sind Ester entsprechender Alkohole, in erster Linie solche mit starken anorganischen oder organischen Säuren, wie Mineralsäuren, z. B. Halogenwasserstoffsäuren, wie Chlorwasserstoff-, Bromwasserstoff- oder Jodwasserstoffsäure, ferner Schwefelsäure, oder Halogen-schwefelsäure, z. B. Fluorschwefelsäure, oder starken organischen Sulfonsäuren, wie gegebenenfalls, z. B. durch Halogen, wie Fluor, substituierten Niederalkansulfonsäuren, oder aromatischen Sulfonsäuren, wie z. B. gegebenenfalls durch Niederalkyl, wie Methyl, Halogen, wie Brom, und/oder Nitro substituierten Benzolsulfonsäuren, z. B. Methansulfon-, Trifluormethansulfon- oder p-Toluolsulfonsäure. Solche Ester sind u. a. Niederalkylhalogenide, Diniederalkylsulfate, wie Dimethylsulfat, ferner Fluorsulfonsäureester, wie -niederalkylester, z. B. Fluorsulfonsäuremethylester, oder gegebenenfalls Halogen-substituierte Methansulfonsäure-niederalkylester, z. B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z. B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z. B. Natrium- oder Kaliumcarbonat oder -hydrogencarbonat (üblicherweise zusammen mit einem Sulfat), oder organische Basen, wie üblicherweise sterisch gehinderte Triniederalkylamine, z. B. N,N-Diisopropyl-N-ethyl-amin (vorzugsweise zusammen mit Halogensulfonsäureniederalkylestern oder gegebenenfalls halogensubstituierten Methansulfonsäureniederalkylestern) an, wobei unter Kühlen, bei Raumtemperatur oder unter Erwärmen, z. B. bei Temperaturen von etwa — 20 °C bis etwa + 50 °C und, wenn notwendig, in einem geschlossenen Gefäss und/oder in einer Inertgas-, z. B. Stickstoffatmosphäre, gearbeitet wird.

Weitere Mittel zur Veresterung einer Carboxylgruppe in einer Verbindung der Formel IX sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z. B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Veretherungsmittel vorzugsweise in einem inerten Lösungsmittel, wie einem Ether oder einem halogenierten Kohlenwasserstoff, z. B. Diethylether, Tetrahydrofuran oder Methylenchlorid, oder in einem Gemisch davon, wenn notwendig, in Gegenwart einer Base, wie einer organischen Base, z. B. eines, vorzugsweise sterisch gehinderten, Triniederalkylamins, z. B. N,N-Diisopropyl-N-ethyl-amin, und unter Kühlen, bei Raumtemperatur oder unter leichtem Erwärmen, z. B. bei etwa — 20 °C bis etwa 50 °C, wenn notwendig, in einem geschlossenen Gefäss und/oder in einer Inertgas-, z. B. Stickstoffatmosphäre.

Verfahren g (Oxidation)

Die Verbindungen der Formel X, worin $R^{21}$ für Wasserstoff steht, liegen zum überwiegenden Teil in der tautomeren Form vor, worin ein Proton direkt an Phosphor gebunden ist. Die Oxidation kann z. B. mit wässerigem Kaliumpermanganat bei Temperaturen um 0 °C durchgeführt werden. In wässerigem Medium sind auch Alkalijodate, -perjodate und -hypochlorite, Peressigsäure, N-Chlor-4-methyl-benzolsulfonsäureamid u. a. als Oxidationsmittel geeignet.

Verfahren h ([Furanose→Pyranose]-Umwandlung)

Als Schutzgruppen verwendet man z. B. die obengenannten. Bivalente Hydroxyschutzgruppen sind insbesondere gegebenenfalls substituierte Alkyliden- oder Cycloalkylidengruppen. Alkyliden ist darin insbesondere Niederalkyliden, wie Isopropyliden, und Cycloalkyliden in erster Linie Cyclopentyliden oder Cyclohexyliden. Als Substituenten der Alkylidenreste seien insbesondere aromatische Reste, z. B. Phenylreste, genannt.

Die Abspaltung der in dieser Anmeldung genannten Schutzgruppen im allgemeinen und die Abspaltung gemäss Verfahren h) im besonderen geschieht in an sich bekannter Weise mittels Solvolyse, insbesondere Hydrolyse, Alkoholyse oder Acidolyse, oder in einigen Fällen auch mittels vorsichtiger Reduktion, gegebenenfalls stufenweise oder gleichzeitig. Silylschutzgruppen werden vorteilhafterweise mit Fluoriden, z. B. Tetraethylammoniumfluorid, abgespalten.

Eine besonders bevorzugte Ausführungsform des Verfahrens h) geht von Verbindungen der Formel XI aus, worin $R^{19}$ und $R^{20}$ zusammen für einen gegebenenfalls substituierten Alkyliden- oder Cycloalkylidenrest und $R^{17}$ und $R^{18}$ zusammen für die Gruppe

$$R^2-\overset{|}{C}=N-$$

stehen. In diesem Fall erfolgt die Spaltung, die besonders zur Herstellung von N-Benzoyl-Verbindungen der Formel I geeignet ist, mit verdünnter Säure, am besten bei einem pH-Wert von 2-4, z. B. 3, als Eintopfreaktion ebenfalls in an sich bekannter Weise, z. B. mit einem sauren Ionenaustauscher, insbesondere einem solchen mit Sulfonsäuregruppen, wie Amberlite IR-120 (ein Styrolharz mit stark sauren Sulfogruppen) oder Dowex 50 (Polystyrolsulfonsäuren) oder einer starken anorganischen oder organischen Säure, wie Salzsäure, Bromwasserstoffsäure, Schwefelsäure oder einer Sulfonsäure, z. B. Methansulfonsäure, oder einer gegebenenfalls im aromatischen Ring substituierten Phenylsulfonsäure, wie p-Toluolsulfonsäure, oder Trifluoressigsäure. Arbeitet man dabei in Gegenwart von Wasser, erhält man in l-Stellung eine freie Hydroxygruppe.

Verfahren i (Abspaltung von Schutzgruppen)

Die Abspaltung von Schutzgruppen aus einer Verbindung der Formel I erfolgt analog wie bei Verfahren h.

Verfahren j (Hydrolyse von Phosphorsäurediester-halogeniden)

In einer Verbindung der Formel XII steht A für ein Halogen, wie Brom oder Jod, in allererster Linie aber für Chlor.

Die Hydrolyse wird mit Wasser oder einem wasserabgebenden Mittel, vorzugsweise bei erhöhter Temperatur, z. B. zwischen 30 und 95 °C, durchgeführt.

Die Ausgangsstoffe sind z. B. durch Chlorierung der entsprechenden Phosphorigsäurediester, z. B. mit elementarem Chlor, erhältlich.

Die zur Ausführung der obengenannten Verfahren benötigten Ausgangsstoffe sind bekannt oder können nach an sich bekannten Verfahren, z. B. analog einem der vorstehend beschriebenen Verfahren, hergestellt werden.

Die Salze der Verbindungen der Formel I entstehen meist bereits bei der Aufarbeitung, z. B. bei der Dialyse gegen eine Pufferlösung mit pH 7. Sie können in an sich bekannter Weise hergestellt werden. So kann man Salze von Verbindungen der Formel I z. B. durch Behandeln mit Metallverbindungen, wie Alkalimetallsalzen von geeigneten organischen Carbonsäuren, z. B. dem Natriumsalz der α-Ethylcapronsäure, oder mit anorganischen Alkali- oder Erdalkalimetallverbindungen, wie entsprechenden Hydroxiden, Carbonaten und Hydrogencarbonaten, wie Natrium- oder Kaliumhydroxid, -carbonat oder -hydrogencarbonat, oder entsprechenden Calciumverbindungen oder mit Ammoniak oder geeigneten organischen Aminen bilden, wobei man vorzugsweise stöchiometrische Mengen oder einen kleinen Ueberschuss des salzbildenden Mittels verwendet.

Gemische von Isomeren können in an sich bekannter Weise, z. B. durch fraktionierte Kristallisation, Chromatographie etc. in die einzelnen Isomeren, Racemate z. B. unter Bildung von Derivaten mit optisch aktiven Verbindungen und Trennung der so erhältlichen Diastereomerengemische in die optisch aktiven Antipoden, aufgetrennt werden.

Die oben beschriebenen Verfahren, inklusive die Verfahren zur Abspaltung von Schutzgruppen und die zusätzlichen Verfahrensmassnahmen, werden in an sich bekannter Weise, z. B. in An- oder Abwesenheit von Lösungs- und Verdünnungsmitteln, wenn notwendig, in Anwesenheit von Kondensationsmitteln oder Katalysatoren, bei erniedrigter oder erhöhter Temperatur, z. B. in einem Temperaturbereich von etwa — 20 °C bis etwa 150 °C, in einem geschlossenen Gefäss und/oder in einer Inertgas-, z. B. Stickstoffatmosphäre, durchgeführt.

Dabei sind unter Berücksichtigung aller im Molekül befindlichen Substituenten, wenn erforderlich, z. B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die Erfindung betrifft ebenfalls pharmazeutische Präparate, welche eine pharmakologisch wirksame Menge der Aktivsubstanz, gegebenenfalls zusammen mit pharmazeutisch verwendbaren Trägerstoffen enthalten, die sich zur enteralen, z. B. oralen oder rektalen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z. B. Lactose, Dextrose, Sukrose, Mannitol, Sorbitol, Cellulose und/oder Glycerin, und/oder Schmiermitteln, z. B. Kieselerde, Talk, Stearinsäure oder Salze davon, wie Magnesium- oder Calciumstearat, und/oder Polyethylenglykol, enthalten. Tabletten können ebenfalls Bindemittel, z. B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika enthalten können, werden in an sich bekannter Weise, z. B. mittels konventioneller Misch-, Granulier-, Dragier-, Lösungs- oder Lyophilisierungsverfahren, hergestellt und enthalten von etwa 0,001 % bis 99 %, insbesondere von etwa 0,01 % bis etwa 10 %, in erster Linie zwischen 0,1 % und 5 %, des bzw. der Aktivstoffe, wobei eine Wirkstoffkonzentration unterhalb von 1 %, insbesondere für topisch zu applizierende Präparate geeignet ist.

Für die Prophylaxe und Therapie von Virusinfektionen besonders geeignet sind die folgenden topisch zu applizierenden Darreichungsformen : Crèmes, Salben oder Pasten mit einem Wirkstoffgehalt von 0,001 % bis 1 %, insbesondere von 0,01 % bis 0,1 %, z. B. 0,05 %, z. B. Salben zur intranasalen Applikation oder Lippenstifte, oder wässrige Lösungen mit einem Wirkstoffgehalt von 0,001 % bis 1 %, insbesondere 0,05 % bis 0,5 %, z. B. 0,1 %, vorzugsweise isotonische, sterile und physiologisch verträgliche Lösungen, z. B. Augentropfen, vorzugsweise in Mikrobehältern zur einmaligen Verwendung, oder Sprays zur Anwendung im Mund- und Rachenraum.

Besonders geeignet sind die in den Beispielen beschriebenen pharmazeutischen Präparate.

Crémes sind Oel-in-Wasser-Emulsionen, die mehr als 50 % Wasser aufweisen. Als ölige Grundlage verwendet man in erster Linie Fettalkohole, z. B. Lauryl-, Cetyl- oder Stearylalkohol, Fettsäuren, z. B. Palmitin- oder Stearinsäure, flüssige bis feste Wachse, z. B. Isopropylmyristat, Wollwachs oder Bienenwachs, und/oder Kohlenwasserstoffe, z. B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z. B. Fettsäureester von Polyalkoholen oder Aethylenoxidaddukte davon, wie Polyglycerinfettsäureester oder Polyoxyäthylensorbitan-fettsäureester (Tweens®), ferner Polyoxyäthylen-fettalkoholäther oder -fettsäureester, oder entsprechende ionische Emulgatoren, wie Alkalimetallsalze von Fettalkoholsulfaten, z. B. Natriumlaurylsulfat, Natriumcetylsulfat oder Natriumstearylsulfat, die man üblicherweise in Gegenwart von Fettalkoholen, z. B. Cetylalkohol oder Stearylalkohol, verwendet. Zusätze zur Wasserphase sind u. a. Mittel, welche die Austrocknung der Crémes vermindern, z. B. Polyalkohol, wie Glycerin, Sorbit, Propylenglykol und/oder Polyäthylenglykole, ferner Konservierungsmittel, Riechstoffe, etc.

Salben sind Wasser in-Oel-Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z. B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z. B. Cetylalkohol oder Wollwachsalkohole, bzw. Wollwachs, enthalten. Emulgatoren sind entsprechende lipophile Substanzen, wie Sorbitan-fettsäureester (Spans®), z. B. Sorbitanoleat und/oder Sorbitanisostearat. Zusätze zur Wasserphase sind z. B. Feuchthaltungsmittel, wie Polyalkohole, z. B. Glycerin, Propylenglykol, Sorbit und/oder Polyäthylenglykol, wie Konservierungsmittel, Riechstoffe.

Fettsalben sind wasserfrei und enthalten als Grundlage insbesondere Kohlenwasserstoffe, z. B.

Paraffin, Vaseline und/oder flüssige Paraffine, ferner natürliche oder partialsynthetische Fette, z. B. Kokosfettsäuretriglycerid, oder vorzugsweise gehärtete Oele, z. B. hydriertes Erdnuss- oder Rizinusöl, ferner Fettsäurepartialester des Glycerins, z. B. Glycerinmono- und -distearat, sowie z. B. die im Zusammenhang mit den Salben erwähnten, die Wasseraufnahmefähigkeit steigernden Fettalkohole, Emulgatoren und/oder Zusätze.

Pasten sind Crèmes und Salben mit sekretabsorbierenden Puderbestandteilen, wie Metalloxiden, z. B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z. B. Dichlordifluormethan und Dichlortetrafluoräthan, als Treibmittel verwendet werden. Als Oelphase verwendet man u. a. Kohlenwasserstoffe, z. B. Paraffinöl, Fettalkohole, z. B. Cetylalkohol, Fettsäureester, z. B. Isopropylmyristat, und/oder andere Wachse. Als Emulgatoren verwendet man u. a. Gemische von solchen mit vorwiegend hydrophilen Eigenschaften, wie Polyoxyäthylen-sorbitan-fettsäureester (Tweens®), und solchen mit vorwiegend lipophilen Eigenschaften, wie Sorbitanfettsäureester (Spans®). Dazu kommen die üblichen Zusätze, wie Konservierungsmittel, etc.

Tinkturen und Lösungen weisen meistens eine wässrig-äthanolische Grundlage auf, der u. a. Polyalkohole, z. B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d. h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z. B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst ; bei Verarbeitung als Suspension wird er nach der Emulgierung mit einem Teil der Grundlage vermischt und dann dem Rest der Formulierung beigegeben.

Die Dosierung des Wirkstoffes hängt von verschiedenen Faktoren, wie Applikationsweise, Spezies, der Abwehrlage des Organismus sowie in entscheidendem Masse von der Art der zu behandelnden Krankheit ab. So liegen die täglich zu verabreichenden Dosen bei oraler Applikation an Warmblütern von etwa 70 kg zwischen etwa 0,000 1 und 0,1 g, wobei eine Dosierung von weniger als 0,001 g in erster Linie zur Vermeidung der Metastasenbildung nach Entfernung des Primärtumors verwendet wird.

Zur Vorbeugung von Virusinfektionen appliziert man eine einmalige Dosis von etwa 0,5 mg bis etwa 50 mg, vorzugsweise 25 bis 15 mg, z. B. 7 mg, Wirkstoff an einen Warmblüter von etwa 70 kg Körpergewicht, z. B. den Menschen. Bei Bedarf in Zeiten erhöhter Ansteckungsgefahr wiederholt man die Verabreichung dieser Dosis in Abständen von 1-3 Wochen, z. B. alle 2 Wochen.

Die therapeutische Dosis für Warmblüter von etwa 70 kg Körpergewicht liegt im Falle einer Virusinfektion zwischen 1 mg und 50 mg, vorzugsweise zwischen 5 und 20 mg, z. B. bei 10 mg, insbesondere bei oraler Applikation. Die Dosierung bei intranasaler Applikation liegt bis zum Faktor 10 niedriger. Bei Bedarf wiederholt man die Verabreichung der Hexopyranoseverbindungen der Formel I im Abstand von einigen Tagen, z. B. 1-3 Tagen, bis zum Eintritt einer Besserung der Erkrankung.

Die nachfolgenden Beispiele illustrieren die Erfindung, ohne sie in irgendeiner Form einzuschränken. Die $R_f$-Werte werden auf Kieselgeldünnschichtplatten der Firma Merck ermittelt. Das Verhältnis der Laufmittel in den verwendeten Laufmittelgemischen zueinander ist in Volumenanteilen (V/V), Temperaturen sind in Grad Celsius angegeben.

## Beispiel 1

3,60 g (5,66 mMol) N-Acetyl-muramyl-L-alanyl-D-glutaminsäure-($C_\alpha$)-n-butylester (0,6 Mol $H_2O$) und 0,88 g (7,54 mMol) N-Hydroxysuccinimid werden unter Rühren in 20 ml eines Gemisches aus Chloroform : Methanol = 4 : 1 gelöst. Man fügt 1,60 g (7,54 mMol) festes Dicyclohexylcarbodiimid zu und tropft eine Lösung von 3,7 g (4,72 mMol) L-Alanin-2-(1,2-dipalmitoyl-sn-glycero-3-hydroxyphosphoryloxy)-ethylamid-natriumsalz in 40 ml Chloroform innerhalb von vier Stunden bei Raumtemperatur kontinuierlich zu. Nach einer weiteren Stunde wird die Reaktionslösung am Rotationsverdampfer stark eingeengt, 200 ml Dioxan zugefügt und lyophilisiert.

Das Rohprodukt wird in 100 ml Chloroform suspendiert, 600 ml Essigsäureethylester werden hinzugefügt und das Ganze während einer Stunde im Eisbad gerührt. Der unlösliche Dicyclohexylharnstoff wird abfiltriert, das Filtrat zur Trockne verdampft. Der Rückstand wird an 700 g Kieselgel 60 reinst (Merck, Korngrösse : 70-230 mesh ASTM) auf einer Säule von 4,5 cm Durchmesser und 80 cm Länge im System Chloroform : Methanol : Wasser = 70 : 30 : 5 gereinigt. Das Material wird dazu in 14 ml Laufmittel gelöst und auf die Säule gebracht. Nach einem Vorlauf von zweimal 500 ml Gemisch werden 10 ml Fraktionen gefasst. Das in den Fraktionen 133 bis 152 und 153 bis 236 enthaltene Material wird jeweils gesammelt und getrennt einer Diafiltration unterworfen. Bei der Hauptfraktion verfährt man wie folgt : Man löst das Material in 300 ml doppelt destilliertem Wasser, erwärmt kurz auf 40 °C und entfernt den unlöslichen Dicyclohexylharnstoff nach dem Abkühlen durch Zentrifugation. Das Ueberstehende wird in eine Dialysierzelle (Hersteller : Amicon Corporation, Danvers, Massachusetts, 01932 USA, Modell 402,

Ultrafilter PM 10/76 mm Ø, inerte, nicht-ionische Polymere auf Polysulfonbasis, mittlere Porengrösse 10 Å) gegeben und bei 4 Atü gegen 1,5 l doppelt destilliertes Wasser, 0,5 l Phosphatpuffer/Natriumchlorid (je 0,1 molar, 1 : 1, pH = 7) und 1,5 l Wasser bis chloridfrei dialysiert. Das Innendialysat (70 ml) wird durch ein Millipore-Filter (0,2 μ) sterilfiltriert und lyophylisiert. Man erhält N-Acetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (3,4 Mol $H_2O$) als farbloses Pulver mit

$[\alpha]_D^{20} = + 14 \pm 1°$ (c = 0,44, Wasser),
$R_f$ = 0,37 (Chloroform : Methanol : Wasser = 70 : 30 : 5) und
$R_f$ = 0,64 (Essigsäureethylester : n-Butanol : Pyridin : Essigsäure : Wasser = 42 : 21 : 21 : 6 : 10).

$C_{63}H_{115}O_{20}N_5PNa \cdot 3,41\ H_2O$ (1 381,63)

| | | | | | |
|---|---|---|---|---|---|
| Ber. : | C 54,77 | H 8,94 | N 5,07 | P 2,24 | $H_2O$ 4,71 |
| Gef. : | C 54,3 | H 9,1 | N 5,6 | P 2,3 | $H_2O$ 4,7 |

$C_{63}H_{115}O_{20}N_5PNa$ (1 316,59)
Ber. : Na 1,75
Gef. : Na 1,57.

Das Ausgangsmaterial erhält man folgendermassen : 5,5 g (8,1 mMol) 4,6-O-Isopropyliden-N-acetylmuramyl-L-alanyl-D-glutaminsäure-($C_\alpha$)-n-butyl-($C_\gamma$)-benzylester, gelöst in 60 ml 60 %iger Essigsäure, werden über Nacht bei Raumtemperatur stehen gelassen. Die schwach gelbliche Lösung wird am Rotationsverdampfer bei 30 °C auf ca. die Hälfte eingeengt, mit 300 ml Dioxan versetzt und lyophilisiert. 4,8 g (~ 7,5 mMol) des Rohproduktes, gelöst in 100 ml eines Gemisches aus Dimethoxyethan und Wasser = 9 : 1, werden mit 0,8 g eines Palladiumoxid-auf-Kohle-Katalysators (10 %ig) versetzt und während vier Stunden mit Wasserstoff behandelt. Der Katalysator wird abgesaugt, das Filtrat zur Trockne verdampft und der Rückstand durch zweimalige Chromatographie an Kieselgel 60 (Merck, 1 : 200, 10 und 5 ml Fraktionen) im System Chloroform : Methanol : Wasser = 70 : 30 : 5 gereinigt. Die das Produkt enthaltenden Fraktionen werden vereinigt und das Lösungsmittel verdampft. Das Rohprodukt, teilweise in Form des Natriumsalzes (aus Kieselgel) vorliegend, wird in 40 ml doppelt destilliertem Wasser gelöst und über 50 ml eines gut vorgereinigten, stark sauren Ionenaustauschers (DOWEX 50 W X8 50/100 mesh, H-Form) gegeben und die Säule mit total 100 ml doppelt destilliertem Wasser nachgewaschen. Das Eluat wird durch ein Millipore-Filter (0,2 μ) filtriert und lyophilisiert. Man erhält 3,7 g (69 % d. Th.) N-Acetyl-muramyl-L-alanyl-D-glutaminsäure-($C_\alpha$)-n-butylester als farbloses Pulver, das 0,6 Mol Wasser enthält, mit

$[\alpha]_D^{20} = + 45 \pm 1°$ (c = 1,142 ; Methanol),
$R_f$ = 0,23 (Chloroform : Methanol : Wasser = 70 : 30 : 5) und
$R_f$ = 0,40 (n-Butanol : Essigsäure : Wasser = 75 : 7,5 : 21).

Das Ausgangsmaterial erhält man folgendermassen :

4,4 g (11 mMol) N-Acetyl-4,6-O-isopropyliden-muraminsäure-natriumsalz (2,5 mMol/g) werden in 60 ml abs. Dimethylformamid suspendiert. Unter gutem Rühren gibt man nacheinander 4,4 g (11 mMol) L-Alanyl-D-glutaminsäure-($C_\alpha$)-n-butylester-($C_\gamma$)-benzylester-hydrochlorid 2,53 g (22 mMol) N-Hydroxysuccinimid und schliesslich 2,5 g (12,1 mMol) Dicyclohexylcarbodiimid zu und rührt das Ganze über Nacht bei Raumtemperatur. Zur Aufarbeitung wird die Suspension mit 100 ml Essigsäureethylester verdünnt und nach halbstündigem Rühren bei 0 °C das Ungelöste (Dicyclohexylharnstoff, Natriumchlorid) abfiltriert. Das Filtrat wird im Hochvakuum bei 30° eingedampft, der Rückstand in 400 ml Essigester aufgenommen und zehnmal mit je 50 ml doppelt destilliertem Wasser extrahiert. Nach Trocknen der organischen Phase und Verdampfen des Lösungsmittels verbleiben 5,6 g N-Acetyl-4,6-O-isopropyliden-L-alanyl-D-glutaminsäure-($C_\alpha$)-n-butylester-($C_\gamma$)-benzylester als amorphes Pulver mit

$[\alpha]_D^{20} = + 30 + 1°$ (c = 0,732 ; Methanol),
$R_f$ = 0,87 (Chloroform : Methanol : Wasser = 70 : 30 : 5) und
$R_f$ = 0,83 (n-Butanol : Essigsäure : Wasser = 75 : 7,5 : 21).

Das Ausgangsprodukt erhält man folgendermassen :

5,6 g (12,1 mMol) N-tert. Butyloxycarbonyl-L-alanyl-D-glutaminsäure-($C_\alpha$)-n-butylester-($C_\gamma$)-benzylester, gelöst in 20 ml abs. Essigsäureethylester, werden unter gutem Rühren und Ausschluss von Feuchtigkeit tropfenweise mit 50 ml 4,5 N Salzsäure in abs. Essigester versetzt und das Ganze während einer Stunde bei 0° stehen gelassen. Die gelbliche Lösung wird bei Raumtemperatur auf ca. 20 ml eingeengt, mit 150 ml Dioxan versetzt und lyophilisiert. Man erhält 4,75 g (97 % d. Th.) L-Alanyl-D-glutaminsäure-($C_\alpha$)-n-butylester-($C_\gamma$)-benzylester-hydrochlorid als farbloses Oel mit

$[\alpha]_D^{20} = + 19 \pm 1°$ (c = 0,313 ; Methanol),
$R_f$ = 0,58 (Chloroform : Methanol : Wasser = 70 : 30 : 5) und
$R_f$ = 0,56 (Essigsäureethylester : n-Butanol : Pyridin : Wasser = 42 : 21 : 6 : 10).

Das Ausgangsprodukt erhält man folgendermassen :

Zu 8,5 g (25,8 mMol) D-Glutaminsäure-($C_\alpha$)-n-butylester-($C_\gamma$)-benzylester-hydrochlorid und 7,4 g (25,8 mMol) N-tert. Butyloxycarbonyl-L-alanin-N-hydroxysuccinimidester, gelöst in 150 ml abs. Dimethylformamid, werden 2,6 g (25,8 mMol) N-Methyl-morpholin gegeben und das Ganze während 20 Stunden bei Raumtemperatur stehen gelassen. Die klare, gelbe Lösung wird im Hochvakuum bei 30 °C eingedampft, der halbfeste Rückstand in 500 ml Essigsäureethylester aufgenommen und siebenmal mit je 50 ml Wasser extrahiert. Die organische Phase wird mit wasserfreiem Natriumsulfat getrocknet. Der nach dem Verdampfen des Essigesters verbleibende Rückstand wird durch Chromatographie an 600 g Kieselgel 60 (Merck, Korngrösse 70-230 mesh ASTM) im System Chloroform : Methanol = 98 : 2 gereinigt. Das in den Fraktionen 36 bis 82 enthaltene Material wird gesammelt und getrocknet. Man erhält N-tert.-Butyloxycarbonyl-L-alanyl-D-glutaminsäure-($C_\alpha$)-n-butylester-($C_\gamma$)-benzylester als farbloses Oel mit
$[\alpha]_D^{20} = +7 \pm 1°$ (c = 0,315 ; Dimethylformamid),
$R_f$ = 0,73 (Chloroform : Isopropanol : Essigsäure = 70 : 8 : 2) und
$R_f$ = 0,75 (n-Butanol : Essigsäure : Wasser = 75 : 7,5 : 21).

Das Ausgangsmaterial erhält man folgendermassen :
Zu 11,0 g (30 mMol) N-tert. Butyloxycarbonyl-D-glutaminsäure-($C_\alpha$)-n-butylester-($C_\gamma$)-benzylester, gelöst in 25 ml abs. Essigsäureethylester, gibt man in der Kälte unter Rühren und Ausschluss von Feuchtigkeit 25 ml 4N HCl in abs. Essigsäureethylester und lässt das Ganze während einer Stunde stehen. Die leichtflüchtigen Anteile werden bei 25 °C abgedampft, der ölige Rückstand in 150 ml abs. Diethylether aufgenommen und letzterer wieder verdampft (3 mal). Der ölige Rückstand wird über Natronasbest im Halbvakuum getrocknet. Man erhält D-Glutaminsäure-($C_\alpha$)-n-butylester-($C_\gamma$)-benzylester-hydrochlorid mit
$[\alpha]_D^{20} = -9 \pm 1°$ (c = 0,646 ; Methanol).
$R_f$ = 0,82 (Chloroform : (Methanol : Wasser = 70 : 30 : 5) und
$R_f$ = 0,68 (Essigsäureethylester : n-Butanol : Pyridin : Essigsäure : Wasser = 42 : 21 : 21 : 6 : 10).

Das Ausgangsmaterial erhält man folgendermassen :
20,0 g (64,6 mMol) N-tert. Butyloxycarbonyl-D-glutaminsäure-($C_\gamma$)-benzylester werden in 750 ml abs. Tetrahydrofuran gelöst, 21,05 g (64,6 mMol) Cäsiumcarbonat (Fluka, purum), gelöst in 80 ml Wasser, eingetropft und das Ganze am Wasserstrahlvakuum zur Trockne verdampft. Der Rückstand wird in 200 ml abs. Dimethylformamid aufgenommen, das Lösungsmittel verdampft und diese Operation wiederholt. Der nach dem Trocknen im Hochvakuum erhaltene kristalline Rückstand wird in einem Liter abs. Dimethylformamid gelöst und dazu unter Rühren 13,3 g (97 mMol) n-Butyl-bromid eingetropft. Die nach 18 stündigem Rühren bei Raumtemperatur entstandene Suspension wird filtriert, das Filtrat auf die Hälfte eingeengt und nach Zugabe von einem Liter Essigsäureethylester zehnmal mit je 100 ml Wasser zügig extrahiert. Die organische Phase wird über Natriumsulfat getrocknet und eingedampft. Der ölige Rückstand wird in 100 ml Diethylether aufgenommen und durch Zugabe von 1 800 ml Petrolether und Stehenlassen bei — 10° zur Kristallisation gebracht. Die Kristallmasse wird abgesaugt, gewaschen und getrocknet. Man erhält N-tert. Butyloxycarbonyl-D-glutaminsäure-($C_\alpha$)-n-butylester-($C_\gamma$)-benzylester in Form von farblosen Nadeln mit Fp. 70-71°,
$[\alpha]_D^{20} = 20 \pm 1°$ (c = 1,149 ; Methanol),
$R_f$ = 0,77 (Chloroform : Isopropanol : Essigsäure = 30 : 8 : 2) und
$R_f$ = 0,92 (Essigsäureethylester : n-Butanol : Pyridin : Essigsäure : Wasser = 42 : 21 : 21 : 6 : 10),

dessen Herstellung bereits in P. Lefrancier und E. Lederer, Fortschritte d. Chem. org. Naturst. *40*, 1-47, siehe S. 13 (1980) kurz beschrieben ist.

Das als Ausgangsmaterial dienende L-Alanin-2-(1,2-dipalmitoyl-sn-glycero-3-hydroxyphosphoryloxy)-ethylamid erhält man wie folgt : 13,25 g (14,9 mMol) N-tert. Butyloxycarbonyl-L-alanin-2-(1,2-dipalmitoyl-sn-glycero-3-hydroxyphosphoryloxy)-ethylamid werden unter Rühren in 170 ml eines auf 0 °C abgekühlten Gemisches von Trifluoressigsäure und Methylenchlorid 1 : 3 (v/v) eingetragen, wobei eine klare Lösung entsteht. Nach 2 1/2 stündigem Stehen bei Raumtemperatur wird die Reaktionslösung bei 30 °C am Rotationsverdampfer eingedampft. Der halbfeste Rückstand wird zur Entfernung überschüssiger Trifluoressigsäure mehrmals mit je 100 ml Methylenchlorid versetzt und letzteres jeweils abgedampft. Nach fünfmaligem Verreiben des Rückstandes mit je 100 ml abs. Diethylether und Abdekantieren des Ueberstehenden erhält man eine gut filtrierbare Suspension. Man nutscht ab, wäscht das feste Material erst mit Diethylether, dann zweimal mit je 100 ml heissem Aceton und trocknet am Wasserstrahlvakuum bei 60-70 °C. Man erhält 10,9 g L-Alanin-2-(1,2-dipalmitoyl-sn-glycero-3-hydroxyphosphoryloxy)-ethylamid in Form farbloser Kristalle mit Fp. 138-147°,
$[\alpha]_D^{20} = +30 \pm 1°$ (c = 1 ; Chloroform : Methanol : Wasser = 70 : 30 : 5),
$R_f$ = 0,14 (Chloroform : Methanol = 7 : 3) und
$R_f$ = 0,40 (Chloroform : Methanol : Wasser = 70 : 30 : 5).

Das so erhaltene Produkt überführt man folgendermassen in das Natriumsalz.
3,82 g (5 mMol) L-Alanin-2-(1,2-dipalmitoyl-sn-glycero-3-hydroxyphosphoryloxy)-ethylamid werden in 100 ml eines Gemisches von Chloroform : Methanol = 7 : 3 durch kurzes Erwärmen auf 35 °C in Lösung

gebracht. Man kühlt ab und tropft vorsichtig 5 ml 1N Natronlauge zu. Die klare Lösung wird auf etwa 1/3 des ursprünglichen Volumens eingeengt und nach Zugabe von 200 ml Dioxan lyophilisiert. Man erhält L-Alanin-2-(1,2-dipalmitoyl-sn-glycero-3-hydroxyphosphoryloxy)-ethylamid-natriumsalz als lockeres Pulver.

### Beispiel 2

Analog Beispiel 1 erhält man die in den Beispielen 3-10 genannten sowie die folgenden Verbindungen :

N-Acetyl-muramyl-N-methyl-L-alanyl-D-glutamyl-($C_\alpha$)-ethylester-($C_\gamma$)-L-alanin-2-(1,2-dipalmitoyl-sn-glycero-3-hydroxyphosphoryloxy)-ethylamidnatriumsalz,

N-Acetyl-desmethylmuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz,

N-Acetyl-muramyl-L-seryl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz,

N-Acetyl-muramyl-L-cysteinyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz,

N-Acetyl-muramyl-L-methionyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz,

N-Acetyl-muramyl-L-alanyl-D-glutamyl-($C_\alpha$)-n-butylester-($C_\gamma$)-L-alanin-2-(1,2-dimyristoyl-sn-glycero-3-hydroxyphosphoryloxy)-ethylamid-natriumsalz,

N-Acetyl-muramyl-L-alanyl-D-glutamyl-($C_\alpha$)-n-butylester-($C_\gamma$)-L-alanin-2-(1,2-distearoyl-sn-glycero-3-hydroxyphosphoryloxy)-ethylamid-natriumsalz,

N-Acetyl-muramyl-L-alanyl-D-glutamyl-($C_\alpha$)-n-butylester-($C_\gamma$)-L-alanin-2-(1,2-dioleoyl-sn-glycero-3-hydroxyphosphoryloxy)-ethylamid-natriumsalz,

N-Benzoyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz

N-Glykolyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz und

N-Acetyl-1,4,6-0-triacetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz.

### Beispiel 3

Augentropfen

Zusammensetzung

| | |
|---|---|
| N-Acetyl-muramyl-L-α-aminobutyryl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (Verb. II) | 0,10 mg |
| Borsäure | 30,00 mg |
| Natriumtetraborat · $10H_2O$ | 0,10 mg |
| Benzalkoniumchlorid | 0,20 mg |
| Wasser zur Injektion | ad. 1,0 ml |

Herstellung

In einem Teil der obengenannten Menges des Wassers für Injektionszwecke werden unter aseptischen Bedingungen und unter Rühren Borsäure, Natriumtetraborat und Benzalkoniumchlorid bei Raumtemperatur gelöst. Anschliessend löst man in der so erhaltenen Lösung Verbindung II auf und ergänzt mit Wasser für Injektionszwecke auf das Endvolumen von 1,0 ml.

Die Lösung oder ein Teil oder Vielfaches davon wird durch einen Membranfilter filtriert und in gereinigte Behälter abgefüllt. Geeignete Behälter sind z. B.

— Behälter aus flexiblem Kunststoff (à 5 ml oder 10 ml) mit Tropfeinsatz,

— Behälter aus Glas (à 5 ml oder 10 ml) mit einer Tropfpipette aus Glas oder Kunststoff und einem elastomeren Pipettensauger oder

— Plastikeinmaldosispipetten (Inhalt 1-2 Tropfen).

### Beispiel 4

Nicht-wässerige Einzeldosis zur Nasenapplikation

Zusammensetzung

| | |
|---|---|
| N-Propionyl-desmethyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (Verb. III) | 0,03 mg |

Miglyol 812 ad. 30,00 mg

Herstellung

0,03 mg Verbindung III werden unter aseptischen Bedingungen in 29,97 mg Miglyol 812 gelöst. Diese Lösung wird in einen handelsüblichen Einmalnasenapplikator, z. B. eine gemäss US-Patent Nr. 3 739 951, abgefüllt, der vor der Anwendung auf eine Treibstoffdose aufgesetzt wird.

Beispiel 5

Nasentropfen

| Zusammensetzung | I | II |
|---|---|---|
| N-Acetyl-muramyl-L-valyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (Verb. IV) | 0,15 mg | 0,10 mg |
| Thiomersal | 0,02 mg | — |
| Natriummonohydrogenphosphat·2 $H_2O$ | 0,30 mg | 0,30 mg |
| Natriumdihydrogenphosphat·12 $H_2O$ | 10,10 mg | 10,10 mg |
| Benzalkoniumchlorid | — | 0,10 mg |
| Aethylendiamintetraessigsäure-dinatriumsalz (EDTA) | 0,50 mg | 0,50 mg |
| Natriumchlorid | 3,70 mg | 4,50 mg |
| Entmineralisiertes Wasser | 988,30 mg | 987,60 mg |
| pH-Wert: | 5,0 ± 0,3 | 5,0 ± 0,3 |
| Gefrierpunktserniedrigung Δt | – 0,51 °C | – 0,56 °C |

Herstellung

In einem Teil der obengenannten Menge von entmineralisiertem Wasser werden unter Rühren Natriumdihydrogenphosphat, Dinatriumhydrogenphosphat, Natriumchlorid, Thiomersal und EDTA-Dinatriumsalz bei Raumtemperatur gelöst.

In dieser Lösung löst man anschliessend Verbindung IV auf und ergänzt mit dem restlichen entmineralisierten Wasser.

Die Lösung oder ein Teil oder Vielfaches davon wird durch einen Membranfilter filtriert und in gereinigte Behälter abgefüllt. Geeignete Behälter sind z. B.

a) Glas oder Kunststoffbehälter (à 5 ml oder 10 ml) welche eine Pipette aus Glas oder Kunststoff mit einem elastomeren Pipettensauger besitzen,

b) Knautschflaschen aus Kunststoff mit einem Steigrohr und einem Sprühkopf aus Kunststoff,

c) Einzeldosisbehälter aus Kunststoff (Inhalt 2-3 Tropfen) oder

d) Glas oder Kunststoffflaschen, die mit einem normierten Pumpdosierspray aus Kunststoff versehen sind (ohne Treibgas).

Beispiel 6

Gel

Zusammensetzung

| | |
|---|---|
| N-Acetyl-muramyl-L-alanyl-D-glutamyl-($C_\alpha$)-n-butylester-($C_\gamma$)-L-alanin-2-(1,2-dilauroyl-sn-glycero-3-hydroxyphosphoryloxy)-ethylamid-natriumsalz (Verb. V) | 0,01 g |
| Glycerin 85 % | 10,00 g |
| Methylparaben | 0,12 g |
| Propylparaben | 0,03 g |
| Natriumcarboxymethylcellulose (hohe Viskosität) | 2,50 g |
| entmineralisiertes Wasser | 87,34 g |

Herstellung

Methyl- und Propylparaben werden in einem Teil des heissen entmineralisierten Wassers gelöst. Hierauf wird die Natriumcarboxymethylcellulose unter kräftigem Rühren in die erhaltene Lösung eingearbeitet. Man lässt den Schleim unter Rühren ausquellen. Nach dem Erkalten werden das Glycerin und eine Lösung des Wirkstoffs (Verbindung V) in das restliche Wasser hinzugefügt.

Beispiel 7

Crème

Zusammensetzung

| | |
|---|---|
| N-Acetyl-muramyl-L-alanyl-D-glutamyl-($C_\alpha$)-n-butylester-($C_\gamma$)-L-alanin-2-(1-palmitoyl-2-oleoyl-sn-glycero-3-hydroxyphosphoryloxy)-ethylamid-natriumsalz (Verb. VI) | 0,10 g |
| Sorbitanmonostearat | 0,60 g |
| Cetylalkohol | 3,00 g |
| Isopropylpalmitat | 2,00 g |
| Methylparaben | 0,12 g |
| Paraffinöl dickflüssig | 10,00 g |
| PEG (20)-Sorbitanmonostearat | 4,40 g |
| Propylparaben | 0,03 g |
| 70 %ige Lösung von kirstallinem Sorbit in entmineralisiertem Wasser | 6,00 g |
| Stearinsäure | 9,00 g |
| entmineralisiertes Wasser | 64,67 g |

Herstellung

Die Fettphase, bestehend aus Sorbitanmonostearat, Cetylalkohol, Stearinsäure, PEG (20)-Sorbitanmonostearat, Isopropylpalmitat und Paraffinöl, wird zusammengeschmolzen. Hierauf werden Methyl- und Propylparaben in einem Teil des heissen entmineralisierten Wassers gelöst. Der Wasserphase wird die Sorbitlösung hinzugefügt. Bei ca. 75 °C wird sodann unter Rühren die Wasserphase zur Fettphase gegeben. Die Crèmegrundlage wird hierauf unter Rühren abkühlen gelassen. Bei ca. 40 °C wird eine Lösung des Wirkstoffs (Verbindung VI) in dem restlichen Wasser der Crèmegrundlage zugesetzt.

Beispiel 8

Nasensalbe

Zusammensetzung

| | |
|---|---|
| N-Acetyl-muramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz (Verb. VII) | 0,03 g |
| Paraffinöl dickflüssig | 20,00 g |
| weisses Vaselin | 30,00 g |
| Wollfett, wasserfrei | 40,00 g |
| entmineralisiertes Wasser | 19,97 g |

Herstellung

Die Fettphase, bestehend aus Paraffinöl, Vaselin und Wollfett, wird zusammengeschmolzen. Die wässrige Lösung des Wirkstoffs wird bei ca. 50 °C in die Fettphase eingearbeitet.

Beispiel 9

Hautsalbe

Zusammensetzung

| | |
|---|---|
| N-Propionyl-desmethylmuramyl-L-alanyl-D-glutamyl-($C_\alpha$)-n-butylester-($C_\gamma$)-glycin-2-(1,2-dipalmitoyl-sn-glycero-3-hydroxyphosphoryloxy)-ethylamid-natriumsalz (Verb. VIII) | 0,25 g |
| Sorbitansesquioleat | 10,00 g |
| weisses Bienenwachs | 5,00 g |
| Cetylalkohol | 2,50 g |
| Methylparaben | 0,15 g |
| Paraffinöl dickflüssig | 20,00 g |
| Propylparaben | 0,02 g |
| Stearylalkohol | 2,50 g |
| weisses Vaselin | 40,00 g |
| entmineralisiertes Wasser | 19,58 g |

Herstellung

Die Fettphase, bestehend aus Sorbitansesquioleat, weissem Bienenwachs, Cetylalkohol, Paraffinöl, Stearylalkohol und weissem Vaselin, wird zusammengeschmolzen. Hierauf werden Methyl- und Propylparaben in der Hauptmenge des Wassers heiss gelöst. Bei ca. 80 °C wird die Wasserphase in die Fettphase eingearbeitet. Bei ca. 40 °C wird eine Lösung des Wirkstoffs (Verbindung VIII) in dem restlichen Wasser der so erhaltenen Salbengrundlage zugefügt.

## Beispiel 10

Lippenstift

Zusammensetzung

| | |
|---|---|
| N-Acetyl-muramyl-N-methyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz (Verb. IX) | 1,00 g |
| Polyethylenglykol mit dem mittleren Molekulargewicht 400 | 15,00 g |
| Polyethylenglykol mit dem mittleren Molekulargewicht 1000 | 83,00 g |
| Polyethylenglykol mit dem mittleren Mokelulargewicht 4000 | 1,00 g |

Herstellung

Der Wirkstoff wird in der Schmelze der Polyethylenglykole fein verteilt. Die zähflüssige Schmelze wird in geeignete Stifthülsen gegossen und erstarren gelassen.

## Beispiel 11

3,50 g (6,9 mMol) N-Acetyl-muramyl-L-alanyl-D-glutaminsäure-($C_\alpha$)-methylester und 1,20 g N-Hydroxysuccinimid werden in 50 ml eines Gemisches aus Dimethylformamid, Isopropanol und Chloroform (1 : 3 : 6 ; v/v) gelöst, 2,10 g (10,35 mMol) Dicyclohexylcarbodimid zugefügt und unter Ausschluss von Feuchtigkeit eine Stunde bei Raumtemperatur gerührt. Die Suspension wird mit 50 ml Essigsäureethylester versetzt, 30 Minuten im Eisbad gerührt und der unlösliche Dicyclohexylharnstoff abfiltriert. Das Filtrat wird am Rotationsverdampfer auf ca. 40 ml eingeengt, der Aktivester durch Zugabe von 150 ml abs. Diethylether ausgefällt (zweimal), abfiltriert und getrocknet, worauf man den Ester mit N-Hydroxysuccinimid mit $R_f$ = 0,43 (Chloroform : Methanol : Wasser = 70 : 30 : 5) erhält.

3,43 g (4,4 mMol) L-Alanin-2-(1,2-dipalmitoyl-sn-glycero-3-hydroxyphosphoryloxy)-ethylamid werden in 45 ml Chloroform suspendiert und bei 40° 0,86 ml (6,1 mMol) Triethylamin, gelöst in 5 ml Chloroform, innerhalb von fünf Minuten eingetropft, wobei eine klare Lösung entsteht. Dazu tropft man unter gutem Rühren unter Ausschluss von Feuchtigkeit innerhalb von fünf Minuten eine Lösung des oben beschriebenen Aktivesters in 100 ml eines Gemisches aus Dimethylformamid, Chloroform und Dioxan (1 : 14 : 6 ; v/v). Nach 21/2 Stunden Rühren bei Raumtemperatur wird die leicht trübe Lösung am Rotationsverdampfer (30°) zur Trockene verdampft. Das Rohprodukt wird an Kieselgel (60 reinst, Merck, Korngrösse : 70-230 mesh ASTM) im System Chloroform : Methanol : Wasser = 70 : 30 : 5 (5 ml Fraktionen) gereinigt. Die das Produkt enthaltenden Fraktionen (DC) werden gesammelt. Der nach dem Verdampfen des Lösungsmittels verbleibende Rückstand wird in 250 ml doppelt destilliertem Wasser gelöst und analog Beispiel 1 durch Diafiltration (AMICON-Rührzelle, Typ 402, Ultrafilter PM 30/76 mm ∅) gereinigt. Die in der Zelle verbleibende Lösung wird durch ein Millipore-Filter (0,2 μ) filtriert und lyophilisiert. Man erhält N-Acetyl-muramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz 2,96 Mol $H_2O$ als farbloses Pulver mit

$[\alpha]_D^{20} = +11 \pm 1°$ (c = 0,285 ; 10 %ige Essigsäure),

$R_f$ = 0,25 (Chloroform : Methanol : Wasser = 70 : 30 : 5) und

$R_f$ = 0,39 (Essigsäureethylester : n-Butanol : Pyridin : Essigsäure : Wasser = 42 : 21 : 21 : 6 : 10).

Die Ausgangsverbindung erhält man folgendermassen :

7,7 g (21,2 mMol) L-Ananyl-D-glutaminsäure-($C_\alpha$)-methylester-($C_\gamma$)-benzylester-hydrochlorid und 8,96 g (23,3 mMol) N-Acetyl-4,6-O-isopropyliden-muraminsäure-natriumsalz werden analog Beispiel 1 nach der Dicyclohexylcarbodiimid/N-Hydroxy-succinimid-Methode verknüpft. Das Rohprodukt wird ohne weitere Reinigung in 100 ml 60 %iger Essigsäure gespalten. Nach 5 1/2-stündigem Rühren bei Raumtemperatur wird die Lösung stark eingeengt, mit Wasser versetzt, wiederum eingeengt und nach Zugabe von 100 ml Dioxan lyophilisiert. Der Rückstand wird an 600 g Kieselgel 60 im System Chloroform : Methanol : Wasser = 70 : 30 : 5 (7 ml Fraktionen) gereinigt (zweimal).

Die das gewünschte Material enthaltenden Fraktionen werden vereinigt und das Lösungsmittel verdampft. Es verbleibt N-Acetyl-muramyl-L-alanyl-D-glutaminsäure-($C_\alpha$)-methylester-($C_\gamma$)-benzylester als farbloser Schaum mit $R_f$ = 0,63 (Chloroform : Methanol : Wasser = 70 : 30 : 5).

6,0 g (10 mMol) N-Acetyl-muramyl-L-alanyl-D-glutaminsäure-($C_\alpha$)-methylester-($C_\gamma$)-benzylester, gelöst in einem Gemisch aus 1,2-Dimethoxyethan und Wasser 95 : 5 (v/v), werden in Gegenwart von 0,6 g eines Palladium-auf-Kohle-Katalysators (10 %ig) während 1 1/2 Stunden bei Normaldruck hydriert. Der Katalysator wird abfiltriert und das Filtrat bei Raumtemperatur unter vermindertem Druck eingedampft. Der Rückstand wird an 400 g Kieselgel 60 im System Chloroform : Methanol : Wasser = 70 : 30 : 5 (10 ml Fraktionen) wie oben gereinigt. Die das Produkt enthaltenden Fraktionen werden vereinigt und das Lösungsmittel verdampft. Der verbleibende Rückstand, teilweise in Form des Na-Salzes, wird analog wie im Beispiel 1 an 50 ml DOWEX 50WX8 (50/100 mesh, H-Form, starksaurer Kationenaustauscher) entsalzt. Das Filtrat wird durch ein Millipore-Filter (0,45 μ) filtriert und lyophilisiert. Man erhält N-Acetyl-muramyl-L-alanyl-D-glutaminsäure-($C_\alpha$)-methylester (1,27 Mol $H_2O$) als farbloses Pulver mit

$[\alpha]_D^{20} = +47 \pm 1°$ (c = 1,249 ; Methanol),
$R_f$ = 0,08 (Chloroform : Methanol : Wasser = 70 : 30 : 5),
$R_f$ = 0,30 (Acetonitril : Wasser = 3 : 1) und
$R_f$ = 0,43 (Essigsäureethylester : Essigsäure : Wasser : Methanol = 67 : 10 : 23 : 12).

Beispiel 12

9,4 g (11,1 mMol) N-Acetyl-muramyl-L-alanyl-D-glutaminsäure-($C_\alpha$)-tert.-butylester werden analog Beispiel 11 in den Aktivester übergeführt und dann, wie dort beschrieben, mit 6,48 g (8,2 mMol) L-Alanin-2-(1,2-dipalmitoyl-sn-glycero-3-hydroxyphosphoryloxy)-ethylamid in Gegenwart von Triethylamin verknüpft. Das Rohprodukt wird in 250 ml doppelt destilliertem Wasser suspendiert und kurz auf 37 °C erwärmt. Man rührt 15 Minuten im Eisbad, filtriert den unlöslichen Dicyclohexylharnstoff ab und unterwirft das Filtrat der in Beispiel 1 beschriebenen Diafiltration (AMICON-Rührzelle 402, Ultrafilter PM 30/76 mm Ø). Die in der Zelle verbleibende Lösung (120 ml) wird lyophilisiert und das Produkt an 550 g Kieselgel 60 wie üblich chromatographiert. Das gesuchte Material wird gesammelt, in 200 ml doppelt destilliertem Wasser aufgenommen (pH = 7), sterilfiltriert (0,45 μ) und lyophilisiert. Man erhält N-Acetyl-muramyl-L-alanyl-D-glutamyl-($C_\alpha$)-tert.-butylester-($C_\gamma$)-L-alanin-2-(1,2-dipalmitoyl-sn-glycero-3-hydroxyphosphoryloxy)-ethylamid-natriumsalz als farbloses Pulver, enthaltend 3,4 Mol Wasser, mit

$[\alpha]_D^{20} = +14 \pm 1°$ (c = 0,817 ; 10 %ige Essigsäure),
$R_f$ = 0,29 (Chloroform : Methanol : Wasser = 70 : 30 : 5) und
$R_f$ = 0,53 (Essigsäureethylester : n-Butanol : Pyridin : Essigsäure : Wasser = 42 : 21 : 21 : 6 : 10).

Das als Ausgangsmaterial benötigte Muramyldipeptidderivat erhält man analog Beispiel 1 durch Verknüpfung von N-Acetyl-4,6-O-isopropyliden-muraminsäure-natriumsalz und L-Alanyl-D-glutaminsäure-($C_\alpha$)-tert.-butylester-($C_\gamma$)-benzylester nach der Dicyclohexylcarbodiimid/N-Hydroxysuccinimid-Methode. Nach saurer Abspaltung des Isopropylidenrestes mit 60 %iger Essigsäure und nach Entfernung des Benzylrestes durch katalytische Hydrierung analog Beispiel 1 erhält man N-Acetyl-muramyl-L-alanyl-D-glutaminsäure-($C_\alpha$)-tert.-butylester als farbloses Pulver mit

$[\alpha]_D^{20} = +44 \pm 1°$ (c = 1,004 ; Methanol),
$R_f$ = 0,20 (Chloroform : Methanol : Wasser = 70 : 30 : 5) und
$R_f$ = 0,41 (Acetonitril : Wasser = 3 : 1).

Das als Ausgangsmaterial benötigte Dipeptidderivat erhält man folgendermassen :

38,3 g (63,5 mMol) N-[2-(4-Biphenylyl)-propyloxycarbonyl]-L-alanyl-D-glutaminsäure-($C_\alpha$)-tert.-butylester-($C_\gamma$)-benzylester werden in 700 ml eines Gemisches aus Trifluorethanol und Wasser = 9 : 1 (v/v) gelöst und durch tropfenweise Zugabe von 1,23 normaler Salzsäure im gleichen Lösungsmittelgemisch (1 Volumteil konz. Salsäure und 9 Volumteile Trifluorethanol) wird der « pH »-Wert auf 1,5 gesenkt (2 1/2 Stunden, Verbrauch 51,66 ml, d. h. 81 % d. Th.). Die schwach gelbliche Lösung wird am Rotationsverdampfer im Wasserstrahlvakuum bei 30° auf knapp 100 ml eingeengt, 200 ml Dioxan zugefügt und das Ganze gefriergetrocknet. Der halbfeste Rückstand wird in 30 ml abs. Diethylether aufgenommen und bei 0 bis 5° unter Rühren durch Zugabe von 150 ml Petrolether ausgefällt. Man lässt in der Kälte (— 20°) stehen, dekantiert ab und wiederholt dieselbe Prozedur noch zweimal. Der ölige Rückstand wird schliesslich in 100 ml tert.-Butanol aufgenommen, lyophilisiert und der Rückstand über Natronasbest (Merck) im Hochvakuum getrocknet.

Man erhält L-Alanyl-D-glutaminsäure-($C_\alpha$)-tert.-butylester-($C_\gamma$)-benzylester-hydrochlorid als stark hygroskopisches Pulver mit

$[\alpha]_D^{20} = +7 \pm 1°$ (c = 1,630 ; Chloroform),
$R_f$ = 0,60 (Chloroform : Methanol : Wasser = 70 : 30 : 5) und
$R_f$ = 0,45 (n-Butanol : Essigsäure : Wasser = 10 : 1 : 2,8).

Die geschützte Verbindung erhält man wie folgt :

Zu einer auf 0° abgekühlten Lösung von 65,96 g (0,2 Mol) D-Glutaminsäure-($C_\alpha$)-tert.-butylester-($C_\gamma$)-benzylester-hydrochlorid und 65,47 g (0,2 Mol) N-[2-(4-Biphenylyl)-propyloxycarbonyl]-L-alanin in 650 ml abs. Dimethylformamid gibt man unter Rühren 22 ml (0,24 Mol) N-Methylmorpholin und schliesslich 59,11 g (0,24 Mol) 2-Ethoxy-N-ethoxycarbonyl-1,2-dihydro-chinolin (EEDQ). Nach zehnstündigem Rühren bei Raumtemperatur wird die gelbe Lösung am Rotationsverdampfer bei 30° eingedampft. Der Rückstand wird in 1 Liter Essigsäureethylester aufgenommen, fünfmal mit je 200 ml Wasser extrahiert, die Wasserphase mit 0,5 l Essigester rückextrahiert und die vereinigten organischen Phasen getrocknet. Das

nach dem Verdampfen des Lösungsmittels anfallende Rohprodukt (150 g) wird durch mehrfache Chromatographie an Kieselgel 60 (1 : 20) mit Essigester gereinigt. Man erhält N-[2-(4-Biphenylyl)-propyloxycarbonyl-L-alanyl-D-glutaminsäure-($C_\alpha$)-tert.-butylester-($C_\gamma$)-benzylester als farbloses Oel mit
$[\alpha]_D^{20}$ = — 14 ± 1° (c = 1,625 ; Essigsäureethylester),
$R_f$ = 0,73 (Chloroform : Isopropanol : Essigsäure = 70 : 8 : 2) und
$R_f$ = 0,51 (Toluol : Essigester = 1 : 1).

Das als Ausgangsmaterial dienende D-Glutaminsäure-($C_\alpha$)-tert.-butylester-($C_\gamma$)-benzylester-hydrochlorid wird analog dem in der Literatur [R. Roeske, J. Org. Chem. *28,* 1251 (1963)] beschriebenen L-Derivat durch Umsatz von D-Glutaminsäure-($C_\gamma$)-benzylester mit Isobuten in einer Mischung aus 1,4-Dioxan und Schwefelsäure in Form von farblosen Nadeln mit Schmelzpunkt 108-109° erhalten,
$[\alpha]_D^{20}$ = — 16 + 1° (c = 1,235 ; Ethanol),
$R_f$ = 0,84 (Chloroform : Methanol : Wasser = 70 : 30 : 5) und
$R_f$ = 0,64 (Essigsäureethylester : n-Butanol : Pyridin : Essigsäure : Wasser = 42 : 21 : 21 : 6 : 10).

Beispiel 13

Zu einer Lösung von 4,62 g (6,05 mMol) L-Alanin-2-(1,2-dipalmitoyl-sn-glycero-3-hydroxyphosphoryloxy)-ethylamid in 100 ml eines Gemisches aus Chloroform : Isopropanol : Wasser = 70 : 30 : 2 werden unter Rühren bei 15° innerhalb von 10 Minuten 14,52 ml (7,26 mMol) einer 0,5 molaren Triethylaminlösung in einem Gemisch aus Chloroform : Isopropanol : Wasser = 70 : 30 : 2 zugetropft.

Danach werden in 20 Minuten 6,0 g (7,26 mMol) N-Propionyl-desmethylmuramyl-L-alanyl-D-glutaminsäure-($C_\alpha$)-methylester-($C_\gamma$)-N-hydroxysuccinimidester, der noch etwas Dicyclohexylharnstoff enthält, in 4 Portionen à 1,5 g in fester Form zugegeben, wobei die Lösung zusehends trüber wird.

Nach ca. 15 minütigem Rühren bei 15° wird die Kühlung entfernt und 2 1/2 Stunden bei Raumtemperatur weitergerührt.

Anschliessend wird die so erhaltene trübe Reaktionslösung im Vakuum bei 30° eingedampft. Das Rohprodukt (10,75 g) wird danach in 170 ml bidestilliertem Wasser suspendiert (pH 5,5), die Suspension durch Zugabe von 0,3 ml Triethylamin auf pH 6,0 gestellt, vom Unlöslichen (Dicyclohexylharnstoff) abfiltriert und das Filtrat im Hochvakuum lyophilisiert.

Das so erhaltene Lyophilisat wird in 300 ml bidestilliertem Wasser gelöst und die Lösung in einer AMICON-Dialysierzelle (Modell 402, Ultrafilter PM 10/76 mm Ø) bei 3 Atü (Atmosphären Ueberdruck) bis auf 75 ml eingeengt, dann gegen 250 ml Phosphatpuffer : Natriumchlorid (je 0,1 molar, 1 : 1, pH 7) und 1 750 ml bidestilliertes Wasser chloridfrei filtriert. Die in der Zelle zurückbleibende Lösung (ca. 75 ml) wird anschliessend nacheinander durch zwei Milliporefilter (NALGENE S, 0,45 μ und 0,2 μ) filtriert und die so erhaltene klare, farblose Lösung im Hochvakuum lyophilisiert.

Man erhält 7,36 g noch verunreinigtes N-Propionyl-desmethylmuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz als farbloses Pulver, das durch CRAIG-Verteilung mit Tetrachlorkohlenstoff : Chloroform : Methanol : 0,5 prozentige Natriumchloridlösung = 7 : 17 : 18 : 6 weiter gereinigt wird. Nach 547 Stufen befindet sich das gewünschte Produkt in den Fraktionen 160-220, K = 0,58. Die das Produkt enthaltenden Fraktionen werden vereinigt und im Hochvakuum ohne zu Heizen (Abkühlung) zur Trockne eingedampft. Man erhält ein farbloses Pulver, das noch Natriumchlorid enthält. Zur Entfernung desselben wird das Produkt in 600 ml bidestilliertem Wasser gelöst und in einer AMICON-Dialysierzelle (Modell 402, Ultrafilter PM 30/76 mm Ø) bei 3 Atü zuerst auf ca. 50 ml Innenvolumen, dann mit insgesamt 1 750 ml bidestilliertem Wasser chloridfrei filtriert.

Die in der Zelle zurückbleibende Lösung (ca. 50 ml) wird anschliessend nacheinander durch zwei Milliporefilter (NALGENE S, 0,45 μ bzw. 0,2 μ) sterilfiltriert und im Hochvakuum lyophilisiert.

Man erhält N-Propionyl-desmethylmuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz als farbloses Pulver, das 1,90 Mol Wasser enthält, mit
$[\alpha]_D^{20}$ = + 3,7 ± 0,1° (c = 0,672 ; Chloroform),
$[\alpha]_D^{20}$ = + 1,7 ± 0,1° (c = 1,044 ; Wasser),
$R_f$ = 0,42 (Chloroform : Methanol : Wasser = 70 : 30 : 5) und
$R_f$ = 0,62 (Essigester : n-Butanol : Pyridin : Essigsäure : Wasser = 42 : 21 : 21 : 6 : 10).

Das Ausgangsmaterial erhält man folgendermassen :

5,12 g (10,1 mMol) N-Propionyl-desmethylmuramyl-L-alanyl-D-glutaminsäure-α-methylester werden in 100 ml eines Gemisches aus Chloroform : Isopropanol = 7 : 3 aufgenommen und durch Zugabe von 10 ml Dimethylformamid vollständig gelöst.

Zu dieser Lösung werden 2,68 g (13 mMol) Dicyclohexylcarbodiimid und 1,50 g (13 mMol) N-Hydroxysuccinimid hinzugefügt und die so erhaltene klare, farblose Lösung 1 1/2 Stunden bei Raumtemperatur gerührt und anschliessend 17 Stunden bei 4° stehen gelassen.

Die so erhaltene Suspension wird mit 400 ml Diethylether versetzt und danach noch 1 Stunde bei Raumtemperatur gerührt.

Anschliessend wird das Kristallisat abgesaugt, mit Diethylether nachgewaschen und im Vakuum über Phosphorpentoxid getrocknet.

Man erhält rohen N-Propionyl-desmethylmuramyl-L-alanyl-D-glutaminsäure-($C_\alpha$)-methylester-($C_\gamma$)-N-hydroxysuccinimidester in Form farbloser Kristalle, die hauptsächlich noch etwas Dicyclohexylharnstoff enthalten und ohne weitere Reinigung verwendet werden, mit

$R_f$ = 0,46 (Chloroform : Methanol = 5 : 1) und
$R_f$ = 0,72 (Chloroform : Methanol = 7 : 3).

Das Ausgangsmaterial erhält man folgendermassen :

Eine Lösung von 13,0 g (21,75 mMol) N-Propionyl-desmethylmuramyl-L-alanyl-D-glutaminsäure-($C_\alpha$)-methylester-($C_\gamma$)-benzylester in 250 ml eines Gemisches aus Dimethoxyethan : Wasser = 20 : 1 wird mit 2,5 g 10 prozentiger Palladiumkohle als Katalysator 1 Stunde bei Raumtemperatur und Normaldruck hydriert.

Danach wird vom Katalysator abfiltriert, das Filtrat im Vakuum zur Trockne eingedampft und der so erhaltene Rückstand dreimal in je 50 ml Wasser aufgenommen und erneut eingedampft.

Man löst erneut in ca. 100 ml bidestilliertem Wasser, filtriert diese Lösung durch ein Milliporefilter (NALGENE S, 0,2 µ) und lyophilisiert im Hochvakuum.

Man erhält N-Propionyl-desmethylmuramyl-L-alanyl-D-glutaminsäure-α-methylester (0,68 Mol Wasser) als farbloses Lyophilisat mit

$[\alpha]_D^{20} = +14{,}9 \pm 0{,}1°$ (c = 1,067 ; Methanol),
$R_f$ = 0,78 (Chloroform : Methanol : Wasser = 70 : 30 : 5) und
$R_f$ = 0,57 (Essigester : n-Butanol : Pyridin : Essigsäure : Wasser = 42 : 21 : 21 : 6 : 10).

Das Ausgangsmaterial erhält man wie folgt :

Eine Lösung von 20,7 g (32,46 mMol) 4,6-O-Isopropyliden-N-propionyl-desmethylmuramyl-L-alanyl-D-glutaminsäure-($C_\alpha$)-methylester-($C_\gamma$)-benzylester in 400 ml 60 %iger Essigsäure wird 21 Stunden bei Raumtemperatur gerührt, dann bei 30° im Vakuum eingedampft und der so erhaltene Rückstand nacheinander dreimal mit je 100 ml Wasser versetzt und erneut eingedampft. Das Rohprodukt wird anschliessend durch Säulenchromatographie an 1 000 g Kieselgel (60 reinst, Merck, 0,063-0,200 mm) im System Methylenchlorid : Methanol = 85 : 15 gereinigt (15 ml Fraktionen). Die Fraktionen 207-290 werden vereinigt und im Vakuum bei 30° eingedampft.

Man erhält N-Propionyl-desmethylmuramyl-L-alanyl-D-glutaminsäure-($C_\alpha$)-methylester-($C_\gamma$)-benzylester, der 0,24 Mol Wasser enthält, mit

$[\alpha]_D^{20} = +16{,}1 \pm 0{,}1°$ (c = 1,463 ; Methanol),
$R_f$ = 0,24 (Chloroform : Methanol = 9 : 1),
$R_f$ = 0,55 (Chloroform : Methanol = 5 : 1 ) und
$R_f$ = 0,94 (Chloroform : Methanol : Wasser = 70 : 30 : 5).

Das Ausgangsmaterial erhält man folgendermassen :

In eine Suspension von 13,2 g (35,77 mMol) 4,6-O-Isopropyliden-N-propionyl-desmethylmuraminsäure-natriumsalz in 200 ml Dimethyl-formamid gibt man bei Raumtemperatur unter Rühren 9,6 g (46,5 mMol) Dicyclohexylcarbodiimid, 5,35 g (46,5 mMol) N-Hydroxysuccinimid und 12,8 g (35,77 mMol) L-Alanyl-D-glutaminsäure-($C_\alpha$)-methylester-($C_\gamma$)-benzylester-hydrochlorid und rührt das Ganze 21 Stunden bei Raumtemperatur.

Die so erhaltene weisse Suspension wird mit 200 ml Essigester versetzt, 1 Stunde bei 0° gerührt, vom Kristallisat (Dicyclohexylharnstoff) abgenutscht, mit eiskaltem Essigester nachgewaschen und das Filtrat im Vakuum bei 30° eingedampft.

Der so erhaltene Rückstand wird in 300 ml Essigester aufgenommen und zweimal nacheinander mit je 50 ml 2 N Zitronensäure, Wasser, 10 %iger Natriumhydrogencarbonatlösung und Wasser gewaschen.

Die Essigesterphasen werden vereinigt, über Natriumsulfat getrocknet, filtriert und im Vakuum eingedampft.

Man erhält 4,6-O-Isopropyliden-N-propionyl-desmethylmuramyl-L-alanyl-D-glutaminsäure-($C_\alpha$)-methylester-($C_\alpha$)-benzylester mit

$R_f$ = 0,65 (Chloroform : Methanol = 9 : 1) und
$R_f$ = 0,86 (Chloroform : Methanol = 5 : 1).

Die beiden Ausgangsprodukte erhält man wie folgt :

Zu einer Lösung von 20,3 g (48,0 mMol) N-tert.-Butyloxycarbonyl-L-alanyl-D-glutaminsäure-($C_\alpha$)-methylester-($C_\gamma$)-benzylester [P. Lefrancier, M. Derrien, I. Lederman, F. Nief, J. Choay und E. Lederer, Int. J. Peptide Protein Res. *11*, 289-296 (1978)] in 100 ml absolutem Essigester gibt man bei 0° 100 ml einer ca. 5 N Salzsäure in absolutem Essigester und rührt 2 Stunden bei 0°.

Die gelbliche Lösung wird dann im Vakuum eingedampft, der so erhaltene Rückstand zweimal in je 100 ml absolutem Essigester aufgenommen und erneut eingedampft. Nach Digerieren mit zweimal je 100 ml absolutem Diethylether trocknet man den Rückstand im Hochvakuum und erhält L-Alanyl-D-glutaminsäure-($C_\alpha$)-methylester-($C_\gamma$)-benzylester-hydrochlorid, das 0,31 Mol Wasser enthält, mit

$[\alpha]_D^{20} = + 18{,}6 \pm 0{,}1°$ (c = 0,043 ; Methanol),
$R_f$ = 0,72 (Chloroform : Methanol : Wasser = 70 : 30 : 5),
$R_f$ = 0,44 (Chloroform : Methanol = 5 : 1) und
$R_f$ = 0,31 (Chloroform : Methanol = 9 : 1).

Eine Lösung von 34,4 g (73,8 mMol, 2,14 mMol/g, enthält noch Natriumchlorid) 1α-O-Benzyl-4,6-O-isopropyliden-N-propionyl-desmethyl-muraminsäure-natriumsalz in 340 ml Wasser wird bei konstantem pH-Wert von 7,1 mit 6,0 g 10 %iger Palladiumkohle als Katalysator 23 Stunden bei Raumtemperatur und Normaldruck hydriert.

Danach wird vom Katalysator abfiltriert, das Filtrat bei pH 7,1 im Vakuum bei 30° eingedampft und der so erhaltene Rückstand im Hochvakuum über Phosphorpentoxid getrocknet.

Man erhält 4,6-O-Isopropyliden-N-propionyl-desmethylmuraminsäure-natriumsalz als farbloses Pulver mit
$R_f$ = 0,50 (Chloroform : Methanol : Wasser = 70 : 30 : 5) und
$R_f$ = 0,66 (Acetonitril : Wasser = 3 : 1).

Das Ausgangsmaterial erhält man wie folgt :

Eine Lösung von 14,3 g (32,7 mMol) 1α-O-Benzyl-4,6-O-isopropyliden-N-propionyl-desmethylmuraminsäure-methylester in 130 ml Methanol wird mit 24,4 ml (48,7 mMol) 2N Natronlauge versetzt und 1 1/2 Stunden bei Raumtemperatur gerührt.

Danach wird die so erhaltene klare, schwach gelbe Lösung mit 1N Salzsäure auf pH = 7,0 gestellt und im Vakuum bei 30° eingedampft.

Nach Trocknen über Phosphorpentoxid erhält man 1α-O-Benzyl-4,6-O-isopropyliden-N-propionyl-desmethylmuraminsäure-natriumsalz in Form farbloser Kristalle mit
$R_f$ = 0,67 (Chloroform : Methanol : Wasser = 70 : 30 : 5) und
$R_f$ = 0,74 (Acetonitril : Wasser = 3 : 1).

Das Ausgangsprodukt erhält man wie folgt :

Zu einer Suspension von 6,75 g (224,7 mMol) Natriumhydrid in 120 ml absolutem Tetrahydrofuran tropft man bei 5° unter Stickstoff und Rühren innerhalb von 5 Minuten eine Lösung von 31,3 g (85,65 mMol) 1α-O-Benzyl-2-desoxy-4,6-O-isopropyliden-2-propionamido-α-D-glucopyranosid in 200 ml absolutem Tetrahydrofuran, wobei die Temperatur auf 20° steigt.

Die so erhaltene Suspension wird noch zwei Stunden bei 40° gerührt, dann auf 0° abgekühlt und unter Stickstoff und Rühren innerhalb von 30 Minuten zu einer auf — 15° abgekühlten Lösung von 20,7 g (135,77 mMol) Bromessigsäuremethylester in 95 ml absolutem Tetrahydrofuran zugetropft.

Die so erhaltene Suspension wird noch 3 Stunden bei 0-5° gerührt, dann werden 20 ml Methanol und 20 ml Tetrahydrofuran zugegeben. Man stellt mit 4,5 ml Eisessig auf pH = 6 ein und dampft im Vakuum bei 30°. ein.

Den Rückstand löst man anschliessend in 200 ml Methylenchlorid und wäscht die so erhaltene Lösung dreimal mit je 125 ml Wasser. Die Methylenchloridphasen werden vereinigt, über Natriumsulfat getrocknet, filtriert und im Vakuum bei 30° eingedampft.

Man erhält ein Rohprodukt in Form schwach gelber Kristalle, die durch Säulenchromatographie an 500 g neutralem Aluminiumoxid (Woelm N, Super 1) mit Essigester als Elutionsmittel (10 ml Fraktionen) gereinigt werden. Die Fraktion 14-36 werden vereinigt und im Vakuum eingedampft.

Man erhält 1α-O-Benzyl-4,6-O-isopropyliden-N-propionyl-desmethyl-muraminsäure-methylester in Form farbloser Kristalle vom Schmelzpunkt 121-123° (aus Diethylether : Petrolether = 1 : 2) mit
$[\alpha]_D^{20} = + 146{,}9 \pm 0{,}1°$ (c = 0,849 ; Chloroform) und
$R_f$ = 0,67 (Chloroform : Methanol = 9 : 1).

## Beispiel 14

Analog Beispiel 13 erhält man aus 5,525 g (6,43 mMol) rohem (enthält noch etwas Dicyclohexylharnstoff) N-Propionyl-desmethyl-muramyl-L-alanyl-D-glutaminsäure-($C_\alpha$)-tert.-butylester-($C_\gamma$)-N-hydroxysuccinimidester und 3,78 g (4,95 mMol) L-Alanin-2-(1,2-dipalmitoyl-sn-glycero-3-hydroxyphosphoryloxy)-ethylamid N-Propionyl-desmethyl-muramyl-L-alanyl-D-glutamyl-($C_\alpha$)-tert.-butylester-($C_\gamma$)-L-alanin-2-(1,2-dipalmitoyl-sn-glycero-3-hydroxyphosphoryloxy)-ethylamid-natriumsalz als farbloses Pulver, das 2,64 Mol Wasser enthält, mit
$[\alpha]_D^{20} = + 2{,}5 \pm 0{,}1°$ (c = 0,649 ; Wasser),
$[\alpha]_D^{20} = + 5{,}8 \pm 0{,}1°$ (c = 0,694 ; Chloroform) und
$R_f$ = 0,60 (Chloroform : Methanol : Wasser = 70 : 30 : 5).

Das Ausgangsmaterial erhält man wie folgt :

Analog Beispiel 13 erhält man aus 3,44 g (6,15 mMol) N-Propionyl-desmethylmuramyl-L-alanyl-D-glutaminsäure-α-tert.-butylester, 1,68 g (8,14 mMol) Dicyclohexylcarbodiimid und 0,94 g (8,14 mMol) N-Hydroxysuccinimid rohen N-Propionyl-desmethylmuramyl-L-alanyl-D-glutaminsäure-($C_\alpha$)-tert.-buty-

lester-($C_\gamma$)-N-hydroxysuccinimidester in Form farbloser Kristalle, die hauptsächlich noch etwas Dicyclohexylharnstoff enthalten und ohne weitere Reinigung verwendet werden, mit $R_f$ = 0,61 (Chloroform : Methanol : Wasser = 70 : 30 : 5).

Das Ausgangsmaterial erhält man wie folgt :

Eine Lösung von 5,1 g (7 mMol) 1α-O-Benzyl-N-propionyl-desmethyl-muramyl-L-alanyl-D-glutaminsäure-($C_\alpha$)-tert.-butylester-($C_\gamma$)-benzyl-ester in 100 ml eines Gemisches aus Dimethoxyethan : Wasser = 9 : 1 wird mit 1,5 g 5 %iger Palladiumkohle (E 101 N, Degussa) als Katalysator 20 Stunden bei Raumtemperatur und Normaldruck hydriert. Nach 20 Stunden wird vom Katalysator abfiltriert und dann erneut wie oben mit 1,5 g frischem Katalysator 26 Stunden weiterhydriert. Danach wird wieder vom Katalysator abfiltriert und das Filtrat im Vakuum bei 30° zur Trockne eingedampft.

Der so erhaltene Rückstand wird in einem Gemisch aus 100 ml Methylenchlorid und 5 ml Isopropanol gelöst, die Lösung mit 500 ml eines Gemisches aus Diethylether : Petrolether = 3 : 2 versetzt und noch 1 Stunde bei Raumtemperatur gerührt.

Anschliessend saugt man das ausgefallene Produkt ab und wäscht mit Diethylether nach. Man erhält N-Propionyl-desmethylmuramyl-L-alanyl-D-glutaminsäure-α-tert.-butylester in Form eines farblosen Pulvers, das 0,52 Mol Wasser enthält, mit

$[\alpha]_D^{20} = +20,2 \pm 0,1°$ (c = 0,902 ; Wasser),
$R_f$ = 0,32 (Chloroform : Methanol : Wasser = 70 : 30 : 5) und
$R_f$ = 0,54 (Acetonitril : Wasser = 3 : 1).

Das Ausgangsmaterial erhält man folgendermassen :

In analoger Weise wie in Beispiel 13 erhält man aus 7,3 g (9,5 mMol) 1α-O-Benzyl-4,6-O-isopropyliden-N-propionyl-desmethylmuramyl-L-alanyl-D-glutaminsäure-($C_\alpha$)-tert.-butylester-($C_\gamma$)-benzylester und 150 ml 60 %iger Essigsäure 1α-O-Benzyl-N-propionyl-desmethylmuramyl-L-alanyl-D-glutaminsäure-($C_\alpha$)-tert.-butylester-($C_\gamma$)-benzylester in Form von farblosen Kristallen vom Schmelzpunkt 152-153° (aus Methanol : Wasser = 1 : 5) mit

$[\alpha]_D^{20} = +68,9 \pm 0,1°$ (c = 0,991 ; Methanol),
$R_f$ = 0,40 (Chloroform : Methanol = 9 : 1) und
$R_f$ = 0,70 (Acetonitril : Wasser = 3 : 1).

Das Ausgangsprodukt erhält man wie folgt :

Analog Beispiel 13 erhält man aus 4,65 g (9,62 mMol, 2,066 mMol/g, enthält noch Natriumchlorid) 1α-O-Benzyl-4,6,O-isopropyliden-N-propionyl-desmethylmuraminsäure-natriumsalz, 2,38 g (11,54 mMol) Dicyclohexylcarbodiimid, 1,33 g (11,54 mMol) N-Hydroxysuccinimid und 3,86 g (9,62 mMol) L-Alanyl-D-glutaminsäure-($C_\alpha$)-tert.-butylester-($C_\gamma$)-benzyl-ester-hydrochlorid 1α-O-Benzyl-4,6-O-isopropyliden-N-propionyl-desmethylmuramyl-L-alanyl-D-glutaminsäure-($C_\alpha$)-tert.-butylester-($C_\gamma$)-benzylester, der 0,31 Mol Wasser enthält, mit

$[\alpha]_D^{20} = +38,4 \pm 0,1°$ (c = 1,086 ; Methylenchlorid),
$R_f$ = 0,86 (Acetonitril : Wasser = 3 : 1) und
$R_f$ = 0,83 (Chloroform : Methanol = 9 : 1).

## Beispiel 15

Analog Beispiel 13 erhält man aus 3,07 g (4 mMol) rohem (enthält noch etwas Dicyclohexylharnstoff) N-Propionyl-desmethyl-muramyl-L-alanyl-D-glutaminsäure-($C_\alpha$)-n-butylester-($C_\gamma$)-N-hydroxy-succinimidester und 2,37 g (3,1 mMol) L-Alanin-2-(1,2-dipalmitoyl-sn-glycero-3-hydroxyphosphoryloxy)-ethylamid N-Propionyl-desmethyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz als farbloses Pulver, das 2,93 Mol Wasser enthält, mit

$[\alpha]_D^{20} = +6,9 \pm 0,1°$ (c = 0,504 ; Methylenchlorid),
$R_f$ = 0,65 (Chloroform : Methanol : Wasser = 70 : 30 : 5) und
$R_f$ = 0,63 (Chloroform : Methanol = 7 : 3).

Das Ausgangsmaterial erhält man folgendermassen :

Analog Beispiel 13 erhält man aus 2,2 g (3,9 mMol) N-Propionyl-desmethylmuramyl-L-alanyl-D-glutaminsäure-α-n-butylester, 1,2 g (5,8 mMol) Dicyclohexylcarbodiimid und 0,69 g (5,8 mMol) N-Hydroxysuccinimid rohen N-Propionyl-desmethylmuramyl-L-alanyl-D-glutaminsäure-($C_\alpha$)-n-butylester-($C_\gamma$)-N-hydroxysuccinimidester in Form farbloser Kristalle, die hauptsächlich noch etwas Dicyclohexylharnstoff enthalten und ohne weitere Reinigung verwendet werden, mit $R_f$ = 0,66 (Chloroform : Methanol : Wasser = 70 : 30 : 5).

Das Ausgangsmaterial erhält man folgendermassen :

Analog Beispiel 14 (Hydrierdauer 20 Minuten) erhält man aus 7,3 g (11,3 mMol) N-Propionyl-desmethylmuramyl-L-alanyl-D-glutaminsäure-($C_\alpha$)-n-butylester-($C_\gamma$)-benzylester N-Propionyl-desmethylmuramyl-L-alanyl-D-glutaminsäure-α-n-butylester als farbloses Pulver, das 0,88 Mol Wasser

enthält, mit
$[\alpha]_D^{20} = + 16,4 \pm 0,1°$ (c = 0,959 ; Wasser),
$R_f = 0,61$ (Methylenchlorid : Methanol : Wasser = 70 : 30 : 5) und
$R_f = 0,52$ (Acetonitril : Wasser = 3 : 1).

Das Ausgangsmaterial erhält man wie folgt :
In analoger Weise wie in Beispiel 13 erhält man aus 9,5 g (13,97 mMol) 4,6-O-Isopropyliden-N-propionyl-desmethylmuramyl-L-alanyl-D-glutaminsäure-($C_\alpha$)-n-butylester-($C_\gamma$)-benzylester und 120 ml 60 %iger Essigsäure N-Propionyl-desmethylmuramyl-L-alanyl-D-glutaminsäure-($C_\alpha$)-n-butylester-($C_\gamma$)-benzylester, der 0,41 Mol Wasser enthält, mit
$[\alpha]_D^{20} = + 15,7 \pm 0,1°$ (c = 1,019 ; Methanol),
$R_f = 0,23$ (Methylenchlorid : Methanol = 9 : 1),
$R_f = 0,45$ (Methylenchlorid : Methanol = 5 : 1) und
$R_f = 0,82$ (Methylenchlorid : Methanol : $H_2O$ = 70 : 30 : 5).

Das Ausgangsprodukt erhält man folgendermassen :
In analoger Weise wie in Beispiel 13 erhält man aus 3,81 g (10,76 mMol), 2,72 mMol/g, enthält noch Natriumchlorid) 4,6-O-Isopropyliden-N-propionyl-desmethylmuraminsäure-natriumsalz, 2,44 g (11,83 mMol) Dicyclohexylcarbodiimid, 1,36 g (11,83 mMol) N-Hydroxysuccinimid und 4,3 g (10,76 mMol) L-Alanyl-D-glutaminsäure-($C_\alpha$)-n-butylester-($C_\gamma$)-benzylester-hydrochlorid, 4,6-O-Isopropyliden-N-propionyl-desmethyl-muramyl-L-alanyl-D-glutaminsäure-($C_\alpha$)-n-butylester-($C_\alpha$)-benzylester mit
$[\alpha]_D^{20} = + 10,6 \pm 0,1°$ (c = 0,928 ; Methanol),
$R_f = 0,81$ (Methylenchlorid : Methanol : Wasser = 70 : 30 : 5) und
$R_f = 0,57$ (Methylenchlorid : Methanol = 5 : 1).

## Beispiel 16

Zu einer Lösung von 2,85 g (2,09 Mol) gemäss Beispiel 14 erhaltenem N-Propionyl-desmethylmuramyl-L-alanyl-D-glutamyl-($C_\alpha$)-tert.-butylester-($C_\gamma$)-L-alanin-2-(1,2-dipalmitoyl-sn-glycero-3-hydroxyphosphoryloxy)-ethylamid-natriumsalz in 112 ml absolutem Methylenchlorid werden 28 ml Trifluoressigsäure gegeben und das Ganze 3 Stunden bei Raumtemperatur gerührt.

Die farblose, klare Lösung wird dann im Vakuum bei 30° eingedampft und der so erhaltene Rückstand mehrmals in Methylenchlorid aufgenommen und erneut eingedampft.

Man erhält 3,0 g eines farblosen Oels, das in 350 ml Phosphatpuffer/Natriumchlorid (je 0,1 molar, 1 : 1, pH 7) gelöst in einer AMICON-Dialysierzelle (Modell 402, Ultrafilter PM 30/76 mn Ø) bei 3 Atü filtriert wird. Anschliessend wird noch gegen insgesamt 2,1 Liter bidestilliertes Wasser chloridfrei filtriert. Die in der Zelle zurückbleibende Lösung (ca. 50 ml) wird im Hochvakuum lyophilisiert.

Man erhält ein farbloses Lyophilisat, das durch Säulenchromatographie an 260 g Kieselgel (60 reinst, Merck, 0,063-0,200 mm) im System Chloroform : Methanol : Wasser = 70 : 30 : 5 weiter gereinigt wird (10 ml Fraktionen).

Die Fraktionen 90-260 werden vereinigt und im Hochvakuum ohne zu heizen (Abkühlung) zur Trockne eingedampft. Der Rückstand wird in 250 ml bidestilliertem Wasser aufgenommen und in einer AMICON-Dialysierzelle (Modell 402, Ultrafilter PM 30/76 mm Ø) bei 3 Atü zuerst auf ca. 50 ml eingeengt, dann nacheinander gegen 250 ml Phosphatpuffer/Natriumchlorid (je 0,1 molar, 1 : 1, pH 7) und 1 750 ml bidestilliertes Wasser chloridfrei filtriert. Die in der Zelle zurückbleibende Lösung wird anschliessend durch zwei Milliporefilter (NALGENE S, 0,45 μ bzw. 0,2 μ) sterilfiltriert und im Hochvakuum lyophilisiert.

Man erhält N-Propionyl-desmethylmuramyl-L-alanyl-D-glutaminsäure-($C_\gamma$)-L-alanin-2-(1,2-dipalmitoyl-sn-glycero-3-hydroxyphosphoryloxy)-ethyl-amid-dinatriumsalz als farbloses, hygroskopisches Pulver, das 4,80 Mol Wasser enthält, mit
$[\alpha]_D^{20} = - 6,1 \pm 0,1°$ (c = 0,489 ; Methylenchlorid : Ethanol = 1 : 1),
$R_f = 0,28$ (Essigester : n-Butanol : Pyridin : Essigsäure : Wasser = 42 : 21 : 21 : 6 : 10) und
$R_f = 0,62$ (Acetonitril : Wasser = 3 : 1).

## Beispiel 17

4,5 g (34 mMol) gemäss Beispiel 12 erhaltenes N-Acetylmuramyl-L-alanyl-D-glutamyl-($C_\alpha$)-tert.butylester-($C_\gamma$)-L-alanin-2-(1,2-dipalmitoyl-sn-glycero-3-hydroxyphosphoryloxy)-ethylamid-natriumsalz, das im Hochvakuum über Phosphorpentoxid getrocknet worden ist, werden in 75 ml trockenem Dichlormethan gelöst. Man kühlt auf 0° ab und fügt unter Rühren und Ausschluss von Feuchtigkeit 25 ml wasserfreie Trifluoressigsäure zu und lässt auf Raumtemperatur erwärmen. Nach 2 1/2 Stunden wird die klare, farblose Lösung am Rotationsverdampfer bei Raumtemperatur stark eingeengt (~ 10 ml), mehrmals mit je 100 ml Dichlormethan versetzt und letzteres wieder abgedampft. Das verbleibende Oel wird in 100 ml tert.Butanol aufgenommen und lyophilisiert. Das Rohprodukt wird durch zweimalige Chromatographie an je 400 g Kieselgel 60 im System Chloroform : Methanol : Wasser = 70 : 30 : 5 gereinigt. Das in den Fraktionen 92-172 enthaltene Material wird in 100 ml doppelt destilliertem Wasser gelöst und durch

Diafiltration (AMICON-Rührzelle 402, Ultrafilter PM 10/76 mm Ø) analog Beispiel 1 gereinigt. Die in der Zelle verbleibende Lösung wird durch ein Millipore-Filter (0,2 μ) filtriert und lyophilisiert.

Man erhält N-Acetyl-muramyl-L-alanyl-D-glutaminsäure-($C_\gamma$)-L-alanin-2-(1,2-dipalmitoyl-sn-glycero-3-hydroxyphosphoryloxy)-ethylamid-dinatriumsalz, enthaltend 4,89 Mol Wasser, als farbloses, lockeres Pulver mit

$[\alpha]_D^{20} = +10 \pm 1°$ (c = 0,675 ; Methanol),
$R_f$ = 0,08 (Chloroform : Methanol : Wasser = 70 : 30 : 5),
$R_f$ = 0,15 (Essigsäureethylester : n-Butanol : Pyridin : Essigsäure : Wasser = 42 : 21 : 21 : 6 : 10) und
$R_f$ = 0,30 (Chloroform : Methanol : Wasser : Essigsäure = 70 : 40 : 9 : 1).

## Beispiel 18

Alveolar Ratten Makrophagen werden durch Lungenwäsche erhalten, plattiert und *in vitro* 24 Stunden entweder mit Liposomen, die mit dem Wirkstoff beladen sind, oder mit dem Wirkstoff in physiologischer Salzlösung (phosphate buffered saline, PBS) inkubiert. Man fügt $^{125}$I-markierte Tumorzellen hinzu und inkubiert weitere 72 Stunden. Anschliessend wäscht man die getöteten Tumorzellen fort und bestimmt die Zahl der noch lebenden Tumorzellen aufgrund ihrer Radioaktivität. Die Makrophagen-Aktivierung wird aufgrund der Cytotoxizität beurteilt, das heisst nach dem Anteil der bei Versuchsende getöteten Tumorzellen. Die spezifische Cytotoxizität [%] wird folgendermassen berechnet :

$$100 \left[ 1 - \frac{\text{cpm in mit Makrophagen und Wirksubstanz inkubierten Tumorzellen}}{\text{cpm in mit Makrophagen und PBS inkubierten Tumorzellen}} \right]$$

Weitere Einzelheiten der obengenannten Testmethode sind beschrieben in I.J. Fidler et al., J. Biol. Response Modifiers *1*, 43-55 (1982). Einige Versuchsergebnisse betreffend die nachstehend genannten Wirkstoffe sind in der folgenden Tabelle zusammengestellt :

I. N-Acetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz,

II. N-Propionyl-normuramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid-natriumsalz,

III. N-Propionyl-normuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz und

IV. N-Acetyl-muramyl-L-alanyl-D-glutamyl-($C_\alpha$)-tert.butylester-($C_\gamma$)-L-alanin-2-(1,2-dipalmitoyl-sn-glycero-3-hydroxyphosphoryloxy)-ethylamid-natriumsalz

| Wirkstoff | spezifische Cytotoxizität [%] | | | | | | | |
|---|---|---|---|---|---|---|---|---|
| | Wirkstoff in 0,2 ml PBS<br>μg/Kultur | | | | Wirkstoff in 100 μg Liposomen pro 0,2 ml<br>μg/Kultur | | | |
| | 0,02 | 0,2 | 2 | 20 | 0,02 | 0,2 | 2 | 20 |
| I | | 39 | 59 | | | 56 | 51 | 44 |
| II | | 21 | 48 | 74 | | 10 | 23 | 11 |
| III | 3 | 81 | 47 | | 64 | 76 | 79 | |
| IV | 23 | 18 | 20 | | 64 | 68 | 52 | |

## Beispiel 19

Herstellung von 1 000 Tabletten, enthaltend 0,5 % Wirkstoff

Zusammensetzung pro 1 000 Tabletten

| | |
|---|---|
| N-Acetyl-muramyl-L-alanyl-D-glutamyl-($C_\alpha$)-methylester-($C_\gamma$)-L-alanin-2-(1,2-dipalmitoyl-sn-glycero-3-hydroxy-phosphoryloxy)-ethylamid-natriumsalz | 0,5 g |
| Lactose gemahlen | 43,0 g |
| Maisstärke | 52,0 g |
| Pharmacoat 603® (Hydroxypropylmethylcellulose, enthaltend 28-30 % Methoxygruppen, geliefert von Shinetsu Chemical Company, Tokio, Japan) | 3,0 g |
| Aerosil® (Kolloidales Siliziumdioxid, geliefert von Degussa, Frankfurt, | |

| | |
|---|---|
| Bundesrepublik Deutschland) | 1,0 g |
| Magnesiumstearat | 0,5 g |

Herstellung

Der Wirkstoff und 15 g Lactose werden vorgemischt. Die so erhaltene Vormischung wird mit 28 g Lactose und 47 g Maisstärke zusammengemischt. Mit der so erhaltenen Mischung und einer wässerigen Lösung des Pharmacoat wird eine granulierfertige Masse hergestellt, die granuliert, getrocknet und gemahlen wird. Hierzu mischt man 5 g Maisstärke, Aerosil und Magnesiumstearat und komprimiert zu 1 000 Tabletten mit einem Gewicht von je 100 mg.

Die Presslinge können auf an sich bekannte Weise magensaftresistent lackiert werden.

Beispiel 20

Wirkstoff in Form einer lyophilisierten Trockensubstanz

1 mg N-Acetyl-muramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid-natriumsalz und 500 mg Mannit (pyrogenfrei) werden in Wasser für Injektionszwecke gelöst und durch einen Membranfilter sterilfiltriert. Unter aseptischen Bedingungen wird die sterilfiltrierte Lösung in eine sterilisierte Glasampulle oder in ein Glasvial abgefüllt und gefriergetrocknet. Nach der Lyophilisation wird die Ampulle verschlossen bzw. das Vial wird mit einem gummielastomeren Verschluss und Alukappe verschlossen.

**Patentansprüche** (für die Vertragsstaaten : BE, CH, DE, FR, GB, IT, LI, LU, NL, SE)

1. Verbindungen der Formel I,

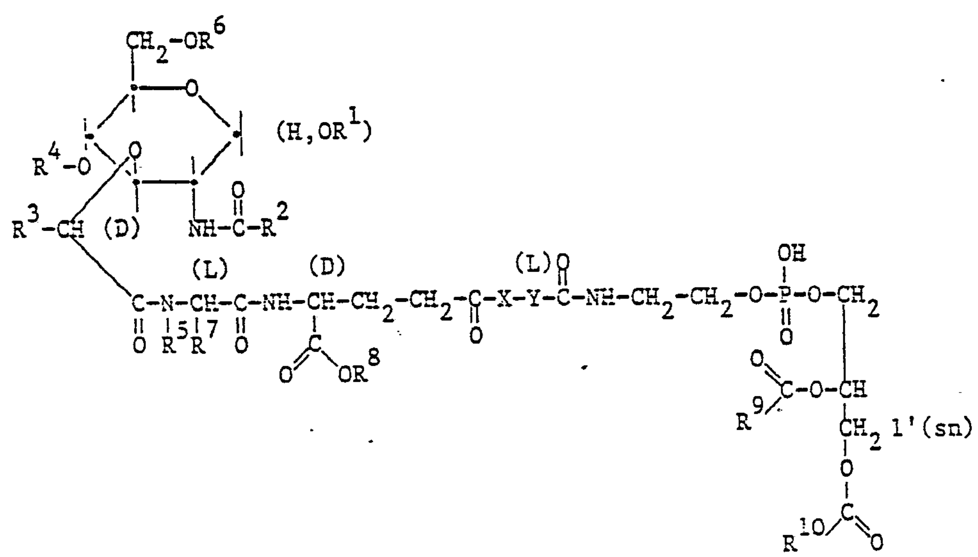

(I)

worin $R^1$, $R^4$ und $R^6$ unabhängig voneinander Wasserstoff oder Niederalkanoyl, $R^2$ $C_{1-4}$-Alkyl, Hydroxymethyl oder Phenyl, $R^3$ Wasserstoff oder Methyl, $R^5$ Wasserstoff oder $C_{1-3}$-Alkyl, $R^7$ unsubstituiertes oder durch Hydroxy, Mercapto oder Methylthio substituiertes $C_{1-3}$-Alkyl, $R^8$ Wasserstoff oder Niederalkyl, X Sauerstoff oder die Gruppe NH, Y $C_{1-4}$-Alkyliden und $R^9$ und $R^{10}$ unabhängig voneinander $C_{11-17}$-Alkyl oder $C_{11-17}$-Alkenyl bedeuten, wobei das Präfix « Nieder » Reste von und mit 1 bis und mit 7 Kohlenstoffatomen bezeichnet, und ihre Salze.

2. Verbindungen der Formel I nach Anspruch 1, worin $R^1$, $R^4$ und $R^6$ unabhängig voneinander Wasserstoff oder $C_{1-3}$-Alkanoyl, $R^2$ $C_{1-2}$-Alkyl oder Hydroxymethyl, $R^3$ Wasserstoff oder Methyl, $R^5$ Wasserstoff oder Methyl, $R^7$ $C_{1-3}$-Alkyl oder Hydroxymethyl, $R^8$ Wasserstoff oder $C_{1-4}$-Alkyl, X Sauerstoff oder die Gruppe NH, Y $C_{1-3}$-Alkyliden und $R^9$ und $R^{10}$ unabhängig voneinander geradkettiges $C_{11-17}$-Alkyl oder Descarbonyl-oleoyl bedeuten, und ihre Salze.

3. Verbindungen der Formel I nach Anspruch 2, worin $R^1$, $R^4$ und $R^6$ unabhängig voneinander Wasserstoff oder Acetyl, $R^2$ Methyl oder Ethyl, $R^3$ Wasserstoff oder Methyl, $R^5$ Wasserstoff oder Methyl, $R^7$ $C_{1-3}$-Alkyl, $R^8$ Wasserstoff oder $C_{1-4}$-Alkyl, X Sauerstoff oder die Gruppe NH, Y $C_{1-2}$-Alkyliden und $R^9$ und $R^{10}$ unabhängig voneinander geradkettiges $C_{11-17}$-Alkyl mit ungerader Anzahl C-Atome oder Descarbonyl-oleoyl bedeuten, und ihre Salze.

4. Verbindungen der Formel I nach einem der Ansprüche 1-3, worin $R^8$ für den in diesen Ansprüchen genannten Alkylrest steht, und ihre Salze.

5. Verbindungen der Formel I nach einem der Ansprüche 1-4 worin mindestens einer der Reste $R^1$, $R^4$ und $R^6$ für Niederalkanoyl steht, und ihre Salze.

6. Verbindungen der Formel I nach einem der Ansprüche 1-4, worin $R^1$, $R^4$ und $R^6$ für Wasserstoff stehen, und ihre Salze.

7. Verbindungen der Formel I nach Anspruch 3, worin $R^1$, $R^4$ und $R^6$ Wasserstoff, $R^2$ Methyl oder Ethyl, $R^3$ Wasserstoff, wenn $R^2$ für Ethyl steht, oder Methyl, wenn $R^2$ für Methyl steht, $R^5$ Wasserstoff oder Methyl, $R^7$ $C_{1-2}$-Alkyl, $R^8$ geradkettiges $C_{1-4}$-Alkyl, X Sauerstoff oder die Gruppe NH, Y Ethyliden und $R^9$ und $R^{10}$ unabhängig voneinander geradkettiges $C_{15-17}$-Alkyl mit ungerader Anzahl C-Atome bedeuten, und ihre Salze.

8. Verbindungen der Formel I nach einem der Ansprüche 1-7, worin $R^3$ Methyl bedeutet, und ihre Salze.

9. Verbindungen der Formel I nach einem der Ansprüche 1-8 worin $R^5$ für Wasserstoff steht, und ihre Salze.

10. Verbindungen der Formel I nach einem der Ansprüche 1-9 worin $R^8$ n-Butyl bedeutet, und ihre Salze.

11. Verbindungen der Formel I nach einem der Ansprüche 1-10, worin X für die Gruppe NH steht, und ihre Salze.

12. Verbindungen der Formel I nach einem der Ansprüche 1-11, worin $R^9$ genau wie $R^{10}$ für n-Pentadecyl steht, und ihre Salze.

13. N-Acetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid und eine Salze nach Anspruch 1.

14. N-Propionyl-desmethylmuramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid und seine Salze nach Anspruch 1.

15. N-Acetyl-muramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid und seine Salze nach Anspruch 1.

16. N-Propionyl-desmethylmuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid und seine Salze nach Anspruch 1.

17. N-Acetyl-muramyl-L-alanyl-D-glutamyl-($C_\alpha$)-tert.-butylester-($C_\gamma$)-L-alanin-2-(1,2-dipalmitoyl-sn-glycero-3-hydroxyphosphoryloxy)-ethylamid und seine Salze nach Anspruch 1.

18. N-Propionyl-desmethylmuramyl-L-alanyl-D-glutamyl-($C_\alpha$)-tert.-butylester-($C_\gamma$)-L-alanin-2-(1,2-dipalmitoyl-sn-glycero-3-hydroxyphosphoryloxy)-ethylamid und seine Salze nach Anspruch 1.

19. N-Acetyl-muramyl-L-alanyl-D-glutaminsäure-($C_\gamma$)-L-alanin-2-(1,2-dipalmitoyl-sn-glycero-3-hydroxyphosphoryloxy)-ethylamid und seine Salze nach Anspruch 1.

20. N-Propionyl-desmethylmuramyl-L-alanyl-D-glutaminsäure-($C_\gamma$)-L-alanin-2-(1,2-dipalmitoyl-sn-glycero-3-hydroxyphosphoryloxy)-ethylamid und seine Salze nach Anspruch 1.

21. Pharmazeutisch verwendbare Salze nach einem der Ansprüche 1-20.

22. Natriumsalze nach Anspruch 21.

23. Pharmazeutische Präparate, die eine Verbindung nach einem der Ansprüche 1-22 zusammen mit einem pharmazeutischen Trägermaterial enthalten.

24. Eine Verbindung nach einem der Ansprüche 1-22 zur Verwendung in einem Verfahren zur Behandlung des menschlichen oder tierischen Körpers.

25. Verwendung einer Verbindung nach einem der Ansprüche 1-22 zur Herstellung von pharmazeutischen Präparaten für die Anwendung zur Prophylaxe und Therapie von Virusinfektionen.

26. Verfahren zur Herstellung von Verbindungen der Formel I

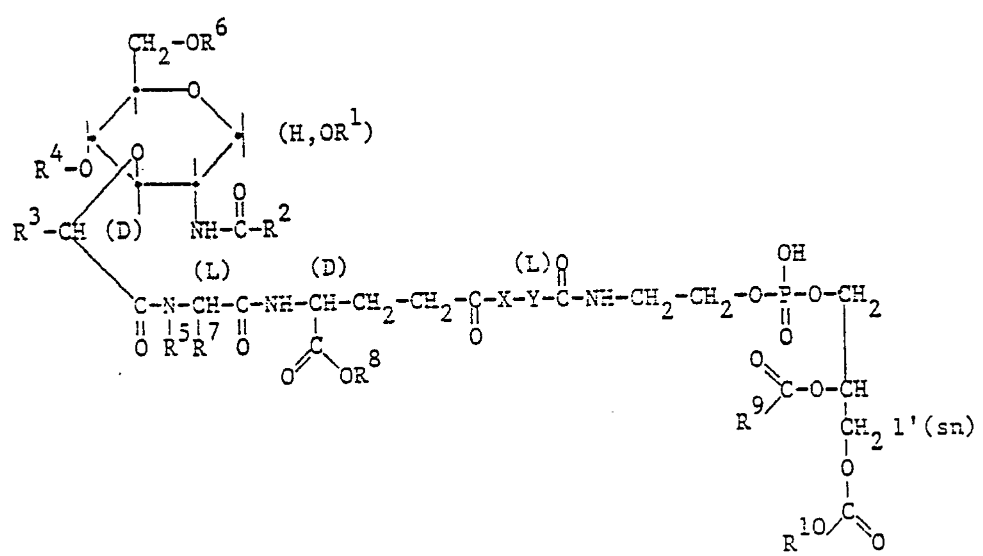

(I)

worin $R^1$, $R^4$ und $R^6$ unabhängig voneinander Wasserstoff oder Niederalkanoyl, $R^2$ $C_{1-4}$-Alkyl, Hydroxymethyl oder Phenyl, $R^3$ Wasserstoff oder Methyl, $R^5$ Wasserstoff oder $C_{1-3}$-Alkyl, $R^7$ unsubstituiertes oder durch Hydroxy, Mercapto oder Methylthio substituiertes $C_{1-3}$-Alkyl, $R^8$ Wasserstoff oder Niederalkyl, X Sauerstoff oder die Gruppe NH, Y $C_{1-4}$-Alkyliden und $R^9$ und $R^{10}$ unabhängig voneinander $C_{11-17}$-Alkyl oder $C_{11-17}$-Alkenyl bedeuten, wobei das Präfix « Nieder » Reste von und mit 1 bis und mit 7 Kohlenstoffatomen bezeichnet, und von ihren Salzen, dadurch gekennzeichnet, dass man

a) eine Verbindung der Formel II,

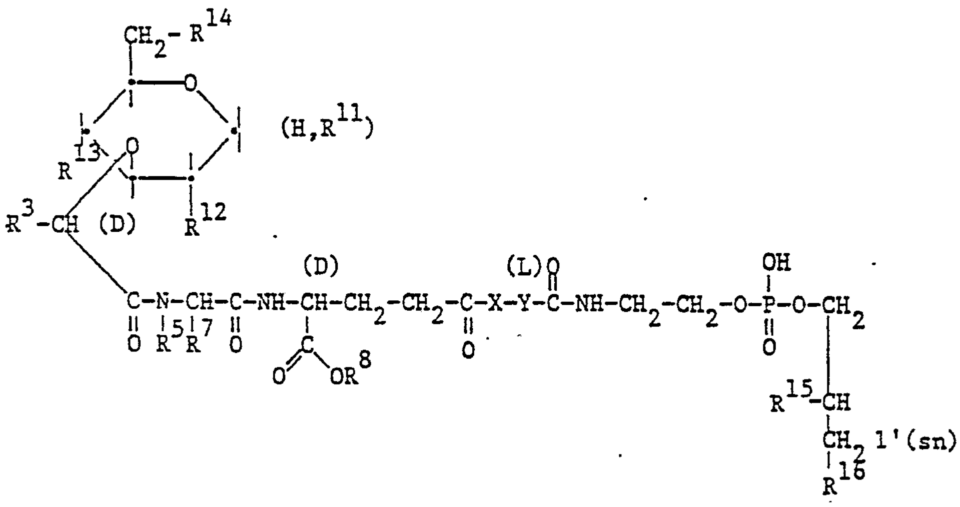

(II)

worin mindestens einer der Reste $R^{11}$, $R^{13}$, $R^{14}$, $R^{15}$ und $R^{16}$ für freies oder in reaktionsfähiger Form vorliegendes Hydroxy steht und/oder worin $R^{12}$ für freies oder in reaktionsfähiger Form vorliegendes Amino steht, und die übrigen Substituenten die obengenannten Bedeutungen haben, mit der Massgabe, dass in einer Verbindung der Formel II vorhandene funktionelle Gruppen mit Ausnahme der an der Reaktion teilnehmenden Gruppe(n) gegebenenfalls in geschützter Form vorliegen, mit einer Carbonsäure oder einem reaktionsfähigen Derivat davon unter Einführung von mindestens einem Acylrest $R^1$,

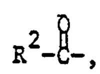,

$R^4$, $R^6$,

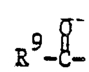

oder

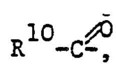,

worin die Substituenten die obengenannten Bedeutungen haben, acyliert und gegebenenfalls vorhandene Schutzgruppen abspaltet, oder

b) eine Verbindung der Formel III,

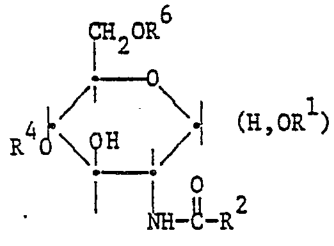

(III)

worin die Substituenten die obengenannten Bedeutungen haben und vorhandene funktionelle Gruppen mit Ausnahme der freien Hydroxygruppe in 3-Stellung gegebenenfalls in geschützter Form vorliegen, wobei die 3-Hydroxygruppe gegebenenfalls in reaktionsfähiger Form vorliegt, mit einer Verbindung der

Formel IV,

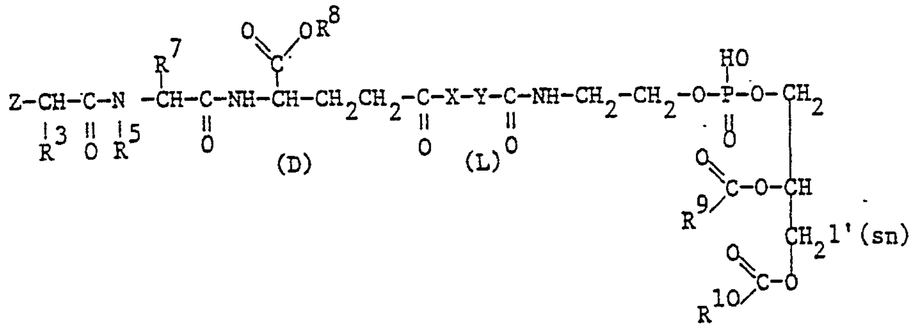

(IV)

worin Z eine gegebenenfalls in reaktionsfähiger Form vorliegende Hydroxygruppe bedeutet und die übrigen Substituenten die obengenannten Bedeutungen haben, mit der Massgabe, dass in einer Verbindung der Formel IV vorhandene funktionelle Gruppen mit Ausnahme von Z gegebenenfalls in geschützter Form vorliegen, umsetzt und vorhandene Schutzgruppen abspaltet, oder

c) eine Verbindung der Formel V,

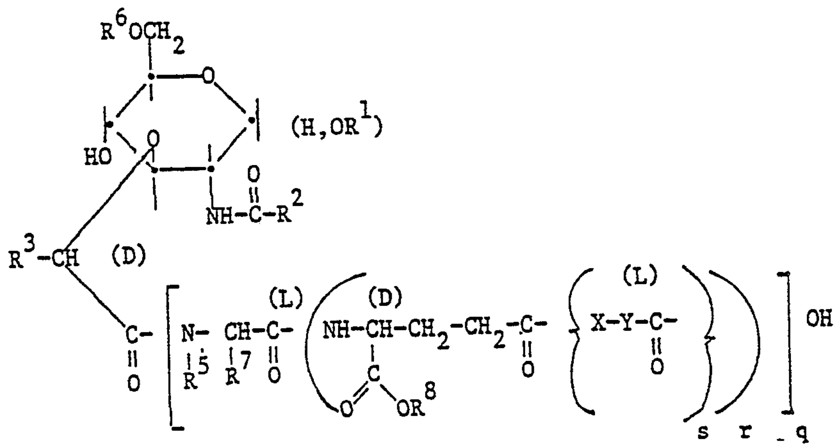

(V)

worin s, r und q unabhängig voneinander für 0 oder 1 und $R^8$ für Niederalkyl oder eine Carboxylschutzgruppe stehen und die übrigen Substituenten die obengenannten Bedeutungen haben, mit der Massgabe, dass in einer Verbindung der Formel V vorhandene freie funktionelle Gruppen mit Ausnahme der an der Reaktion teilnehmenden Gruppe gegebenenfalls in geschützter Form vorliegen, oder ein reaktionsfähiges Carbonsäurederivat davon, mit einer Verbindung der Formel VI,

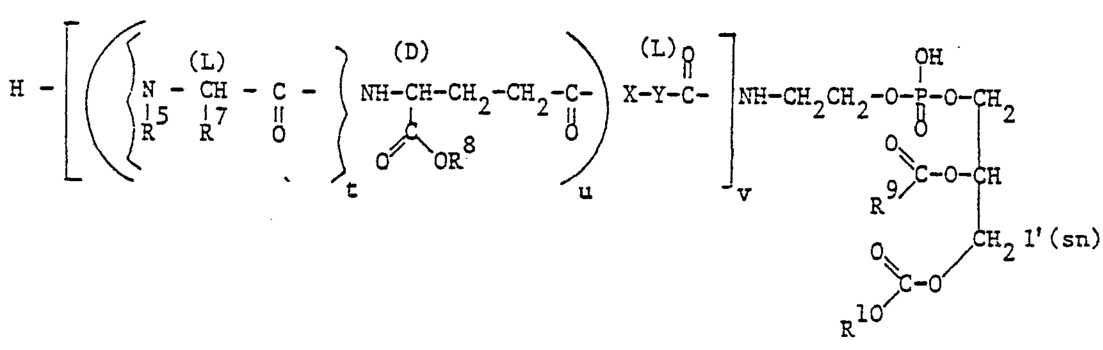

(VI)

worin t, u und v unabhängig voneinander für 0 oder 1 stehen und die Substituenten die obengenannten Bedeutungen haben, mit der Massgabe, dass t, u und v für 1 stehen, wenn im Reaktionspartner der Formel V q für 0 steht, oder dass t für 0, u für 1 und v für 1 stehen, wenn q für 1 und r für 0 stehen, oder dass u für 0 und v für 1 stehen, wenn q für 1, r für 1 und s für 0 stehen, oder dass v für 0 steht, wenn q, r und s für 1 stehen, oder einem reaktionsfähigen Derivat davon umsetzt, und gegebenenfalls vorhandene Schutzgruppen abspaltet, oder

d) eine Verbindung der Formel VII,

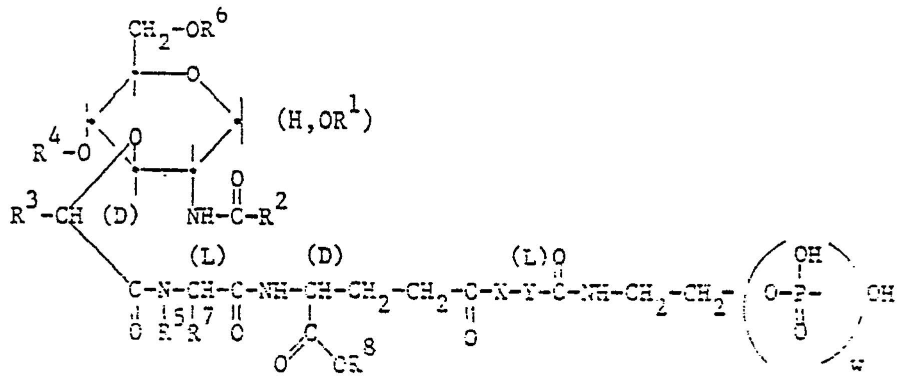

(VII)

worin w für 0 oder 1 steht und die Substituenten die obengenannten Bedeutungen haben, mit der Massgabe, dass freie funktionelle Gruppen mit Ausnahme der an der Reaktion teilnehmenden Gruppe gegebenenfalls in geschützter Form vorliegen, oder ein reaktionsfähiges Phosphorsäurederivat einer solchen Säure der Formel VII, worin w für 1 steht, mit einer Verbindung der Formel VIII,

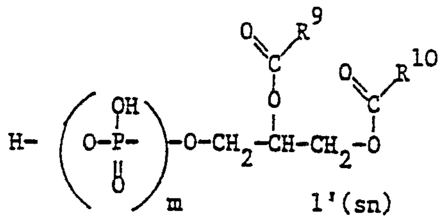

(VIII)

worin die Substituenten die obengenannten Bedeutungen haben und m für 0 oder 1 steht, mit der Massgabe, dass m für 1 steht, wenn im Reaktionspartner der Formel VII w für 0 steht, oder dass m für 0 steht, wenn w für 1 steht, oder mit einem reaktionsfähigen Phosphorsäurederivat einer Säure der Formel VIII, worin m für 1 steht, umsetzt, und vorhandene Schutzgruppen abspaltet, oder

e) das reaktionsfähige Derivat einer Verbindung der Formel VII,

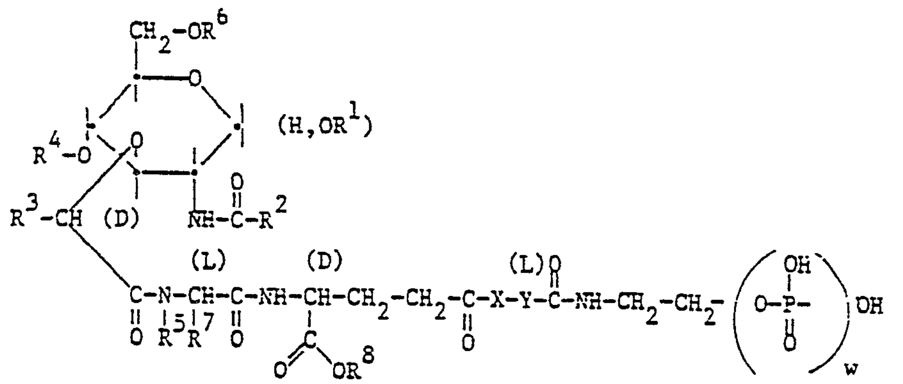

(VII)

worin w für 0 oder 1 steht und die Substituenten die obigen Bedeutungen haben, mit der Massgabe, dass in einer Verbindung der Formel VII vorhandene freie funktionelle Gruppen mit Ausnahme der an der Reaktion teilnehmenden Gruppe, gegebenenfalls durch leicht abspaltbare Schutzgruppen geschützt sind, sowie mit der Massgabe, dass das reaktionsfähige Derivat einer Verbindung der Formel VII, worin w für 0 steht, ein solches mit reaktionsfähiger, veresterter, endständiger Hydroxygruppe ist, und mit der Massgabe, dass das reaktionsfähige Derivat einer Verbindung der Formel VII, worin w für 1 steht, ein Salz ist, mit dem reaktionsfähigen Derivat einer Verbindung der Formel VIII,

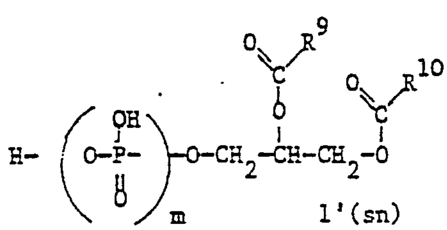

(VIII)

worin die Substituenten die obengenannten Bedeutungen haben und m für 0 oder 1 steht, mit der Massgabe, dass m für 1 steht, wenn im Reaktionspartner der Formel VII w für 0 steht, wobei das reaktionsfähige Derivat einer Verbindung der Formel VIII ein Salz ist, oder dass m für 0 steht, wenn w für 1 steht, wobei das reaktionsfähige Derivat einer Verbindung der Formel VIII ein solches mit reaktionsfähiger, veresterter, endständiger Hydroxygruppe ist, umsetzt und vorhandene Schutzgruppen abspaltet, oder

f) eine Verbindung der Formel IX,

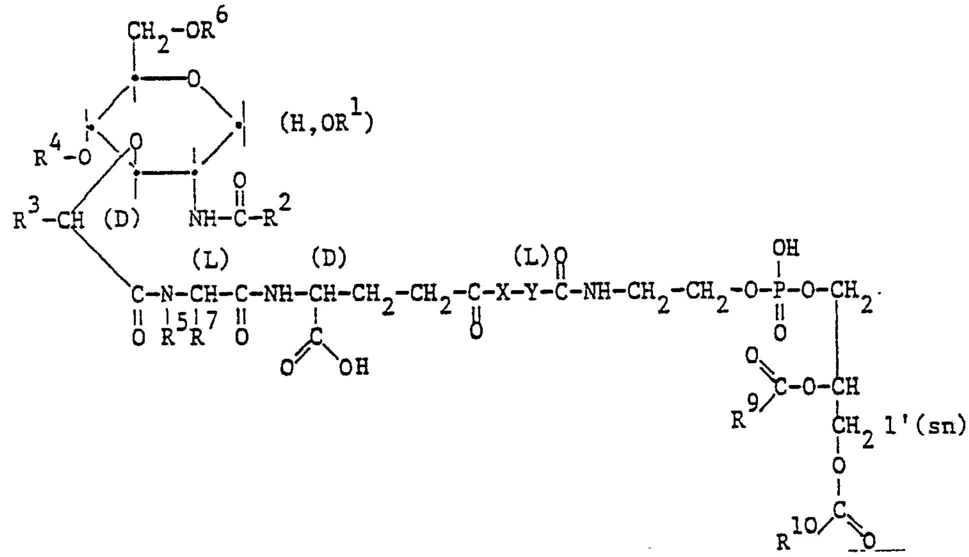

(IX)

worin die Substituenten die obigen Bedeutungen haben, wobei in einer Verbindung der Formel IX vorhandene freie funktionelle Gruppen mit Ausnahme der an der Reaktion teilnehmenden Gruppe gegebenenfalls in geschützter Form vorliegen, oder ein reaktionsfähiges Carbonsäurederivat einer Verbindung der Formel IX, mit einem Niederalkanol $R^8$—OH, worin $R^8$ $C_1$-$C_7$-Alkyl bedeutet, oder einem reaktionsfähigen Derivat davon umsetzt und vorhandene Schutzgruppen abspaltet, oder

g) eine Verbindung der Formel X,

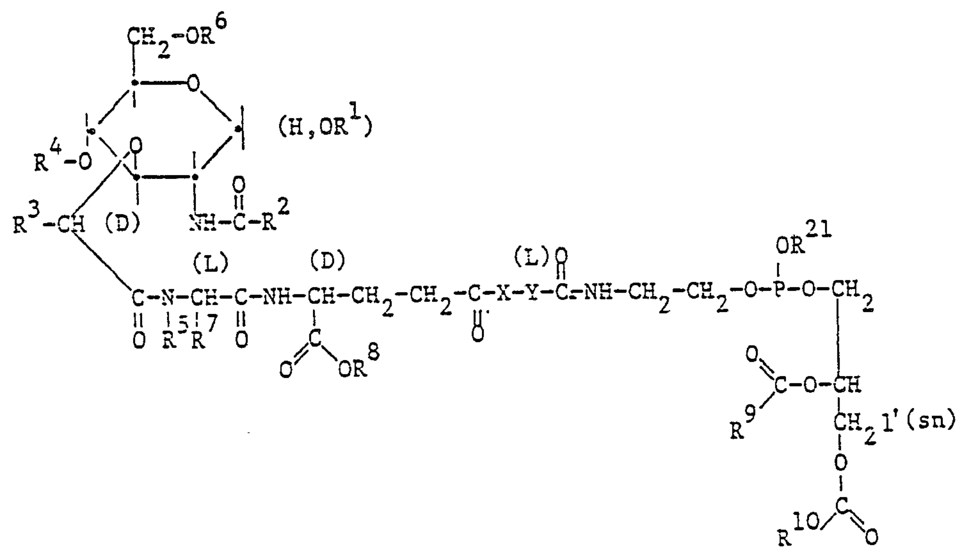

(X)

worin $R^{21}$ für Wasserstoff oder eine Schutzgruppe steht und die übrigen Substituenten die obengenannten Bedeutungen haben, wobei in einer Verbindung der Formel X vorhandene freie funktionelle Gruppen mit Ausnahme der an der Reaktion teilnehmenden Gruppe gegebenenfalls in geschützter Form vorliegen, oder ein Tautomeres dieser Verbindung mit einem Oxidationsmittel oxidiert und vorhandene Schutzgruppen abspaltet, oder

h) in einer Furanoseverbindung der Formel XI,

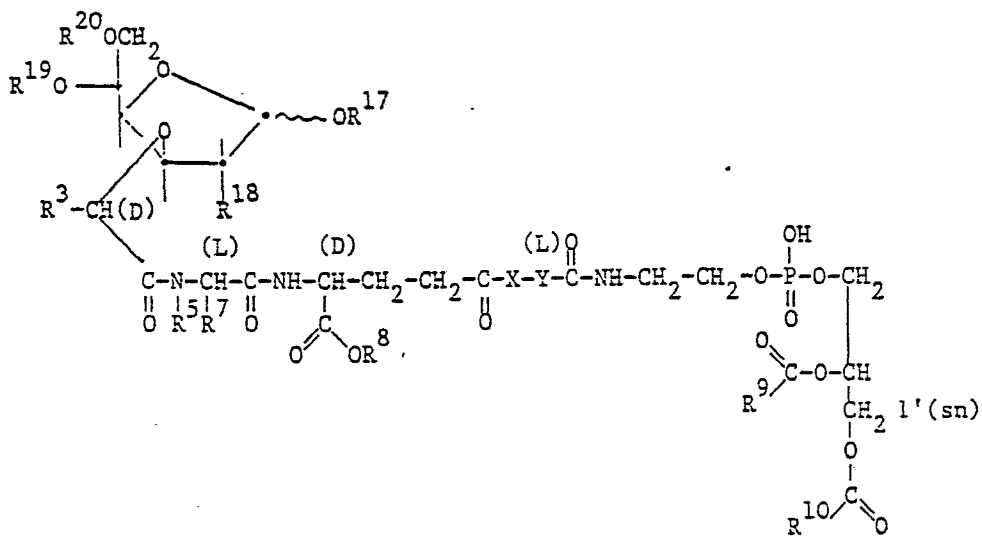

(XI)

worin $R^{17}$ Wasserstoff oder eine leicht abspaltbare Hydroxyschutzgruppe bedeutet und $R^{18}$ die oben für den Rest

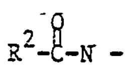

genannte Bedeutung hat, oder $R^{17}$ und $R^{18}$ zusammen eine mit der freien Valenz des C-Atoms an Sauerstoff gebundene, bivalente Schutzgruppe der Formel

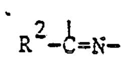

bedeuten, worin $R^2$ die obengenannte Bedeutung hat, und $R^{19}$ und $R^{20}$ Wasserstoff oder eine leicht abspaltbare Hydroxyschutzgruppe bedeuten, oder $R^{19}$ und $R^{20}$ zusammen für eine bivalente Hydroxyschutzgruppe stehen, und die übrigen Substituenten die obengenannten Bedeutungen haben, die Schutzgruppen abspaltet, oder

i) in einer Verbindung der Formel I, worin mindestens einer der Reste $R^1$, $R^2$, $R^4$, $R^6$, $R^7$ oder $R^8$ in geschützter Form vorliegt, die Schutzgruppe(n) abspaltet oder

j) eine Verbindung der Formel XII,

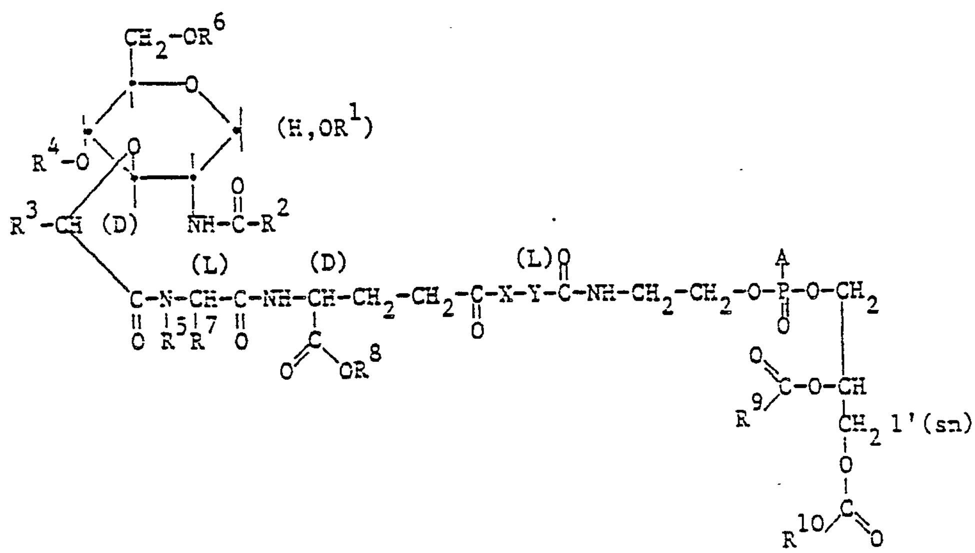

(XII)

worin A ein Halogenatom bedeutet und die übrigen Substituenten die obengenannten Bedeutungen haben, hydrolysiert, und, wenn erwünscht, nach Durchfürhrung eines der Verfahren a-j) eine erhaltene

Verbindung der Formel I in ihr Salz oder ein erhaltenes Salz in ein anderes Salz überführt.

27. Die nach den Verfahren des Anspruchs 26 erhältlichen Verbindungen.

**Patentansprüche** (für den Vertragsstaat AT)

1. Verfahren zur Herstellung von Verbindungen der Formel I

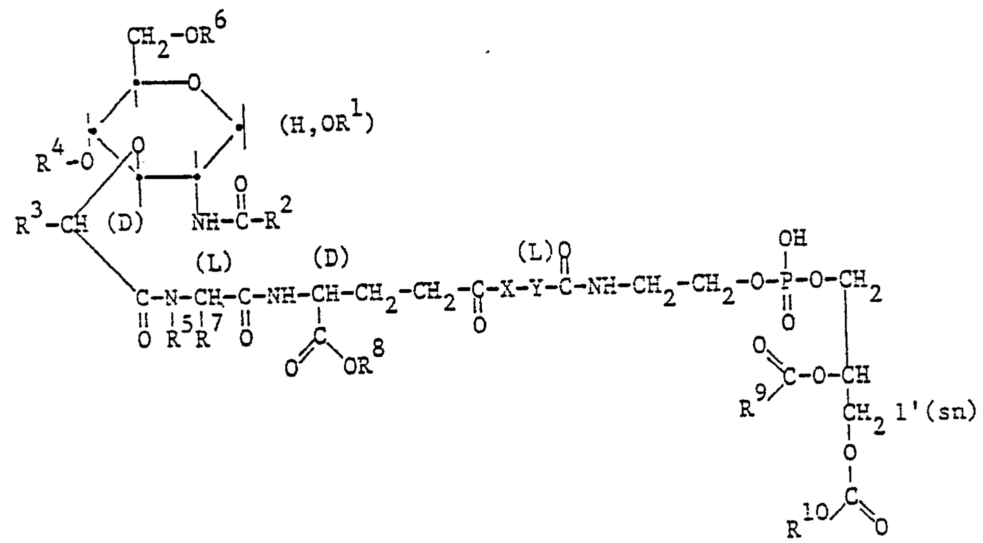

(I)

worin $R^1$, $R^4$ und $R^6$ unabhängig voneinander Wasserstoff oder Niederalkanoyl, $R^2$ $C_{1-4}$-Alkyl, Hydroxymethyl oder Phenyl, $R^3$ Wasserstoff oder Methyl, $R^5$ Wasserstoff oder $C_{1-3}$-Alkyl, $R^7$ unsubstituiertes oder durch Hydroxy, Mercapto oder Methylthio substituiertes $C_{1-3}$-Alkyl, $R^8$ Wasserstoff oder Niederalkyl, X Sauerstoff oder die Gruppe NH, Y $C_{1-4}$-Alkyliden und $R^9$ und $R^{10}$ unabhängig voneinander $C_{11-17}$-Alkyl oder $C_{11-17}$-Alkenyl bedeuten, wobei das Präfix « Nieder » Reste von und mit 1 bis und mit 7 Kohlenstoffatomen bezeichnet, und/oder von ihren Salzen, dadurch gekennzeichnet, dass man

a) eine Verbindung der Formel II,

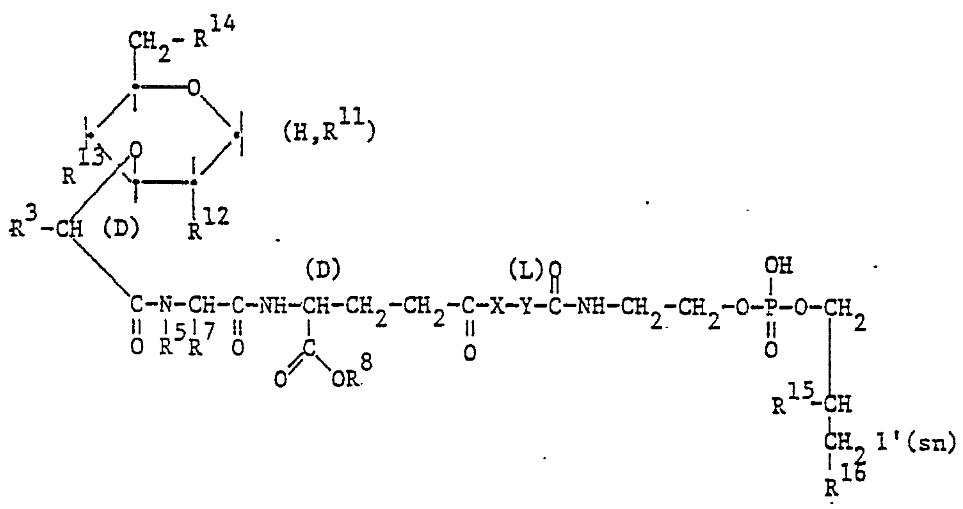

(II)

worin mindestens einer der Reste $R^{11}$, $R^{13}$, $R^{14}$, $R^{15}$ und $R^{16}$ für freies oder in reaktionsfähiger Form vorliegendes Hydroxy steht und/oder worin $R^{12}$ für freies oder in reaktionsfähiger Form vorliegendes Amino steht, und die übrigen Substituenten die obengenannten Bedeutungen haben, mit der Massgabe, dass in einer Verbindung der Formel II vorhandene funktionelle Gruppen mit Ausnahme der an der Reaktion teilnehmenden Gruppe(n) gegebenenfalls in geschützter Form vorliegen, mit einer Carbonsäure oder einem reaktionsfähigen Derivat davon unter Einführung von mindestens einem Acylrest $R^1$,

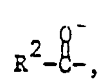

$R^4$, $R^6$,

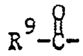

oder

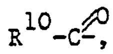

worin die Substituenten die obengenannten Bedeutungen haben, acyliert und gegebenenfalls vorhandene Schutzgruppen abspaltet, oder

b) eine Verbindung der Formel III,

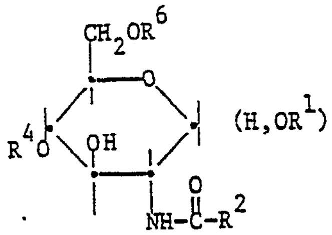

(III)

worin die Substituenten die obengenannten Bedeutungen haben und vorhandene funktionelle Gruppen mit Ausnahme der freien Hydroxygruppe in 3-Stellung gegebenenfalls in geschützter Form vorliegen, wobei die 3-Hydroxygruppe gegebenenfalls in reaktionsfähiger Form vorliegt, mit einer Verbindung der Formel IV,

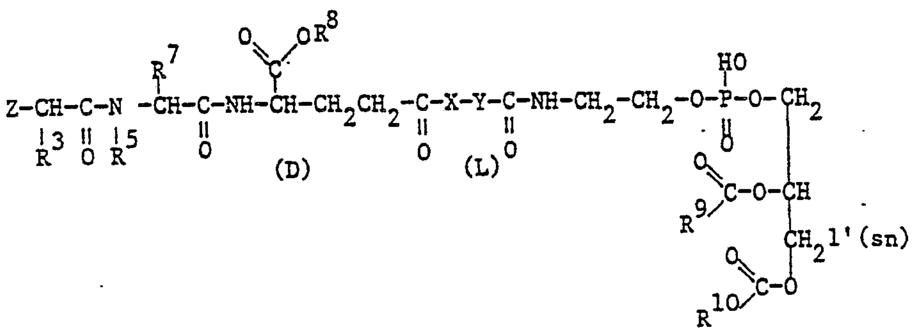

(IV)

worin Z eine gegebenenfalls in reaktionsfähiger Form vorliegende Hydroxygruppe bedeutet und die übrigen Substituenten die obengenannten Bedeutungen haben, mit der Massgabe, dass in einer Verbindung der Formel IV vorhandene funktionelle Gruppen mit Ausnahme von Z gegebenenfalls in geschützter Form vorliegen, umsetzt und vorhandene Schutzgruppen abspaltet, oder

c) eine Verbindung der Formel V,

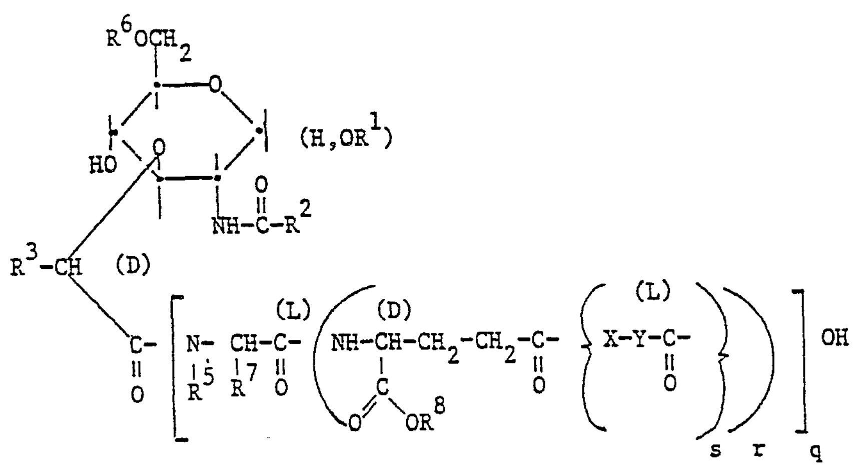

(V)

worin s, r und q unabhängig voneinander für 0 oder 1 und $R^8$ für Niederalkyl oder eine Carboxylschutzgruppe stehen und die übrigen Substituenten die obengenannten Bedeutungen haben, mit der Massgabe, dass in einer Verbindung der Formel V vorhandene freie funktionelle Gruppen mit Ausnahme der an der Reaktion teilnehmenden Gruppe gegebenenfalls in geschützter Form vorliegen, oder ein reaktionsfähiges Carbonsäurederivat davon, mit einer Verbindung der Formel VI,

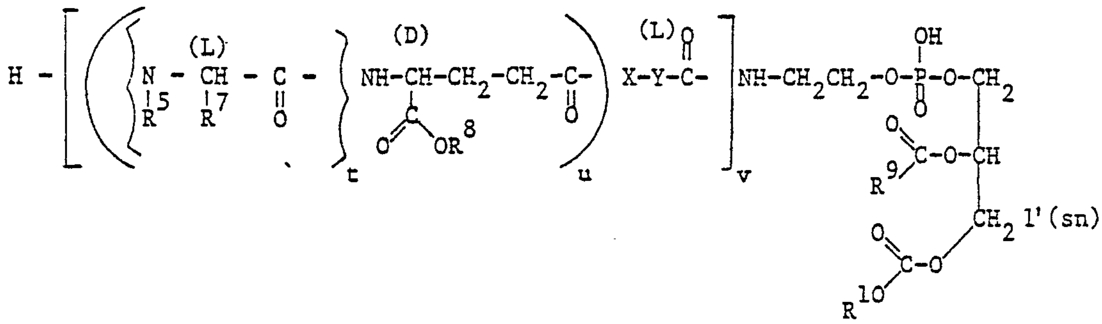

(VI)

worin t, u und v unabhängig voneinander für 0 oder 1 stehen und die Substituenten die obengenannten Bedeutungen haben, mit der Massgabe, dass t, u und v für 1 stehen, wenn im Reaktionspartner der Formel V q für 0 steht, oder dass t für 0, u für 0, u für 1 und v für 1 stehen, wenn q für 1 und r für 0 stehen, oder dass u für 0 und v für 1 stehen, wenn q für 1, r für 1 und s für 0 stehen, oder dass v für 0 steht, wenn q, r und s für 1 stehen, oder einem reaktionsfähigen Derivat davon umsetzt, und gegebenenfalls vorhandene Schutzgruppen abspaltet, oder

d) eine Verbindung der Formel VII,

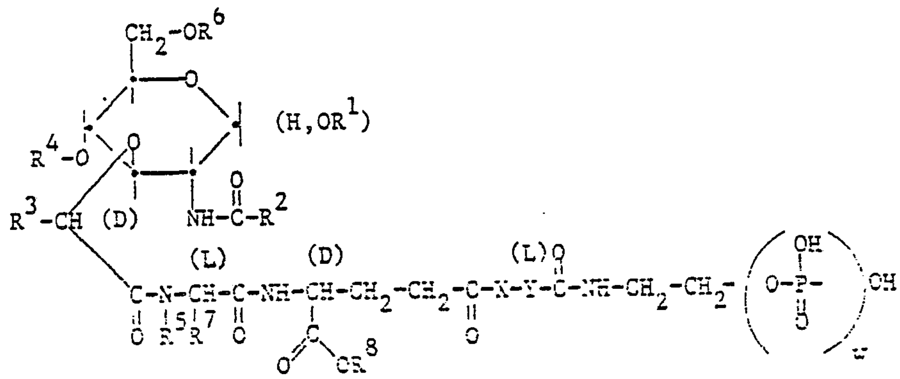

(VII)

worin w für 0 oder 1 steht und die Substituenten die obengenannten Bedeutungen haben, mit der Massgabe, dass freie funktionelle Gruppen mit Ausnahme der an der Reaktion teilnehmenden Gruppe gegebenenfalls in geschützter Form vorliegen, oder ein reaktionsfähiges Phosphorsäurederivat einer solchen Säure der Formel VII, worin w für 1 steht, mit einer Verbindung der Formel VIII,

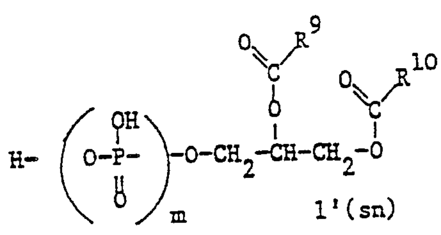

(VIII)

worin die Substituenten die obengenannten Bedeutungen haben und m für 0 oder 1 steht, mit der Massgabe, dass m für 1 steht, wenn im Reaktionspartner der Formel VII w für 0 steht, oder dass m für 0 steht, wenn w für 1 steht, oder mit einem reaktionsfähigen Phosphorsäurederivat einer Säure der Formel VIII, worin m für 1 steht, umsetzt, und vorhandene Schutzgruppen abspaltet, oder

e) das reaktionsfähige Derivat einer Verbindung der Formel VII,

(Siehe Formel VII Seite 40 f.)

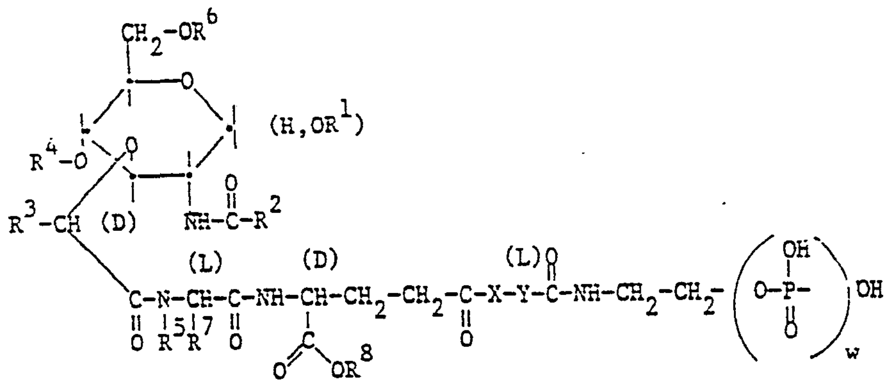

(VII)

worin w für 0 oder 1 steht und die Substituenten die obigen Bedeutungen haben, mit der Massgabe, dass in einer Verbindung der Formel VII vorhandene freie funktionelle Gruppen mit Ausnahme der an der Reaktion teilnehmenden Gruppe, gegebenenfalls durch leicht abspaltbare Schutzgruppen geschützt sind, sowie mit der Massgabe, dass das reaktionsfähige Derivat einer Verbindung der Formel VII, worin w für 0 steht, ein solches mit reaktionsfähiger, veresterter, endständiger Hydroxygruppe ist, und mit der Massgabe, dass das reaktionsfähige Derivat einer Verbindung der Formel VII, worin w für 1 steht, ein Salz ist, mit dem reaktionsfähigen Derivat einer Verbindung der Formel VIII,

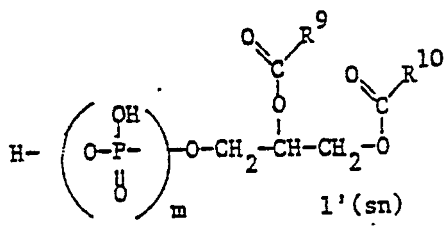

(VIII)

worin die Substituenten die obengenannten Bedeutungen haben und m für 0 oder 1 steht, mit der Massgabe, dass m für 1 steht, wenn im Reaktionspartner der Formel VII w für 0 steht, wobei das reaktionsfähige Derivat einer Verbindung der Formel VIII ein Salz ist, oder dass m für 0 steht, wenn w für 1 steht, wobei das reaktionsfähige Derivat einer Verbindung der Formel VIII ein solches mit reaktionsfähiger, veresterter, endständiger Hydroxygruppe ist, umsetzt und vorhandene Schutzgruppen abspaltet, oder

f) eine Verbindung der Formel IX,

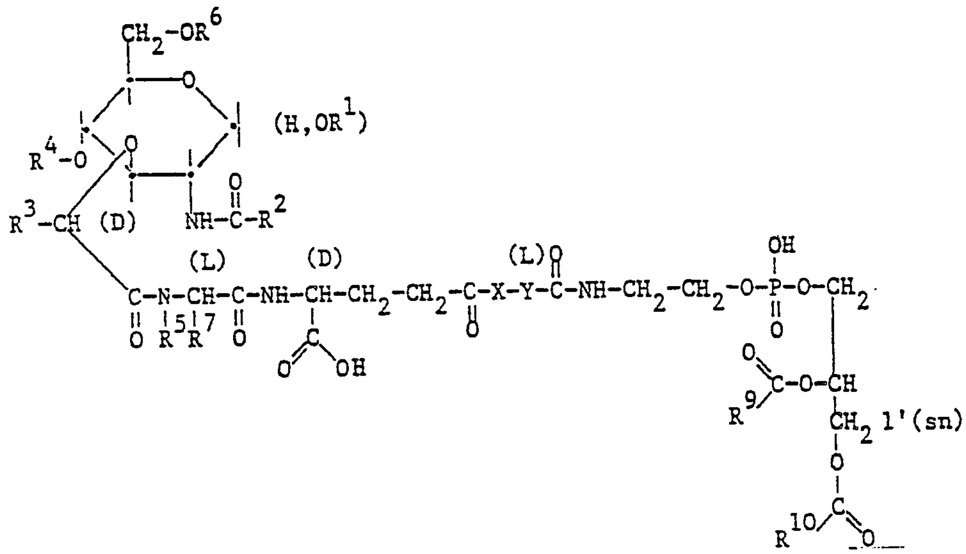

(IX)

worin die Substituenten die obigen Bedeutungen haben, wobei in einer Verbindung der Formel IX vorhandene freie funktionelle Gruppen mit Ausnahme der an der Reaktion teilnehmenden Gruppe gegebenenfalls in geschützter Form vorliegen, oder ein reaktionsfähiges Carbonsäurederivat einer Verbindung der Formel IX, mit einem Niederalkanol $R^8$—OH, worin $R^8$ Niederalkyl bedeutet, oder einem reaktionsfähigen Derivat davon umsetzt und vorhandene Schutzgruppen abspaltet, oder

g) eine Verbindung der Formel X,

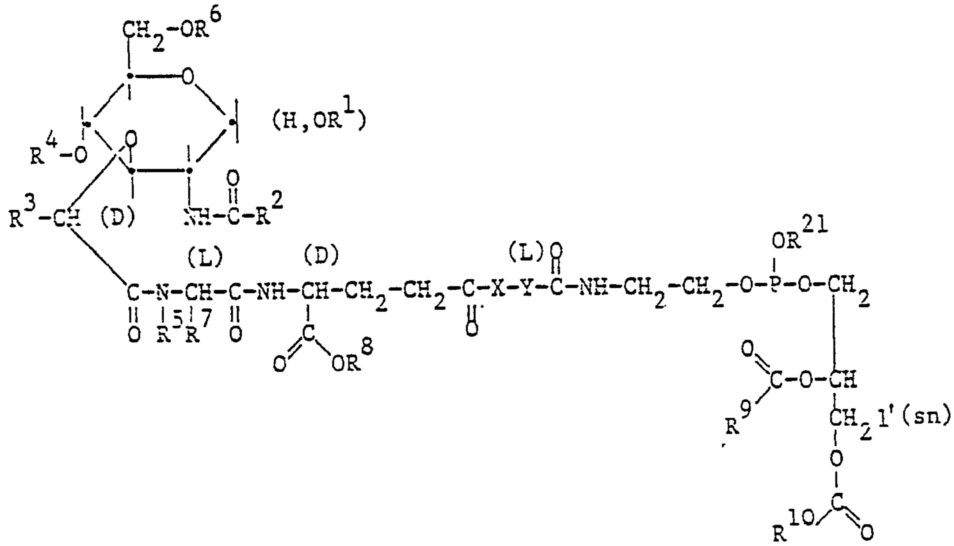

(X)

worin $R^{21}$ für Wasserstoff oder eine Schutzgruppe steht und die übrigen Substituenten die obengenannten Bedeutungen haben, wobei in einer Verbindung der Formel X vorhandene freie funktionelle Gruppen mit Ausnahme der an der Reaktion teilnehmenden Gruppe gegebenenfalls in geschützter Form vorliegen, oder ein Tautomeres dieser Verbindung mit einem Oxidationsmittel oxidiert und vorhandene Schutzgruppen abspaltet, oder

h) in einer Furanoseverbindung der Formel XI,

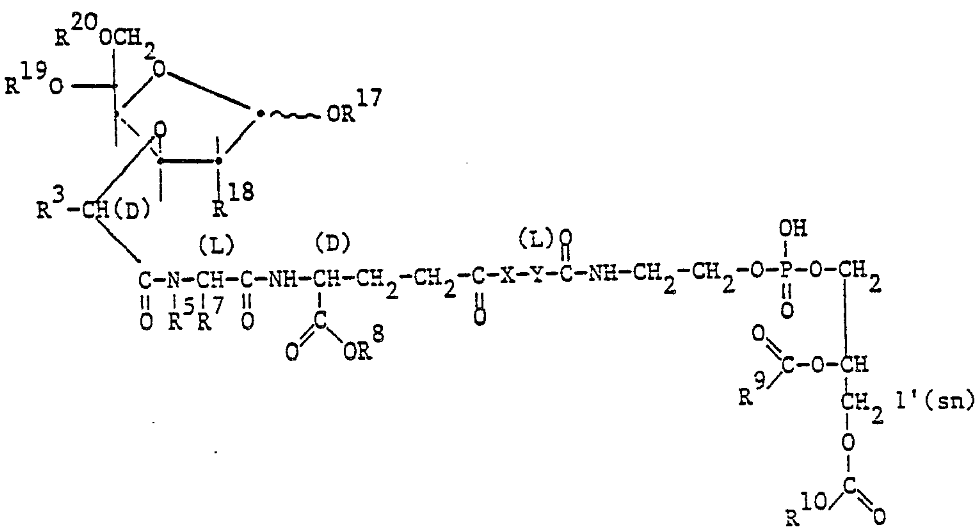

(XI)

worin $R^{17}$ Wasserstoff oder eine leicht abspaltbare Hydroxyschutzgruppe bedeutet und $R^{18}$ die oben für den Rest

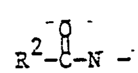

genannte Bedeutung hat, oder $R^{17}$ und $R^{18}$ zusammen eine mit der freien Valenz des C-Atoms an Sauerstoff gebundene, bivalente Schutzgruppe der Formel

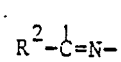

bedeuten, worin $R^2$ die obengenannte Bedeutung hat, und $R^{19}$ und $R^{20}$ Wasserstoff oder eine leicht abspaltbare Hydroxyschutzgruppe bedeuten, oder $R^{19}$ und $R^{20}$ zusammen für eine bivalente Hydroxyschutzgruppe stehen, und die übrigen Substituenten die obengenannten Bedeutungen haben, die Schutzgruppen abspaltet, oder

i) in einer Verbindung der Formel I, worin mindestens einer der Reste $R^1$, $R^2$, $R^4$, $R^6$, $R^7$ oder $R^8$ in

geschützter Form vorliegt, die Schutzgruppe(n) abspaltet oder

j) eine Verbindung der Formel XII,

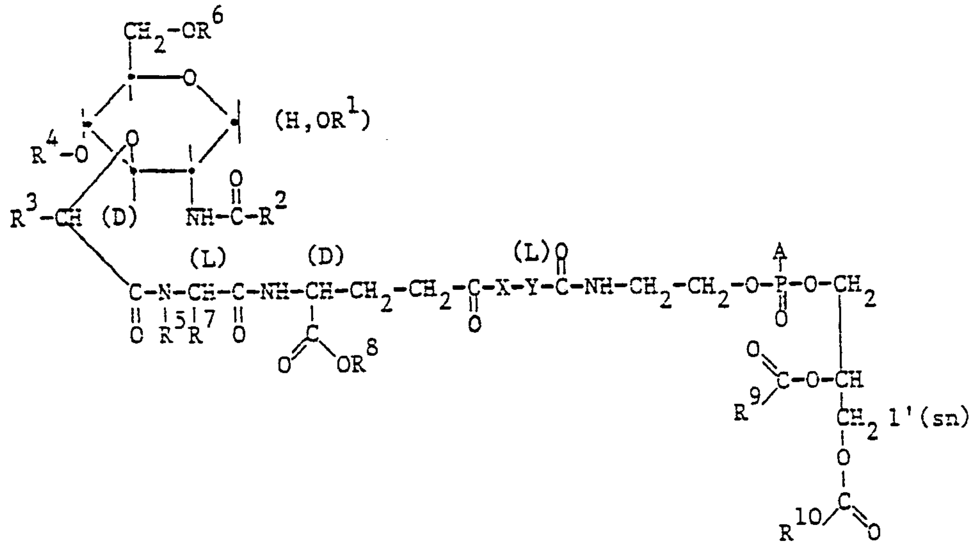

(XII)

worin A ein Halogenatom bedeutet und die übrigen Substituenten die obengenannten Bedeutungen haben, hydrolysiert, und, wenn erwünscht, nach Durchführung eines der Verfahren a-j) eine erhaltene Verbindung der Formel I in ihr Salz oder ein erhaltenes Salz in ein anderes Salz überführt.

2. Verfahren nach Anspruch 1, dadurch gekennzeichnet, dass man die Ausgangsstoffe so wählt, dass man Verbindungen der Formel I, worin $R^1$, $R^4$ und $R^6$ unabhängig voneinander Wasserstoff oder $C_{1-3}$-Alkanoyl, $R^2$, $C_{1-2}$-Alkyl oder Hydroxymethyl, $R^3$ Wasserstoff oder Methyl, $R^5$ Wasserstoff oder Methyl, $R^7$ $C_{1-3}$-Alkyl oder Hydroxymethyl, $R^8$ Wasserstoff oder $C_{1-4}$-Alkyl, X Sauerstoff oder die Gruppe NH Y $C_{1-3}$-Alkyliden und $R^9$ und $R^{10}$ unabhängig voneinander geradkettiges $C_{11-17}$-Alkyl oder Descarbonyl-oleoyl bedeuten, und/oder ihre Salze erhält.

3. Verfahren nach Anspruch 2, dadurch gekennzeichnet, dass man die Ausgangsstoffe so wählt, dass man Verbindungen der Formel I, worin $R^1$, $R^4$ und $R^6$ unabhängig voneinander Wasserstoff oder Acetyl, $R^2$ Methyl oder Ethyl, $R^3$ Wasserstoff oder Methyl, $R^5$ Wasserstoff oder Methyl, $R^7$ $C_{1-3}$-Alkyl, $R^8$ Wasserstoff oder $C_{1-4}$-Alkyl, X Sauerstoff oder die Gruppe NH, Y $C_{1-2}$-Alkyliden und $R^9$ und $R^{10}$ unabhängig voneinander geradkettiges $C_{11-17}$-Alkyl mit ungerader Anzahl C-Atome oder Descarbonyl-oleoyl bedeuten, und/oder ihre Salze erhält.

4. Verfahren nach einem der Ansprüche 1-3, dadurch gekennzeichnet, dass man die Ausgangsstoffe so wählt, dass man Verbindungen der Formel I, worin $R^8$ für den in diesen Ansprüchen genannten Alkylrest steht, und/oder ihre Salze erhält.

5. Verfahren nach einem der Ansprüche 1-4, dadurch gekennzeichnet, dass man die Ausgangsstoffe so wählt, dass man Verbindungen der Formel I, worin mindestens einer der Reste $R^1$, $R^4$ und $R^6$ für Niederalkanoyl steht, und/oder ihre Salze erhält.

6. Verfahren nach einem der Ansprüche 1-4, dadurch gekennzeichnet, dass man die Ausgangsstoffe so wählt, dass man Verbindungen der Formel I, worin $R^1$, $R^4$ und $R^6$ für Wasserstoff stehen, und/oder ihre Salze erhält.

7. Verfahren nach Anspruch 3, dadurch gekennzeichnet, dass man die Ausgangsstoffe so wählt, dass man Verbindungen der Formel I, worin $R^1$, $R^4$ und $R^6$ Wasserstoff, $R^2$ Methyl oder Ethyl, $R^3$ Wasserstoff, wenn $R^2$ für Ethyl steht, oder Methyl, wenn $R^2$ für Methyl steht, $R^5$ Wasserstoff oder Methyl, $R^7$ $C_{1-2}$-Alkyl, $R^8$ geradkettiges $C_{1-4}$-Alkyl, X Sauerstoff oder die Gruppe NH, Y Ethyliden und $R^9$ und $R^{10}$ unabhängig voneinander geradkettiges $C_{15-17}$-Alkyl mit ungerader Anzahl C-Atome bedeuten, und/oder ihre Salze erhält.

8. Verfahren nach einem der Ansprüche 1-7, dadurch gekennzeichnet, dass man die Ausgangsstoffe so wählt, dass man Verbindungen der Formel I, worin $R^3$ Methyl bedeutet, und/oder ihre Salze erhält.

9. Verfahren nach einem der Ansprüche 1-8, dadurch gekennzeichnet, dass man die Ausgangsstoffe so wählt, dass man Verbindungen der Formel I, worin $R^5$ für Wasserstoff steht, und/oder ihre Salze erhält.

10. Verfahren nach einem der Ansprüche 1-9, dadurch gekennzeichnet, dass man die Ausgangsstoffe so wählt, dass man Verbindungen der Formel I, worin $R^8$ n-Butyl bedeutet, und/oder ihre Salze erhält.

11. Verfahren nach einem der Ansprüche 1-10, dadurch gekennzeichnet, dass man die Ausgangsstoffe so wählt, dass man Verbindungen der Formel I, worin X für die Gruppe NH steht, und/oder ihre Salze erhält.

12. Verfahren nach einem der Ansprüche 1-11, dadurch gekennzeichnet, dass man die Ausgangsstoffe so wählt, dass man Verbindungen der Formel I, worin $R^9$ genau wie $R^{10}$ für n-Pentadecyl steht,

und/oder ihre Salze erhält.

13. Verfahren nach Anspruch 1, dadurch gekennzeichnet, dass man die Ausgangsstoffe so wählt, dass man N-Acetyl-muramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid und/oder ein Salz davon erhält.

14. Verfahren nach Anspruch 1, dadurch gekennzeichnet, dass man die Ausgangsstoffe so wählt, dass man N-Propionyl-desmethylmuramyl-L-alanyl-D-glutamyl-($C_\alpha$)-n-butylester-($C_\gamma$)-L-alanin-2-(1,2-dipalmitoyl-sn-glycero-3-hydroxyphosphoryloxy)-ethylamid und/oder ein Salz davon erhält.

15. Verfahren nach Anspruch 1, dadurch gekennzeichnet, dass man die Ausgangsstoffe so wählt, dass man N-Acetyl-muramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid und/oder ein Salz davon erhält.

16. Verfahren nach Anspruch 1, dadurch gekennzeichnet, dass man die Ausgangsstoffe so wählt, dass man N-Propionyl-desmethylmuramyl-L-alanyl-D-glutamyl-($C_\alpha$)-methylester-($C_\gamma$)-L-alanin-2-(1,2-dipalmitoyl-sn-glycero-3-hydroxyphosphoryloxy)-ethylamid und/oder ein Salz davon erhält.

17. Verfahren nach Anspruch 1, dadurch gekennzeichnet, dass man die Ausgangsstoffe so wählt, dass man N-Acetyl-muramyl-L-alanyl-D-glutaminsäure-($C_\gamma$)-L-alanin-2-(1,2-dipalmitoyl-sn-glycero-3-hydroxyphosphoryloxy)-ethylamid und/oder ein Salz davon erhält.

18. Verfahren nach einem der Ansprüche 1-17, dadurch gekennzeichnet, dass man die Ausgangsstoffe so wählt, dass man pharmazeutisch verwendbare Salze erhält.

19. Verfahren nach Anspruch 18, dadurch gekennzeichnet, dass man die Ausgangsstoffe so wählt, dass man Natriumsalze erhält.

**Claims** (for the Contracting States : BE, CH, DE, FR, GB, IT, LI, LU, NL, SE)

1. A compound of the formula I

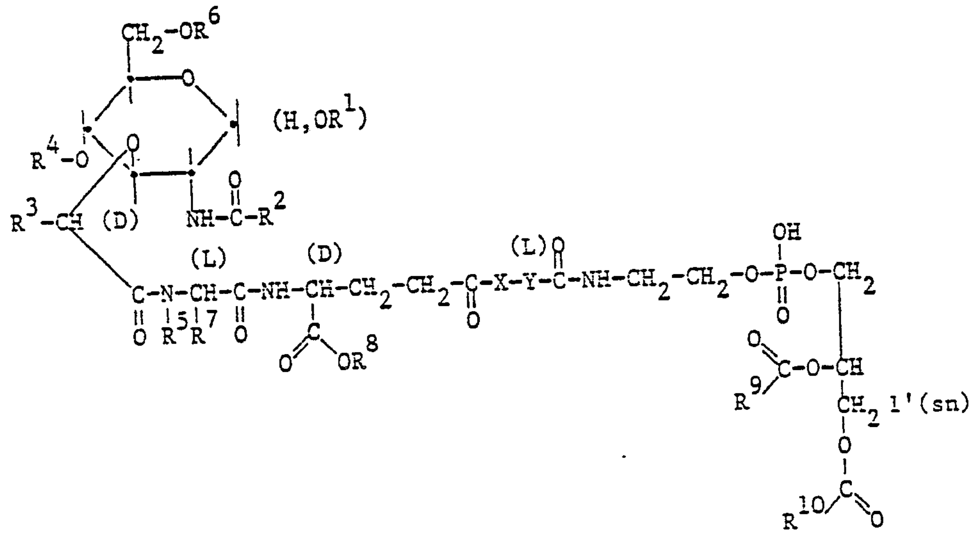

(I)

in which each of $R^1$, $R^4$ and $R^6$, independently of the others, represents hydrogen or lower alkanoyl, $R^2$ represents $C_{1-4}$-alkyl, hydroxymethyl or phenyl, $R^3$ represents hydrogen or methyl, $R^5$ represents hydrogen or $C_{1-3}$-alkyl, $R^7$ represents $C_{1-3}$-alkyl that is unsubstituted or substituted by hydroxy, mercapto or methylthio, $R^8$ represents hydrogen or lower alkyl, X represents oxygen or the group NH, Y represents $C_{1-4}$-alkylidene, and each of $R^9$ and $R^{10}$, independently of the other, represents $C_{11-17}$-alkyl or $C_{11-17}$-alkenyl, with the term « lower » signifying radicals containing from 1 up to and including 7 carbon atoms, or a salt thereof.

2. A compound of the formula I according to claim 1, in which each of $R^1$, $R^4$ and $R^6$, independently of the others, represents hydrogen or $C_{1-3}$-alkanoyl, $R^2$ represents $C_{1-2}$-alkyl or hydroxymethyl, $R^3$ represents hydrogen or methyl, $R^5$ represents hydrogen or methyl, $R^7$ represents $C_{1-3}$-alkyl or hydroxymethyl, $R^8$ represents hydrogen or $C_{1-4}$-alkyl, X represents oxygen or the group NH, Y represents $C_{1-3}$-alkylidene and each of $R^9$ and $R^{10}$, independently of the other, represents straight chain $C_{11-17}$-alkyl or decarbonyloleoyl, or a salt thereof.

3. A compound of the formula I according to claim 2, in which each of $R^1$, $R^4$ and $R^6$, independently of the others, represents hydrogen or acetyl, $R^2$ represents methyl or ethyl, $R^3$ represents hydrogen or methyl, $R^5$ represents hydrogen or methyl, $R^7$ represents $C_{1-3}$-alkyl, $R^8$ represents hydrogen or $C_{1-4}$-alkyl, X represents oxygen or the group NH, Y represents $C_{1-2}$-alkylidene and each of $R^9$ and $R^{10}$, independently of the other, represents straight chain $C_{11-17}$-alkyl having an uneven number of C-atoms or decarbonyloleoyl, or a salt thereof.

4. A compound of the formula I according to any one of claims 1 to 3, in which $R^8$ represents the alkyl

radical mentioned in these claims, or a salt thereof.

5. A compound of the formula I according to any one of claims 1 to 4, in which at least one of the radicals $R^1$, $R^4$ and $R^6$ represents lower alkanoyl, or a salt thereof.

6. A compound of the formula I according to any one of claims 1 to 4, in which each of $R^1$, $R^4$ and $R^6$ represents hydrogen, or a salt thereof.

7. A compound of the formula I according to claim 3, in which each of $R^1$, $R^4$ and $R^6$ represents hydrogen, $R^2$ represents methyl or ethyl, $R^3$ represents hydrogen if $R^2$ represents ethyl, or methyl if $R^2$ represents methyl, $R^5$ represents hydrogen or methyl, $R^7$ represents $C_{1-2}$-alkyl, $R^8$ represents straight chain $C_{1-4}$-alkyl, X represents oxygen or the group NH, Y represents ethylidene and each of $R^9$ and $R^{10}$, independently of the other, represents straight chain $C_{15-17}$-alkyl having an uneven number of C-atoms, or a salt thereof.

8. A compound of the formula I according to any one of claims 1 to 7, in which $R^3$ represents methyl, or a salt thereof.

9. A compound of the formula I according to any one of claims 1 to 8, in which $R^5$ represents hydrogen, or a salt thereof.

10. A compound of the formula I according to any one of claims 1 to 9, in which $R^8$ represents n-butyl, or a salt thereof.

11. A compound of the formula I according to any one of claims 1 to 10, in which X represents the group NH, or a salt thereof.

12. A compound of the formula I according to any one of claims 1 to 11, in which $R^9$ and $R^{10}$ represent n-pentadecyl, or a salt thereof.

13. N-Acetylmuramyl-L-alanyl-D-glutamyl-($C_\alpha$)-n-butyl ester-($C_\gamma$)-L-alanine-2-(1,2-dipalmitoyl-sn-glycero-3-hydroxyphosphoryloxy)-ethylamide, or a salt thereof, according to claim 1.

14. N-Propionyldesmethylmuramyl-L-alanyl-D-glutamyl-($C_\alpha$)-n-butyl ester-($C_\gamma$)-L-alanine-2-(1,2-dipalmitoyl-sn-glycero-3-hydroxyphosphoryloxy)-ethylamide, or a salt thereof, according to claim 1.

15. N-Acetylmuramyl-L-alanyl-D-glutamyl-($C_\alpha$)-methyl ester-($C_\gamma$)-L-alanine-2-(1,2-dipalmitoyl-sn-glycero-3-hydroxyphosphoryloxy)-ethylamide, or a salt thereof, according to claim 1.

16. N-Propionyldesmethylmuramyl-L-alanyl-D-glutamyl-($C_\alpha$)-methyl ester-($C_\gamma$)-L-alanine-2-(1,2-dipalmitoyl-sn-glycero-3-hydroxyphosphoryloxy)-ethylamide, or a salt thereof, according to claim 1.

17. N-Acetylmuramyl-L-alanyl-D-glutamyl-($C_\alpha$)-tert-butyl ester-($C_\gamma$)-L-alanine-2-(1,2-dipalmitoyl-sn-glycero-3-hydroxyphosphoryloxy)-ethylamide, or a salt thereof, according to claim 1.

18. N-Propionyldesmethylmuramyl-L-alanyl-D-glutamyl-($C_\alpha$)-tert-butyl ester-($C_\gamma$)-L-alanine-2-(1,2-dipalmitoyl-sn-glycero-3-hydroxyphosphoryloxy)-ethylamide, or a salt thereof, according to claim 1.

19. N-Acetylmuramyl-L-alanyl-D-glutamic acid-($C_\gamma$)-L-alanine-2-(1,2-dipalmitoyl-sn-glycerol-3-hydroxyphosphoryloxy)-ethylamide, or a salt thereof, according to claim 1.

20. N-Propionyldesmethylmuramyl-L-alanyl-D-glutamic acid-($C_\gamma$)-L-alanine-2-(1,2-dipalmitoyl-sn-glycero-3-hydroxyphosphoryloxy)-ethylamide, or a salt thereof, according to claim 1.

21. A pharmaceutically acceptable salt according to any one of claims 1 to 20.

22. A sodium salt according to claim 21.

23. A pharmaceutical composition containing a compound according to any one of claims 1 to 22, together with a pharmaceutical carrier.

24. A compound according to any one of claims 1 to 22 for use in a method of treating the human or animal body.

25. Use of a compound according to any one of claims 1 to 22 for the preparation of a pharmaceutical composition for the prophylaxis and treatment of virus infections.

26. A process for the preparation of a compound of the formula I

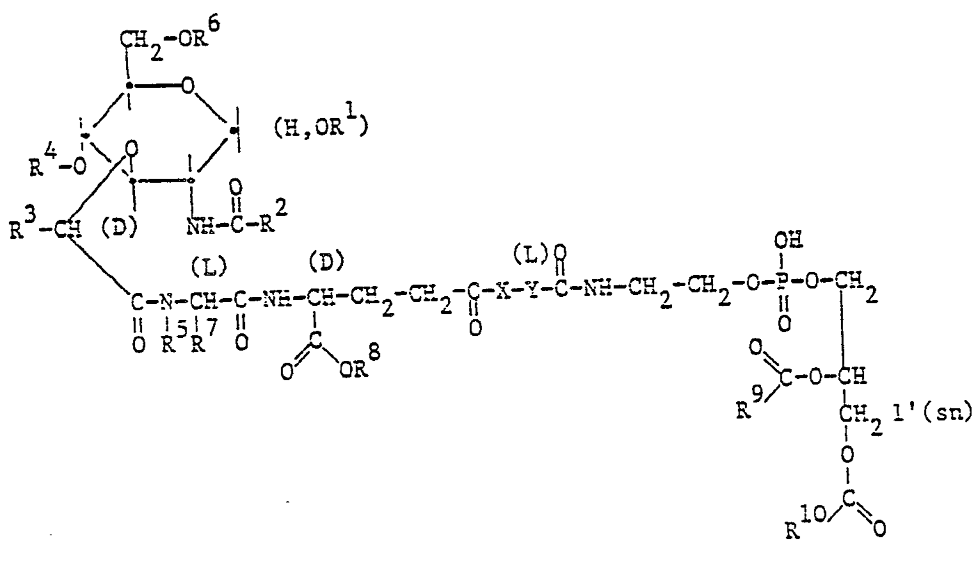

(I)

in which each of $R^1$, $R^4$ and $R^6$, $R^4$ and $R^6$, independently of the others, represents hydrogen or lower alkanoyl, $R^2$ represents $C_{1-4}$-alkyl, hydroxymethyl or phenyl, $R^3$ represents hydrogen or methyl, $R^5$ represents hydrogen or $C_{1-3}$-alkyl, $R^7$ represents $C_{1-3}$-alkyl that is unsubstituted or substituted by hydroxy, mercapto or methylthio, $R^8$ represents hydrogen or lower alkyl, X represents oxygen or the group NH, Y represents $C_{1-4}$-alkylidene, and each of $R^9$ and $R^{10}$, independently of the other, represents $C_{11-17}$-alkyl or $C_{11-17}$-alkenyl, with the terme « lower » signifying radicals containing from 1 up to and including 7 carbon atoms, or of a salt thereof, which process comprises

a) acylating a compound of the formula II,

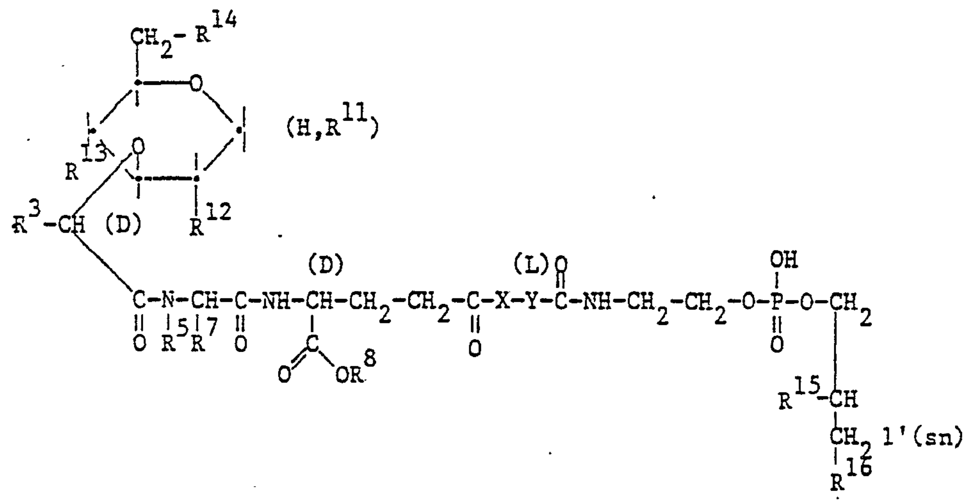

(II)

in which at least one of the radicals $R^{11}$, $R^{13}$, $R^{14}$, $R^{15}$ and $R^{16}$ represents hydroxy that is free or in reactive form, and/or in which $R^{12}$ represents amino that is free or in reactive form, and the remaining substituents have the meanings given above, with the proviso that functional groups present in a compound of the formula II, with the exception of the group(s) participating in the reaction, are in unprotected or protected form, with a carboxylic acid or a reactive derivative thereof to introduce at least one acyl radical $R^1$,

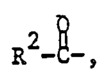

$R^4$, $R^6$

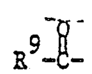

or

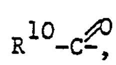

in which the substituents have the meanings given above, and removing any protecting groups present, or

b) reacting a compound of the formula III

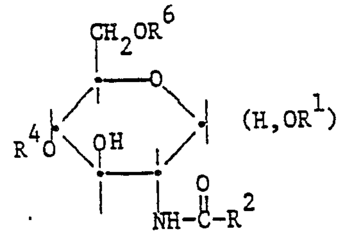

(III)

in which the substituents have the meanings given above and any functional groups present, with the exception of the free hydroxy group in the 3-position, are in unprotected or protected from, which 3-hydroxy group may be present in reactive form, with a compound of the formula IV,

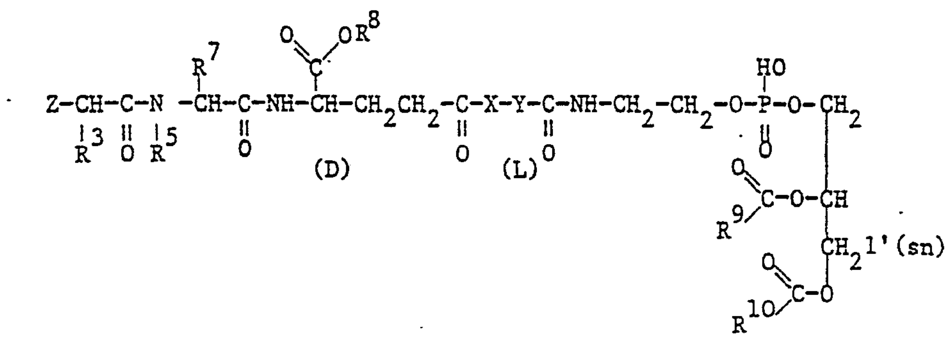

(IV)

in which Z represents a hydroxy group which may be present in reactive form and the ramaining substituents have the meanings given above, with the proviso that functional groups present in a compound of the formula IV, with the exception of Z, are in unprotected or protected form, and removing any protecting groups present, or

c) reacting a compound of the formula V,

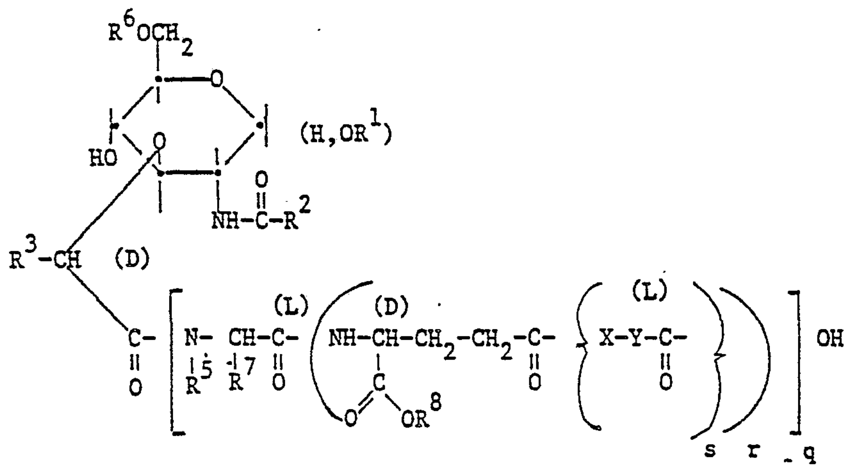

(V)

in which each of s, r and q, independently of the others, represents 0 or 1 and $R^8$ represents lower alkyl or a carboxy-protecting group, and the ramaining substituents have the meanings given above, with the proviso that free functional groups present in a compound of the formula V, with the exception of the group participating in the reaction, are in unprotected or protected form, or a reactive carboxylic acid derivative thereof, with a compound of the formula VI,

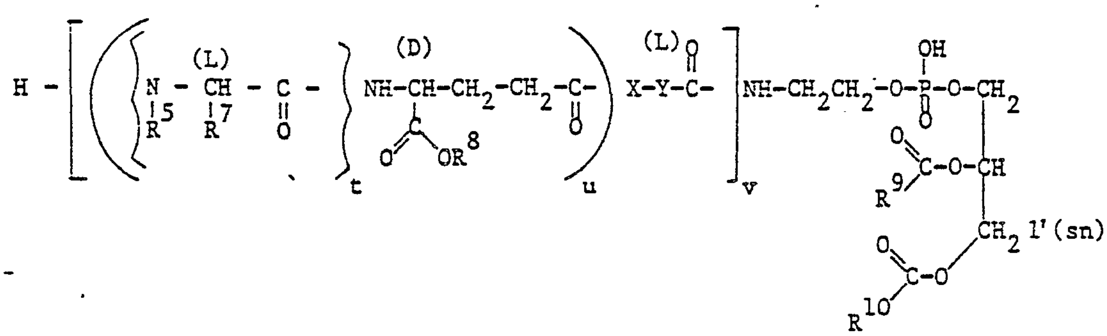

(VI)

in which each of t, u and v, independently of the others, represents 0 or 1 and the substituents have the meanings given above, with the proviso that each of t, u and v represents 1 if in the reactant of the formula V q represents 0, or that t represents 0, u represents 1 and v represents 1 if q represents 1 and r represents 0, or that u represents 0 and v represents 1 if q represents 1, r represents 1 and s represents 0, or that v represents 0 if each of q, r and s represents 1, or with a reactive derivative thereof, and removing any

protecting groups present, or

d) reacting a compound of the formula VII,

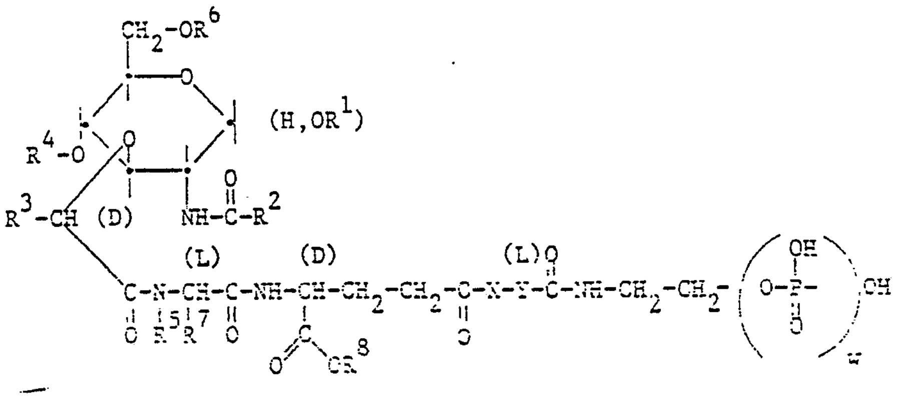

(VII)

in which w represents 0 or 1 and the substituents have the meanings given above, with the proviso that any free functional groups, with the exception of the group participating in the reaction, are in unprotected or protected form, or a reactive phosphoric acid derivative of such an acid of the formula VII in which w represents 1, with a compound of the formula VIII,

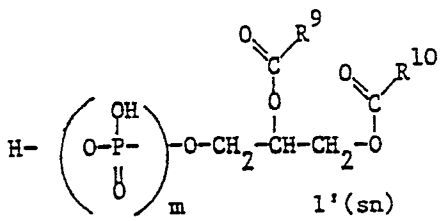

(VIII)

in which the substituents have the meanings given above and m represents 0 or 1, with the proviso that m represents 1 if in the reactant of the formula VII w represents 0, or that m represents 0 if w represents 1, or with a reactive phosphoric acid derivative of an acid of the formula VIII in which m represents 1, and removing any protecting groups present, or

e) reacting the reactive derivative of a compound of the formula VII,

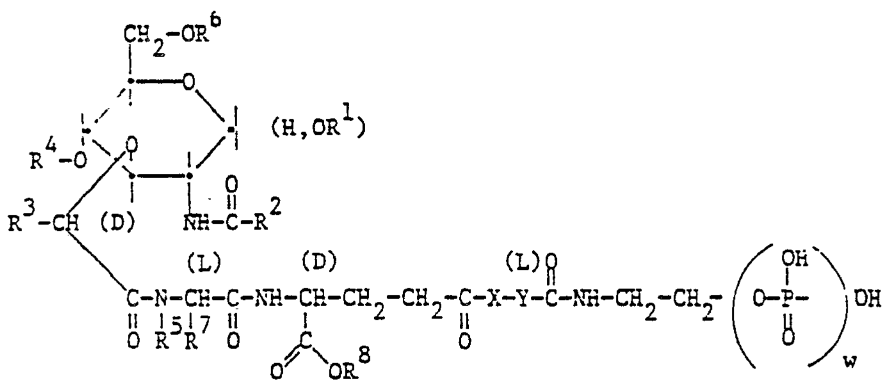

(VII)

in which w represents 0 or 1 and the substituents have the meanings given above, with the proviso that free functional groups present in a compond of the formula VII, with the exception of the group participating in the reaction, are unprotected or protected by readily removable protecting groups, and with the proviso that the reactive derivative of a compound of the formula VII in which w represents 0 is one having a reactive, esterified terminal hydroxy group, and with the proviso that the reactive derivative of a compound of the formula VII in which w represents 1 is a salt, with the reactive derivative of a compound of the formula VIII,

(See formula VIII page 48)

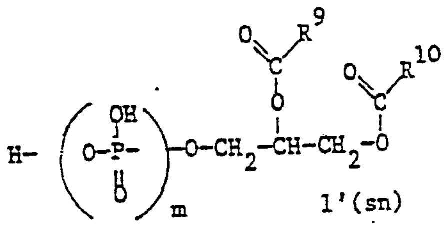

(VIII)

in which the substituents have the meanings given above and m represents 0 or 1, with the proviso that m represents 1 if in the reactant of the formula VII w represents 0, the reactive derivative of a compound of the formula VIII being a salt, or that m represents 0 if w represents 1, the reactive derivative of a compound of the formula VIII being one having a reactive, esterified terminal hydroxy group, and removing any protecting groups present, or

f) reacting a compound of the formula IX,

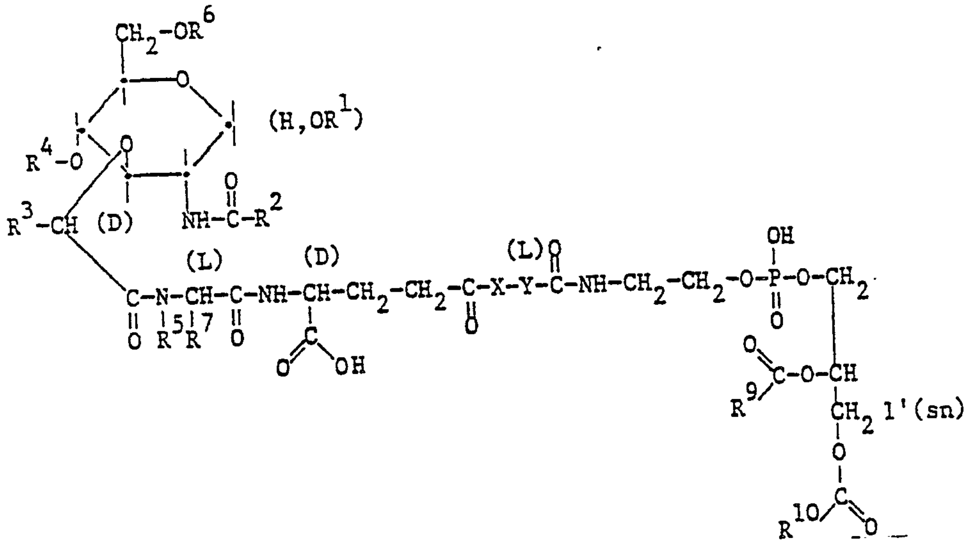

(IX)

in which the substituents have the above meanings, free functional groups present in a compound of the formula IX, with the exception of the group participating in the reaction, being in unprotected or protected form, or a reactive carboxylic acid derivative of a compound of the formula IX, with a lower alkanol $R^8$—OH in which $R^8$ represents $C_1$-$C_7$-alkyl, or with a reactive derivative thereof, and removing any protecting groups present, or

g) oxidising a compound of the formula X,

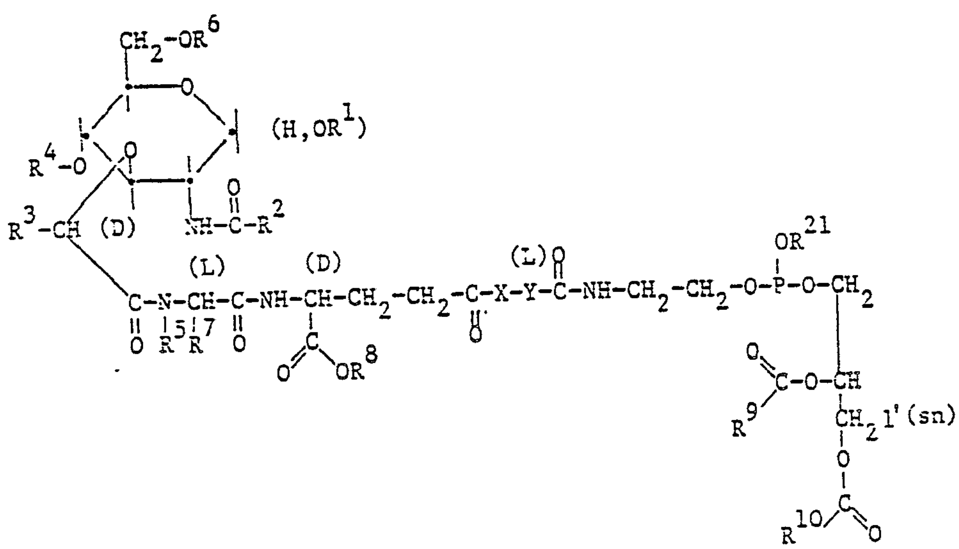

(X)

in which $R^{21}$ represents hydrogen or a protecting group and the remaining substituents have the meanings given above, free functional groups present in a compound of the formula X, with the exception of the group participating in the reaction, being in unprotected or protected form, or a tautomer of this compound, with an oxidising agent, and removing any protecting groups present, or

h) in a furanose compound of the formula XI,

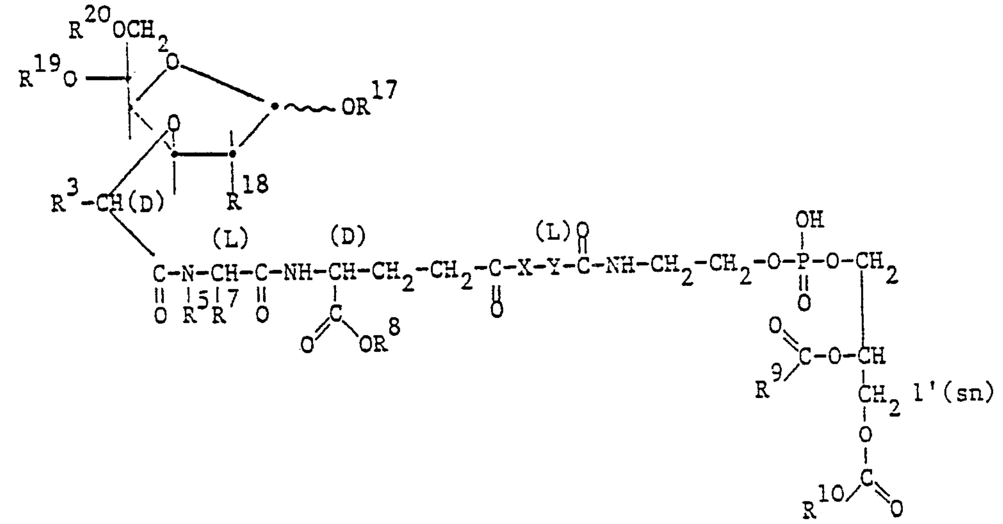

(XI)

in which $R^{17}$ represents hydrogen or a readily removable hydroxy-protecting group and $R^{18}$ has the meaning given above for the radical

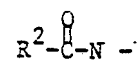

or $R^{17}$ and $R^{18}$ together represent a bivalent protecting group of the formula

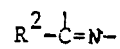

bonded by the free valency of the C atom to oxygen, in which $R^2$ has the meaning given above, and $R^{19}$ and $R^{20}$ represent hydrogen or a readily removable hydroxy-protecting group, or $R^{19}$ and $R^{20}$ together represent a bivalent hydroxy-protecting group, and the remaining substituents have the meanings given above, removing the protecting groups, or

i) in a compound of the formula I in which at least one of the radicals $R^1$, $R^2$, $R^4$, $R^6$, $R^7$ and $R^8$ is in protected form, removing the protecting group(s), or

j) hydrolysing a compound of the formula XII,

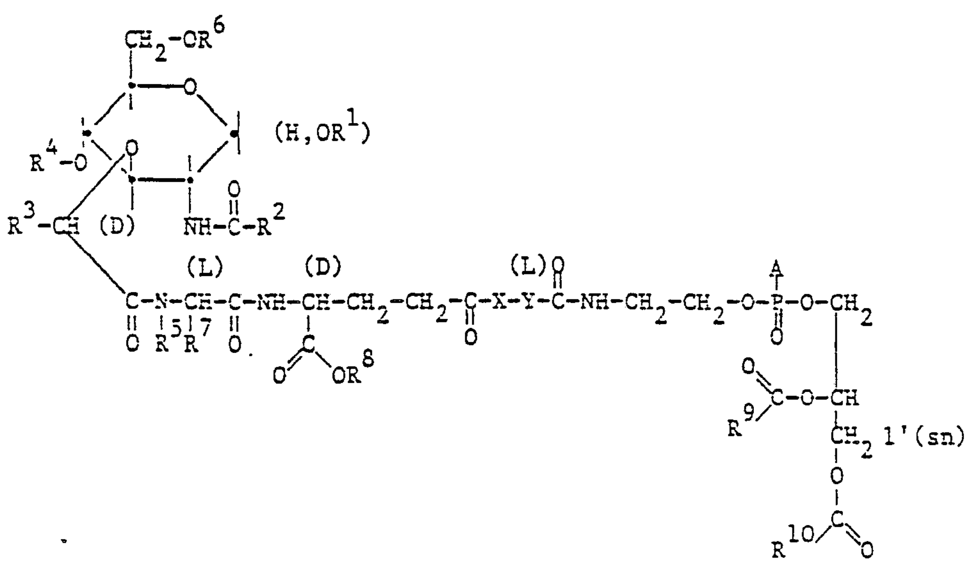

(XII)

in which A represents a halogen atom and the remaining substituents have the meanings given above, and, if desired, after carrying out one of the processes a) to j), converting a resultant compound of the formula I into its salt or converting a resultant salt into a different salt.

27. A compound obtainable in accordance with the processes of claim 26.

**Claims** (for the Contracting State AT)

1. A process for the preparation of a compound of the formula I

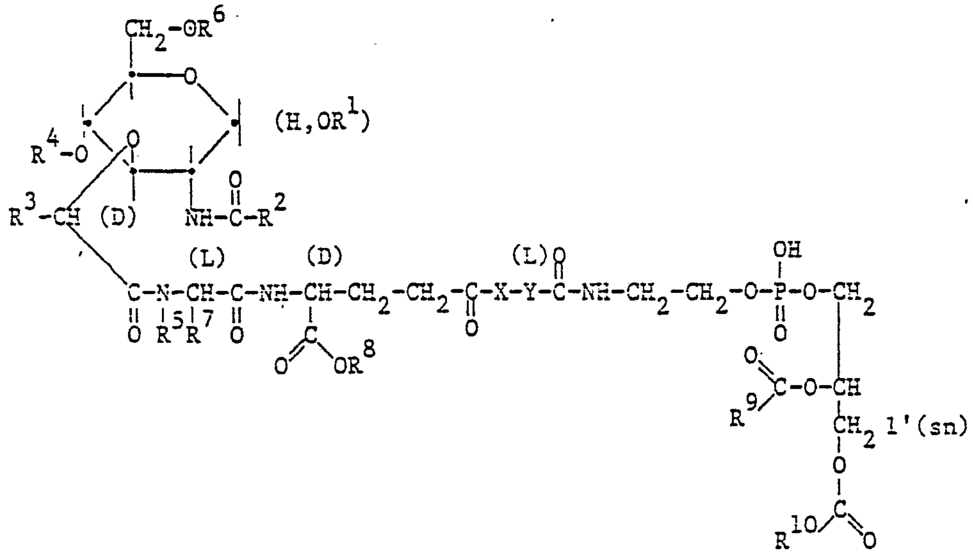

(I)

in which each of $R^1$, $R^4$ and $R^6$, independently of the others, represents hydrogen or lower alkanoyl, $R^2$ represents $C_{1-4}$-alkyl, hydroxymethyl or phenyl, $R^3$ represents hydrogen or methyl, $R^5$ represents hydrogen or $C_{1-3}$-alkyl, $R^7$ represents $C_{1-3}$-alkyl that is unsubstituted or substituted by hydroxy, mercapto or methylthio, $R^8$ represents hydrogen or lower alkyl, X represents oxygen or the group NH, Y represents $C_{1-4}$-alkylidene, and each of $R^9$ and $R^{10}$, independently of the other, represents $C_{11-17}$-alkyl or $C_{11-17}$-alkenyl, with the term « lower » signifying radicals containing from 1 up to and including 7 carbon atoms, and/or of a salt thereof, which process comprises

a) acylating a compound of the formula II,

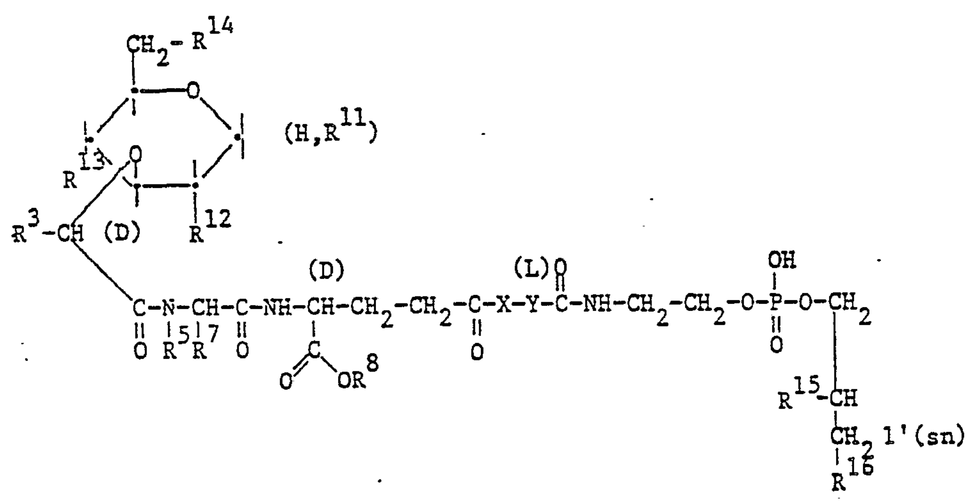

(II)

in which at least one of the radicals $R^{11}$, $R^{13}$, $R^{14}$, $R^{15}$ and $R^{16}$ represents hydroxy that is free or in reactive form, and/or in which $R^{12}$ represents amino that is free or in reactive form, and the remaining substituents have the meanings given above, with the proviso that functional groups present in a compound of the formula II, with the exception of the group(s) participating in the reaction, are in unprotected or protected form, with a carboxylic acid or a reactive derivative thereof to introduce at least one acyl radical $R^1$,

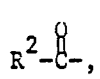

$R^4$, $R^6$,

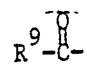

or

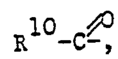

in which the substituents have the meanings given above, and removing any protecting groups present, or

b) reacting a compound of the formula III

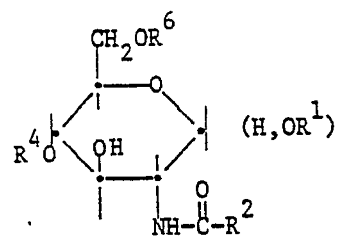

(III)

in which the substituents have the meanings given above and any functional groups present, with the exception of the free hydroxy group in the 3-position, are in unprotected or protected from, which 3-hydroxy group may be present in reactive form, with a compound of the formula IV,

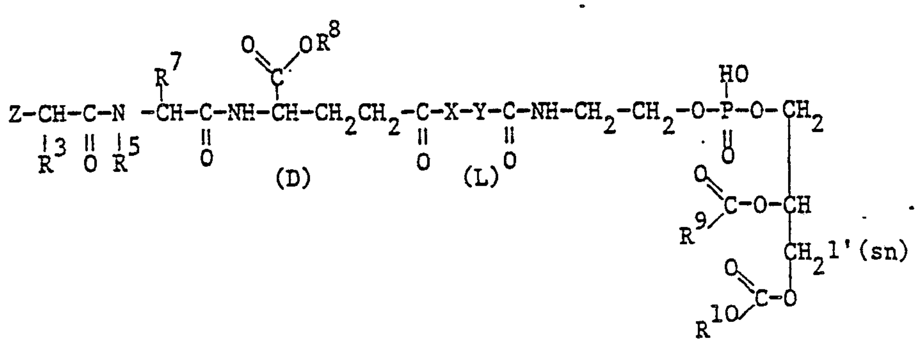

(IV)

in which Z represents a hydroxy group which may be present in reactive form and the remaining substituents have the meanings given above, with the proviso that functional groups present in a compound of the formula IV, with the exception of Z, are in unprotected or protected form, and removing any protecting groups present, or

c) reacting a compound of the formula V,

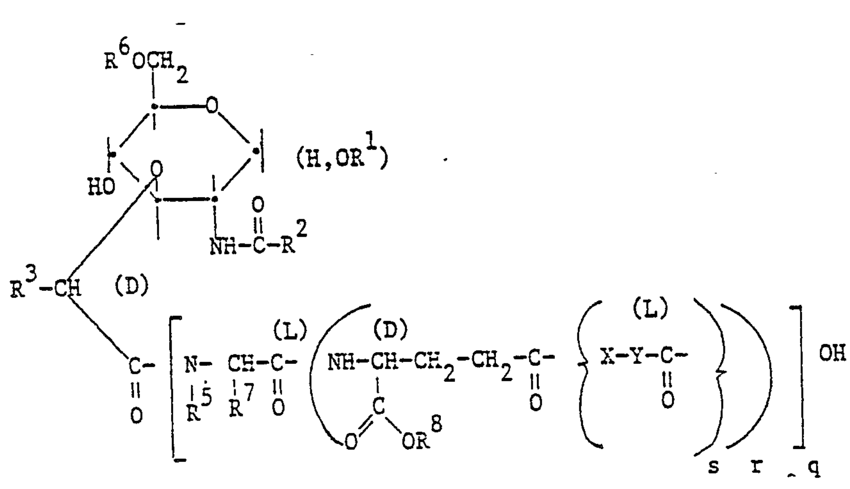

(V)

in which each of s, r and q, independently of the others, represents 0 or 1 and $R^8$ represents lower alkyl or a carboxy-protecting group, and the remaining substituents have the meanings given above, with the proviso that free functional groups present in a compound of the formula V, with the exception of the group participating in the reaction, are in unprotected or protected form, or a reactive carboxylic acid derivative thereof, with a compound of the formula VI,

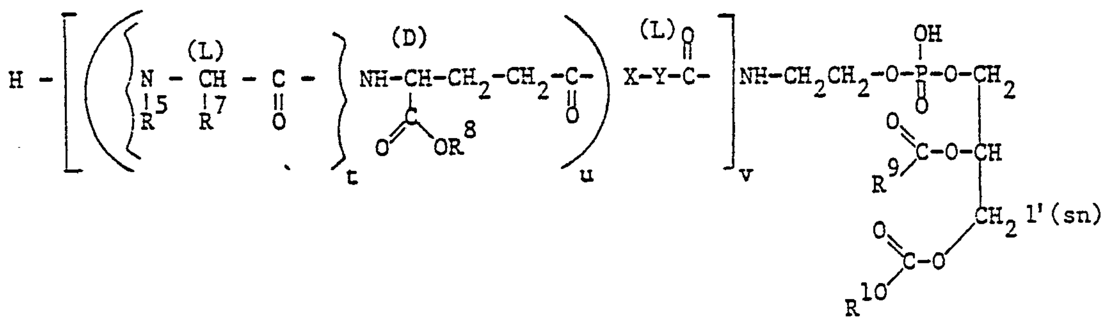

(VI)

in which each of t, u and v, independently of the others, represents 0 or 1 and the substituents have the meanings given above, with the proviso that each of t, u and v represents 1 if in the reactant of the formula V q represents 0, or that t represents 0, u represents 1 and v represents 1 if q represents 1 and r represents 0, or that u represents 0 and v represents 1 if q represents 1, r represents 1 and s represents 0, or that v represents 0 if each of q, r and s represents 1, or with a reactive derivative thereof, and removing any protecting groups present, or

d) reacting a compound of the formula VII,

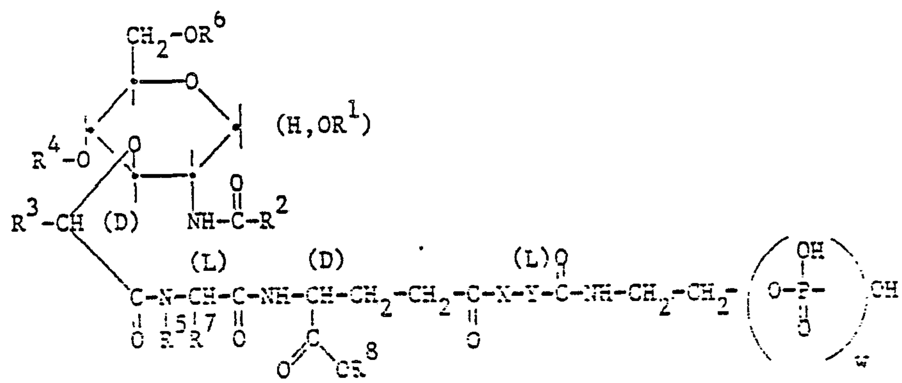

(VII)

in which w represents 0 or 1 and the substituents have the meanings given above, with the proviso that any free functional groups, with the exception of the group participating in the reaction, are in unprotected or protected form, or a reactive phosphoric acid derivative of such an acid of the formula VII in which w represents 1, with a compound of the formula VIII,

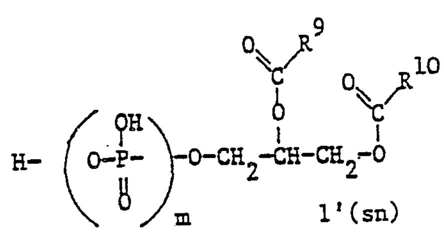

(VIII)

in which the substituents have the meanings given above and m represents 0 or 1, with the proviso that m represents 1 if in the reactant of the formula VII w represents 0, or that m represents 0 if w represents 1, or with a reactive phosphoric acid derivative of an acid of the formula VIII in which m represents 1, and removing any protecting groups present, or

e) reacting the reactive derivative of a compound of the formula VII,

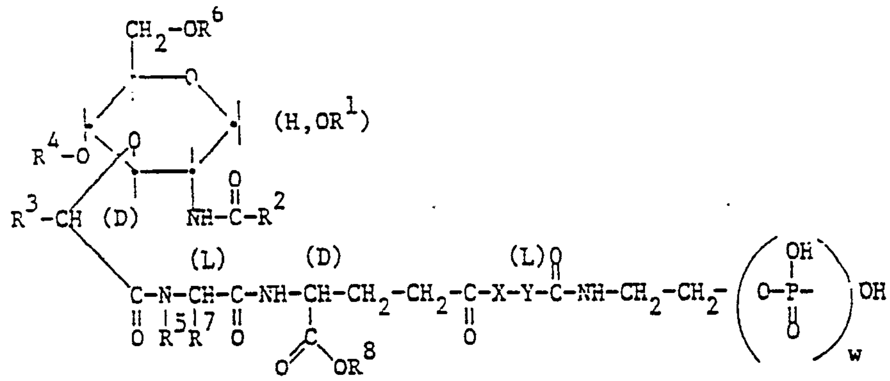

(VII)

in which w represents 0 or 1 and the substituents have the meanings given above, with the proviso that free functional groups present in a compound of the formula VII, with the exception of the group participating in the reaction, are unprotected or protected by readily removable protecting groups, and with the proviso that the reactive derivative of a compound of the formula VII in which w represents 0 is one having a reactive, esterified terminal hydroxy group, and with the proviso that the reactive derivative of a compound of the formula VII in which w represents 1 is a salt, with the reactive derivative of a compound of the formula VIII,

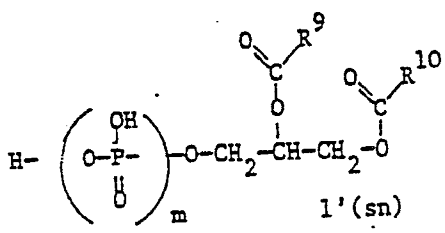

(VIII)

in which the substituents have the meanings given above and m represents 0 or 1, with the proviso that m represents 1 if in the reactant of the formula VII w represents 0, the reactive derivative of a compound of the formula VIII being a salt, or that m represents 0 if w represents 1, the reactive derivative of a compound of the formula VIII being one having a reactive, esterified terminal hydroxy group, and removing any protecting groups present, or

f) reacting a compound of the formula IX,

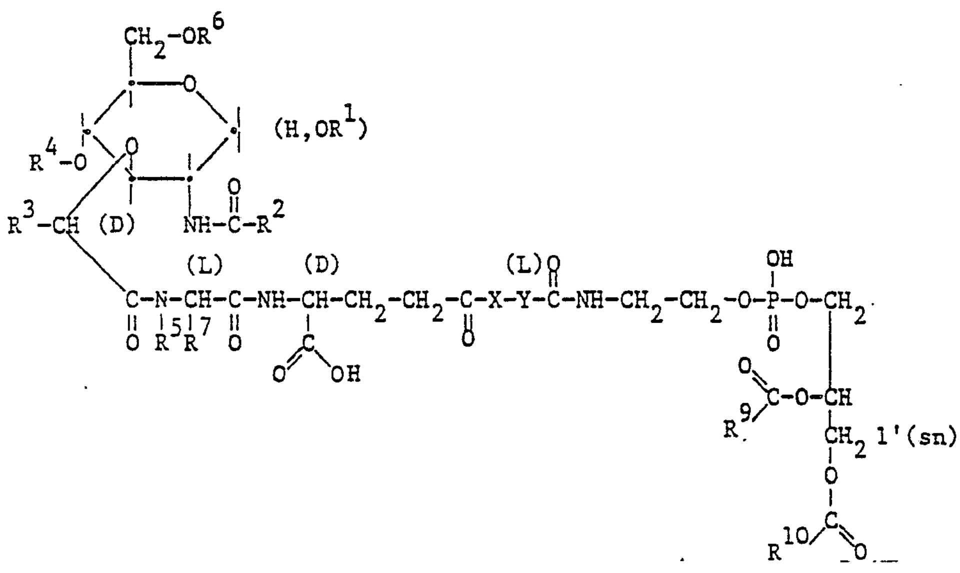

(IX)

in which the substituents have the above meanings, free functional groups present in a compound of the formula IX, with the exception of the group participating in the reaction, being in unprotected or protected form, or a reactive carboxylic acid derivative of a compound of the formula IX, with a lower alkanol $R^8$—OH in which $R^8$ represents $C_1$-$C_7$-alkyl, or with a reactive derivative thereof, and removing any protecting groups present, or

g) oxidising a compound of the formula X,

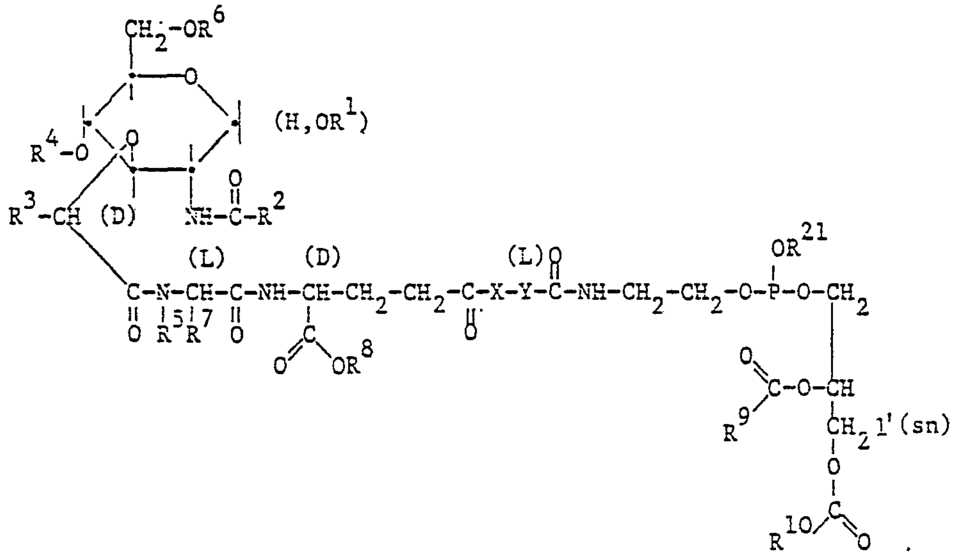

(X)

in which $R^{21}$ represents hydrogen or a protecting group and the remaining substituents have the meanings given above, free functional groups present in a compound of the formula X, with the exception of the group participating in the reaction, being in unprotected or protected form, or a tautomer of this compound, with an oxidising agent, and removing any protecting groups present, or

h) in a furanose compound of the formula XI,

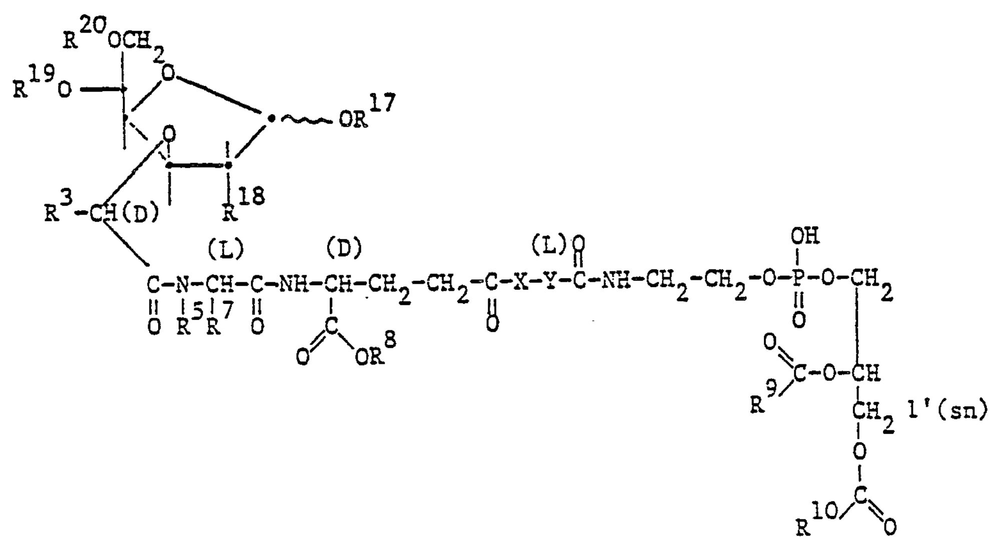

(XI)

in which $R^{17}$ represents hydrogen or a readily removable hydroxy-protecting group and $R^{18}$ has the meaning given above for the radical

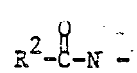

or $R^{17}$ and $R^{18}$ together represent a bivalent protecting group of the formula

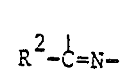

bonded by the free valency of the C atom to oxygen, in which $R^2$ has the meaning given above, and $R^{19}$ and $R^{20}$ represent hydrogen or a readily removable hydroxy-protecting group, or $R^{19}$ and $R^{20}$ together represent a bivalent hydroxy-protecting group, and the remaining substituents have the meanings given above, removing the protecting groups, or

i) in a compound of the formula I in which at least one of the radicals $R^1$, $R^2$, $R^4$, $R^6$, $R^7$ and $R^8$ is in protected form, removing the protecting group(s), or

j) hydrolysing a compound of the formula XII,

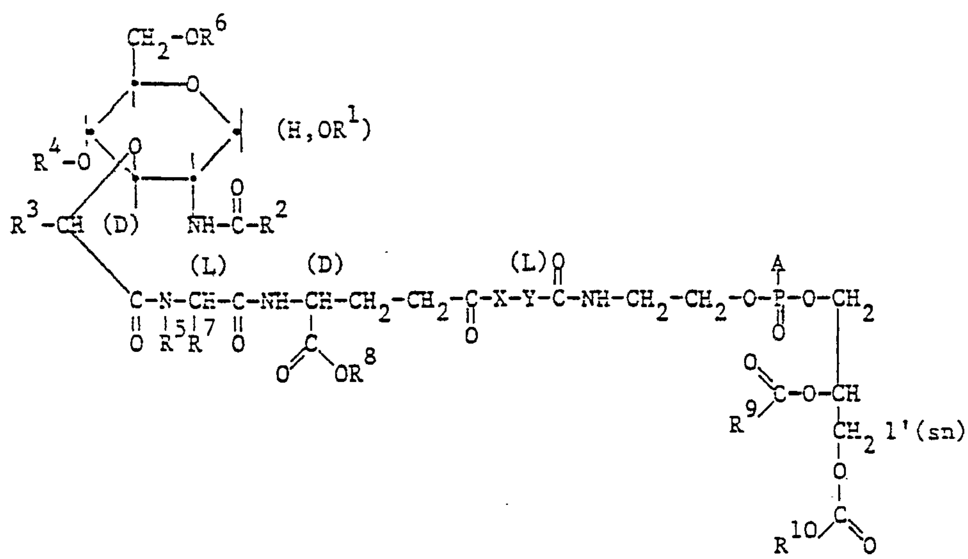

(XII)

in which A represents a halogen atom and the remaining substituents have the meanings given above, and, if desired, after carrying out one of the processes a) to j), converting a resultant compound of the formula I into its salt or converting a resultant salt into a different salt.

2. A process according to claim 1, in which the starting materials are chosen such that a compound of the formula I is obtained, in which each of $R^1$, $R^4$ and $R^6$, independently of the others, represents hydrogen or $C_{1-3}$-alkanoyl, $R^2$ represents $C_{1-2}$-alkyl or hydroxymethyl, $R^3$ represents hydrogen or methyl, $R^5$ represents hydrogen or methyl, $R^7$ represents $C_{1-3}$-alkyl or hydroxymethyl, $R^8$ represents hydrogen or $C_{1-4}$-alkyl, X represents oxygen or the group NH, Y represents $C_{1-3}$-alkylidene and each of $R^9$ and $R^{10}$, independently of the other, represents straight chain $C_{11-17}$-alkyl or decarbonyloleoyl, and/or a salt thereof.

3. A process according to claim 2, in which the starting materials are chosen such that a compound of the formula I is obtained, in which each of $R^1$, $R^4$ and $R^6$, independently of the others, represents hydrogen or acetyl, $R^2$ represents methyl or ethyl, $R^3$ represents hydrogen or methyl, $R^5$ represents hydrogen or methyl, $R^7$ represents $C_{1-3}$-alkyl, $R^8$ represents hydrogen or $C_{1-4}$-alkyl, X represents oxygen or the group NH, Y represents $C_{1-2}$-alkylidene and each of $R^9$ and $R^{10}$, independently of the other, represents straight chain $C_{11-17}$-alkyl having an uneven number of C-atoms or decarbonyloleoyl, and/or a salt thereof.

4. A process according to claims 1-3, in which the starting materials are chosen such that a compound of the formula I is obtained, in which $R^8$ represents the alkyl radical mentioned in these claims, and/or a salt thereof.

5. A process according to claims 1-4, in which the starting materials are chosen such that a compound of the formula I is obstained, in which at least one of the radicals $R^1$, $R^4$ and $R^6$ represents lower alkanoyl, and/or a salt thereof.

6. A process according to claims 1-4, in which the starting materials are chosen such that a compound of the formula I is obtained, in which each of $R^1$, $R^4$ and $R^6$ represents hydrogen, and/or a salt thereof.

7. A process according to claim 3, in which the starting materials are chosen such that a compound of the formula I is obtained, in which each of $R^1$, $R^4$ and $R^6$ represents hydrogen, $R^2$ represents methyl or ethyl, $R^3$ represents hydrogen if $R^2$ represents ethyl, or methyl if $R^2$ represents methyl, $R^5$ represents hydrogen or methyl, $R^7$ represents $C_{1-2}$-alkyl, $R^8$ represents straight chain $C_{1-4}$-alkyl, X represents oxygen or the group NH, Y represents ethylidene and each of $R^9$ and $R^{10}$, independently of the other, represents straight chain $C_{15-17}$-alkyl having an uneven number of C-atoms, and/or a salt thereof.

8. A process according to claims 1-7, in which the starting materials are chosen such that a compound of the formula I is obtained, in which $R^3$ represents methyl, and/or a salt thereof.

9. A process according to claims 1-8, in which the starting materials are chosen such that a compound of the formula I is obtained, in which $R^5$ represents hydrogen, and/or a salt thereof.

10. A process according to claims 1-9, in which the starting materials are chosen such that a compound of the formula I is obtained, in which $R^8$ represents n-butyl, and/or a salt thereof.

11. A process according to claims 1-10, in which the starting materials are chosen such that a compound of the formula I is obtained, in which X represents the group NH, and/or a salt thereof.

12. A process according to claims 1-11, in which the starting materials are chosen such that a compound of the formula I is obtained, in which $R^9$ and $R^{10}$ represent n-pentadecyl, and/or a salt thereof.

13. A process according to claim 1, in which the starting materials are chosen such that N-acetylmuramyl-L-alanyl-D-glutamyl-($C_\alpha$)-n-butyl ester-($C_\gamma$)-L-alanine-2-(1,2-dipalmitoyl-sn-glycero-3-hydroxyphosphoryloxy)-ethylamide is obtained, and/or a salt thereof.

14. A process according to claim 1, in which the starting materials are chosen such that N-propionyldesmethylmuramyl-L-alanyl-D-glutamyl-($C_\alpha$)-n-butyl ester-($C_\gamma$)-L-alanine-2-(1,2-dipalmitoyl-sn-glycero-3-hydroxyphosphoryloxy)-ethylamide is obtained, and/or a salt thereof.

15. A process according to claim 1, in which the starting materials are chosen such that N-acetylmuramyl-L-alanyl-D-glutamyl-($C_\alpha$)-methyl ester-($C_\gamma$)-L-alanine-2-(1,2-dipalmitoyl-sn-glycero-3-hydroxyphosphoryloxy)-ethylamide is obtained, and/or a salt thereof.

16. A process according to claim 1, in which the starting materials are chosen such that N-propionyldesmethylmuramyl-L-alanyl-D-glutamyl-($C_\alpha$)-methyl ester(-$C_\gamma$)-L-alanine-2-(1,2-dipalmitoyl-sn-glycero-3-hydroxyphosphoryloxy)-ethylamide is obtained, and/or a salt thereof.

17. A process according to claim 1, in which the starting materials are chosen such that N-acetylmuramyl-L-alanyl-D-glutamic acid-($C_\gamma$)-L-alanine-2-(1,2-dipalmitoyl-sn-glycero-3-hydroxyphosphoryloxy)-ethylamide is obtained, and/or a salt thereof.

18. A process according to any one of claims 1 to 17, in which the starting materials are chosen such that a pharmaceutically acceptable salt is obtained.

19. A process according to claim 18, in which the starting materials are chosen such that a sodium salt is obtained.

**Revendications** (pour les Etats contractants : BE, CH, DE, FR, GB, IT, LI, LU, NL et SE)

1. Composés de formule I

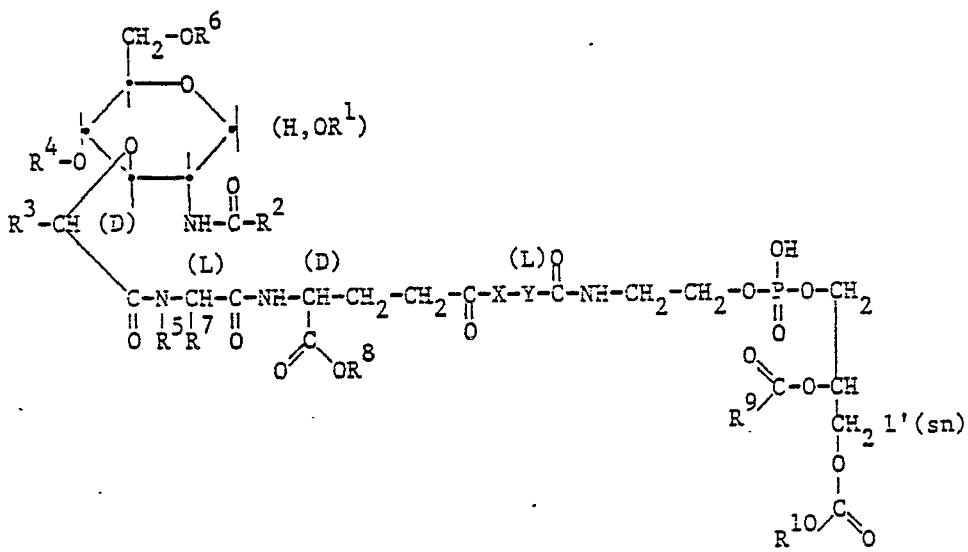

(I)

dans laquelle $R^1$, $R^4$ et $R^6$ représentent chacun, indépendamment les uns des autres, l'hydrogène ou un groupe alcanoyle inférieur, $R^2$ représente un groupe alkyle en C1-C4, hydroxyméthyle ou phényle, $R^3$ représente l'hydrogène ou un groupe méthyle, $R^5$ représente l'hydrogène ou un groupe alkyle en C1-C3, $R^7$ représente un groupe alkyle en C1-C3 non substitué ou substitué par des groupes hydroxy, mercapto ou méthylthio, $R^8$ représente l'hydrogène ou un groupe alkyle inférieur, X représente l'oxygène ou le groupe NH, Y représente un groupe alkylidène en C1-C4 et $R^9$ et $R^{10}$ représentent chacun, indépendamment l'un de l'autre, un groupe alkyle en C11-C17 ou alcényle en C11-C17, le qualificatif « inférieur » s'appliquant à des groupes qui contiennent de 1 à 7 atomes de carbone inclus, et leurs sels.

2. Composés de formule I selon la rev. 1, dans laquelle $R^1$, $R^4$ et $R^6$ représentent chacun, indépendamment les uns des autres, l'hydrogène ou un groupe alcanoyle en C1-C3, $R^2$ représente un groupe alkyke en C1-C2 ou hydroxyméthyle, $R^3$ représente l'hydrogène ou un groupe méthyle, $R^5$ représente l'hydrogène ou un groupe méthyle, $R^7$ représente un groupe alkyle en C1-C3 ou hydroxyméthyle, $R^8$ représente l'hydrogène ou un groupe alkyle en C1-C4, X représente l'oxygène ou le groupe NH, Y représente un groupe alkylidène en C1-C3 et $R^9$ et $R^{10}$ représentent chacun, indépendamment l'un de l'autre, un groupe alkyle à chaîne droite en C11-C17 ou un groupe descarbonyl-oléoyle, et leurs sels.

3. Composés de formule I selon la rev. 2, dans laquelle $R^1$, $R^4$ et $R^6$ représentent chacun, indépendamment les uns des autres, l'hydrogène ou un groupe acétyle, $R^2$ représente un groupe méthyle ou éthyle, $R^3$ représente l'hydrogène ou un groupe méthyle, $R^5$ représente l'hydrogène ou un groupe méthyle, $R^7$ représente un groupe alkyle en C1-C3, $R^8$ représente l'hydrogène ou un groupe alkyle en C1-C4, X représente l'oxygène ou le groupe NH, Y représente un groupe alkylidène en C1-C2 et $R^9$ et $R^{10}$ représentent chacun, indépendamment l'un de l'autre, un groupe alkyle à chaîne droite en C11-C17 et à nombre impair d'atomes de carbone ou un groupe descarbonyl-oléoyle, et leurs sels.

4. Composés de formule I selon l'une des rev. 1 à 3, dans lesquelles $R^8$ représente un groupe alkyle tel que défini dans ces revendications, et leurs sels.

5. Composés de formule I selon l'une des rev. 1 à 4, dans lesquelles l'un au moins des symboles $R^1$, $R^4$ et $R^6$ représente un groupe alcanoyle inférieur, et leurs sels.

6. Composés de formule I selon l'une des rev. 1 à 4, dans lesquelles $R^1$, $R^4$ et $R^6$ représentent l'hydrogène, et leurs sels.

7. Composés de formule I selon la rev. 3, dans lesquelles $R^1$, $R^4$ et $R^6$ représentent l'hydrogène, $R^2$ représente un groupe méthyle ou éthyle, $R^3$ représente l'hydrogène lorsque $R^2$ représente un groupe éthyle, ou un groupe méthyle lorsque $R^2$ représente un groupe méthyle, $R^5$ représente l'hydrogène ou un groupe méthyle, $R^7$ représente un groupe alkyle en C1-C2, $R^8$ représente un groupe alkyle à chaîne droite en C1-C4, X représente l'oxygène ou le groupe NH, Y représente un groupe éthylidène et $R^9$ et $R^{10}$ représentent chacun, indépendamment l'un de l'autre, un groupe alkyle à chaîne droite en C15-C17 et un nombre impair d'atomes de carbone, et leurs sels.

8. Composés de formule I selon l'une des rev. 1 à 7, dans lesquelles $R^3$ représente un groupe méthyle, et leurs sels.

9. Composés de formule I selon l'une des rev. 1 à 8, dans lesquelles $R^5$ représente l'hydrogène, et leurs sels.

10. Composés de formule I selon l'une des rev. 1 à 9, dans lesquelles $R^8$ représente un groupe n-butyle, et leurs sels.

11. Composés de formule I selon l'une des rev. 1 à 10, dans lesquelles X représente le groupe NH, et leurs sels.

12. Composés de formule I selon l'une des rev. 1 à 11, dans lesquelles $R^9$ et $R^{10}$ représentent des groupes n-pentadécyle, et leurs sels.

13. Le N-acétylmuramyl-L-alanyl-D-glutamyl-($C_\alpha$)-(ester n-butylique)-($C_\gamma$)-L-alanine-2-(1,2-dipalmitoyl-sn-glycéro-3-hydroxyphosphoryloxy)-éthylamide et ses sels selon la rev. 1.

14. Le N-propionyl-desméthylmuramyl-L-alanyl-D-glutamyl-($C_\alpha$)-(ester n-butylique)-($C_\gamma$)-L-alanine-2-(1,2-dipalmitoyl-sn-glycéro-3-hydroxyphosphoryloxy)-éthylamide et ses sels selon la rev. 1.

15. Le N-acétylmuramyl-L-alanyl-D-glutamyl-($C_\alpha$)-(ester méthylique)-($C_\gamma$)-L-alanine-2-(1,2-dipalmitoyl-sn-glycéro-3-hydroxyphosphoryloxy)-éthylamide et ses sels selon la rev. 1.

16. Le N-propionyl-desméthylmuramyl-L-alanyl-D-glutamyl-($C_\alpha$)-(ester méthylique)-($C_\gamma$)-L-alanine-2-(1,2-dipalmitoyl-sn-glycéro-3-hydroxyphosphoryloxy)-éthylamide et ses sels selon la rev. 1.

17. Le N-acétylmuramyl-L-alanyl-D-glutamyl-($C_\alpha$)-(ester tert.-butylique)-($C_\gamma$)-L-alanine-2-(1,2-dipalmitoyl-sn-glycéro-3-hydroxyphosphoryloxy)-éthylamide et ses sels selon la rev. 1.

18. Le N-propionyl-desméthylmuramyl-L-alanyl-D-glutamyl-($C_\alpha$)-(ester tert.-butylique)-($C_\gamma$)-L-alanine-2-(1,2-dipalmitoyl-sn-glycéro-3-hydroxyphosphoryloxy)-éthylamide et ses sels selon la rev. 1.

19. Le ($C_\gamma$)-L-alanine-2-(1,2-dipalmitoyl-sn-glycéro-3-hydroxyphosphoryloxy)-éthylamide de l'acide N-acétylmuramyl-L-alanyl-D-glutamique et ses sels selon la rev. 1.

20. Le ($C_\gamma$)-L-alanine-2-(1,2-dipalmitoyl-sn-glycéro-3-hydroxyphosphoryloxy)-éthylamide de l'acide N-propionyl-desméthylmuramyl-L-alanyl-D-glutamique et ses sels selon la rev. 1.

21. Sels acceptables pour l'usage pharmaceutique selon l'une des rev. 1 à 20.

22. Sels de sodium selon la rev. 21.

23. Compositions pharmaceutiques contenant un composé selon l'une des rev. 1 à 22 avec un véhicule pharmaceutique.

24. Un composé selon l'une des rev. 1 à 22 pour l'utilisation dans un procédé pour le traitement de l'organisme humain ou animal.

25. Utilisation d'un composé selon l'une des rev. 1 à 22 pour la préparation de compositions pharmaceutiques servant à la prophylaxie et à la thérapie des infections virales.

26. Procédé de préparation des composés de formule I

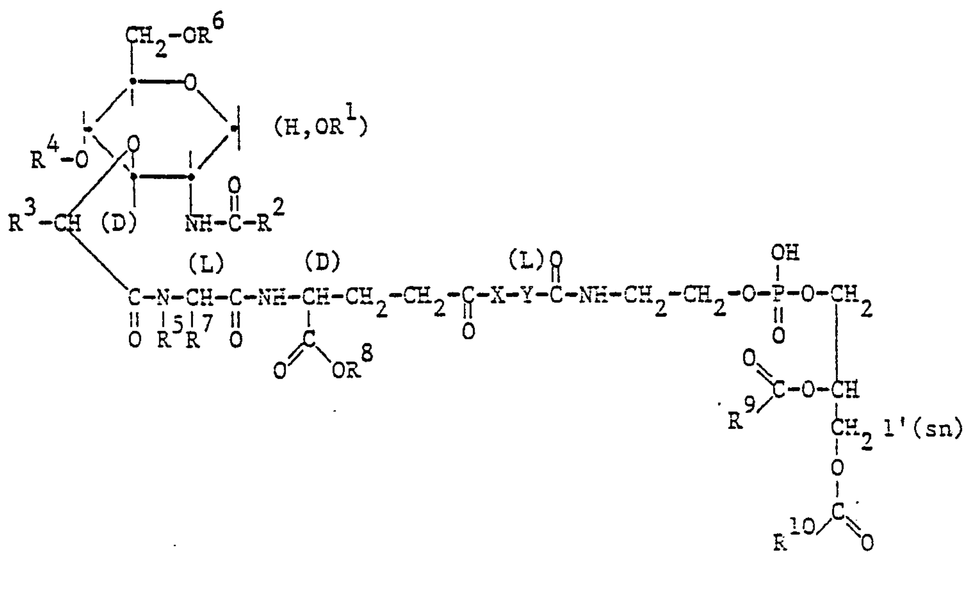

(I)

dans laquelle $R^1$, $R^4$ et $R^6$ représentent chacun, indépendamment les uns des autres, l'hydrogène ou un groupe alcanoyle inférieur, $R^2$ représente un groupe alkyle en C1-C4, hydroxyméthyle ou phényle, $R^3$ représente l'hydrogène ou un groupe méthyle, $R^5$ représente l'hydrogène ou un groupe alkyle en C1-C3, $R^7$ représente un groupe alkyle en C1-C3 non substitué ou substitué par des groupes hydroxy, mercapto ou méthylthio, $R^8$ représente l'hydrogène ou un groupe alkyle inférieur, X représente l'oxygène ou le groupe NH, Y représente un groupe alkylidène en C1-C4 et $R^9$ et $R^{10}$ représentent chacun, indépendamment l'un de l'autre, un groupe alkyle en C11-C17 ou alcényle en C11-C17, le qualificatif « inférieur » s'appliquant à des groupes contenant de 1 à 7 atomes de carbone inclus, et de leurs sels, caractérisé en ce que

a) on acyle un composé de formule II,

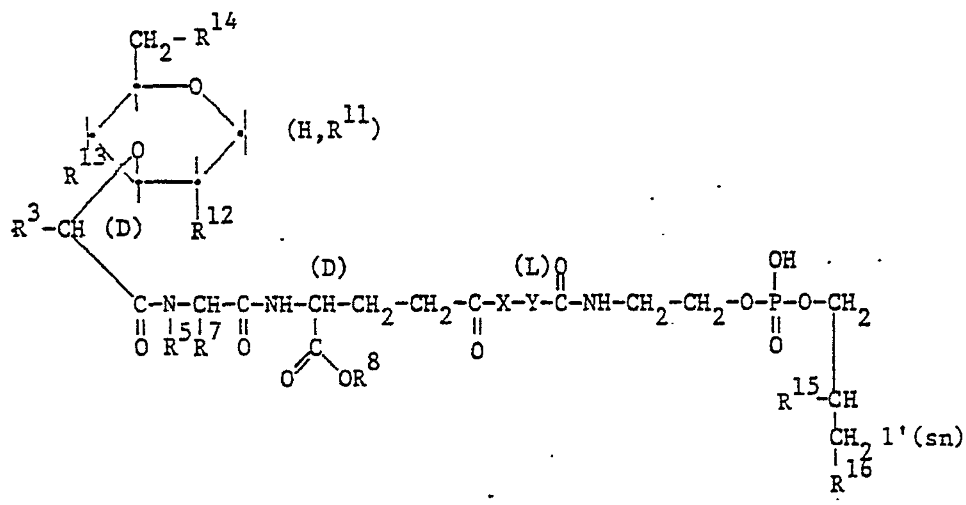

(II)

dans laquelle l'un au moins des symboles $R^{11}$, $R^{13}$, $R^{14}$, $R^{15}$ et $R^{16}$ représente un groupe hydroxy libre ou sous une forme réactive, et/ou $R^{12}$ représente un groupe amino libre ou sous une forme réactive, et les autres symboles ont les significations indiquées ci-dessus, étant spécifié que les groupes fonctionnels présents dans un composé de formule II, à l'exception du ou des groupes participant à la réaction, sont éventuellement à l'état protégé, à l'aide d'un acide carboxylique ou d'un dérivé réactif d'un tel acide, avec introduction d'au moins un groupe acyle $R^1$,

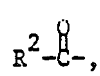

$R^4$, $R^6$,

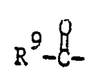

ou

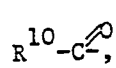

dans lequel les symboles ont les significations indiquées ci-dessus, et on élimine les groupes protecteurs éventuellement présents, ou bien

b) on fait réagir un composé de formule III

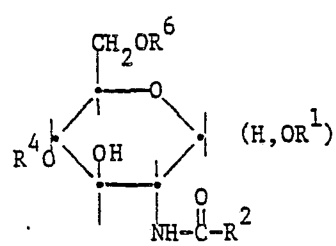

(III)

dans laquelle les symboles ont les significations indiquées ci-dessus et les groupes fonctionnels présents, à l'exception du groupe hydroxy libre en position 3, sont éventuellement à l'état protégé, le groupe 3-hydroxy étant éventuellement sous une forme réactive, avec un composé de formule IV,

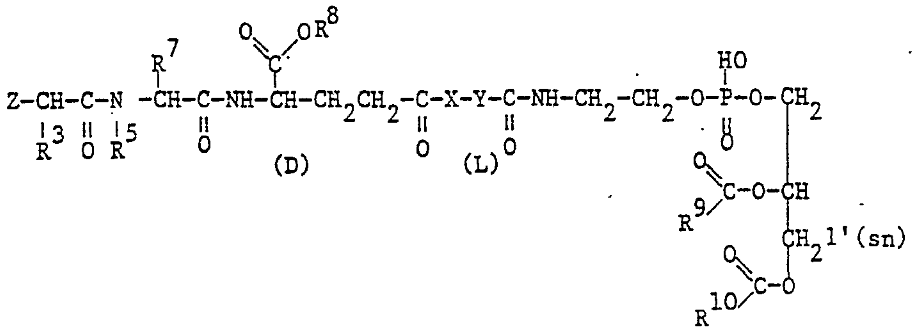

(IV)

dans laquelle Z représente un groupe hydroxy éventuellement à l'état réactif et les autres symboles ont les significations indiquées ci-dessus, étant spécifié que les groupes fonctionnels présents dans un composé de formule IV, à l'exception de Z, sont éventuellement à l'état protégé, et on élimine les groupes protecteurs présents, ou bien

c) on fait réagir un composé de formule V,

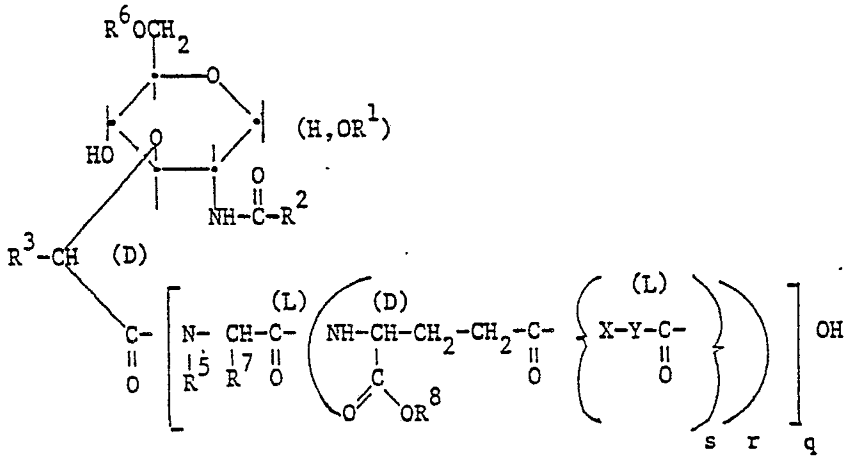

(V)

dans laquelle s, r et q sont chacun, indépendamment les uns des autres, égaux à 0 ou 1 et $R^8$ représente un groupe alkyle inférieur ou un groupe protecteur du groupe carbonyle et les autres symboles ont les significations indiquées ci-dessus, étant spécifié que les groupes fonctionnels libres présents dans un composé de formule V, à l'exception du groupe participant à la réaction, sont éventuellement à l'état protégé, ou un dérivé réactif d'un tel acide carboxylique, avec un composé de formule VI,

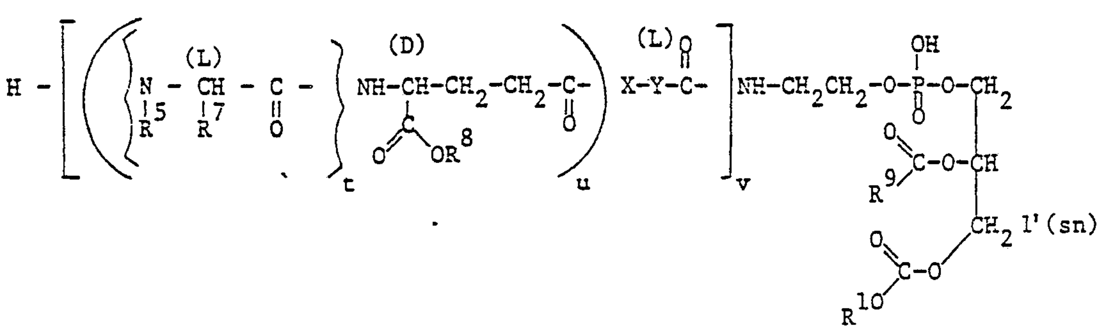

(VI)

dans laquelle t, u et v sont égaux chacun, indépendamment les uns des autres, à 0 ou 1 et les symboles ont les significations indiquées ci-dessus, étant spécifié que t, u et v sont égaux à 1 lorsque, dans le composant de réaction de formule V, q est égal à 0, ou que t est égal à 0, q est égal à 1 et v est égal à 1 lorsque q est égal à 1 et r à 0, ou que q est égal à 0 et v à 1 lorsque q est égal à 1, r à 1 et s à 0, ou que v est égal à 0 lorsque q, r et s sont égaux à 1, ou un dérivé réactif d'un tel composé, et on élimine les groupes protecteurs éventuellement présents, ou bien

d) on fait réagir un composé de formule VII,

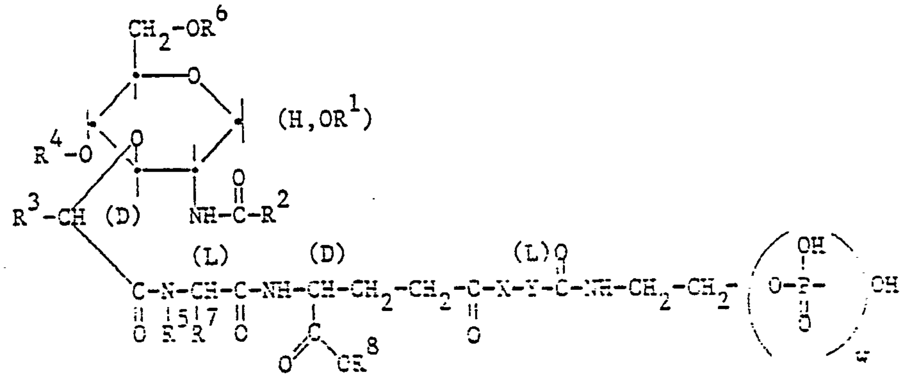

(VII)

dans laquelle w est égal à 0 ou 1 et les autres symboles ont les significations indiquées ci-dessus, étant spécifié que les groupes fonctionnels libres, à l'exception du groupe participant à la réaction, sont éventuellement à l'état protégé, ou un dérivé phosphorique réactif d'un tel acide de formule VII dans laquelle w est égal à 1, avec un composé de formule VIII,

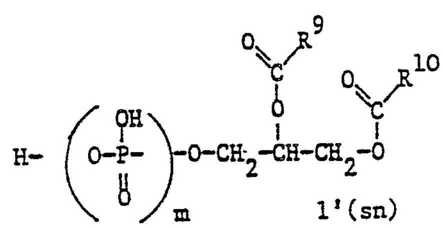

(VIII)

dans laquelle les symboles ont les significations indiquées ci-dessus et m est égal à 0 ou 1, étant spécifié que m est égal à 1 lorsque, dans le composant de la réaction répondant à la formule VII, w est égal à 0, ou que m est égal à 0 lorsque w est égal à 1, ou avec un dérivé phosphorique réactif d'un acide de formule VIII dans laquelle m est égal à 1, et on élimine les groupes protecteurs présents, ou bien

e) on fait réagir le dérivé réactif d'un composé de formule VII

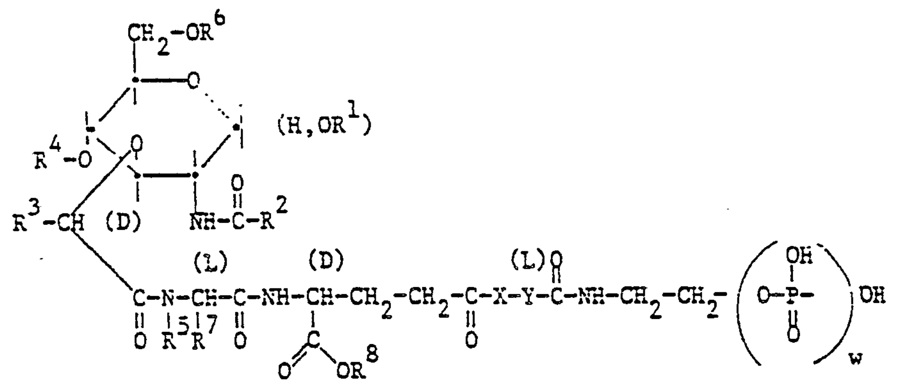

(VII)

dans laquelle w est égal à 0 ou 1 et les symboles ont les significations indiquées ci-dessus, étant spécifié que les groupes fonctionnels libres présents dans un composé de formule VII, à l'exception du groupe participant à la réaction, sont éventuellement protégés par des groupes protecteurs faciles à éliminer, et également que le dérivé réactif d'un composé de formule VII dans laquelle w est égal à 0 est un dérivé à groupe hydroxy terminal estérifié réactif, et également que le dérivé réactif d'un composé de formule VII dans laquelle w est égal à 1, est un sel, avec le dérivé réactif d'un composé de formule VIII,

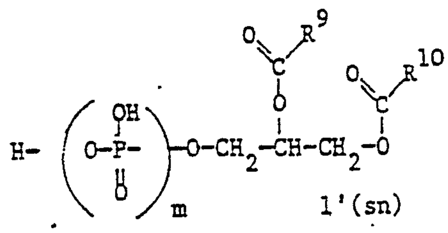

(VIII)

dans laquelle les symboles ont les significations indiquées ci-dessus et m est égal à 0 ou 1, étant spécifié que m est égal à 1 lorsque, dans le composant de la réaction répondant à la formule VII, w est égal à 0, le dérivé réactif d'un composé de formule VIII étant alors un sel, ou que m est égal à 0 lorsque w est égal à 1, le dérivé réactif d'un composé de formule VIII étant alors un dérivé à groupe hydroxy terminal estérifié réactif, et on élimine les groupes protecteurs présents, ou bien

f) on fait réagir un composé de formule IX,

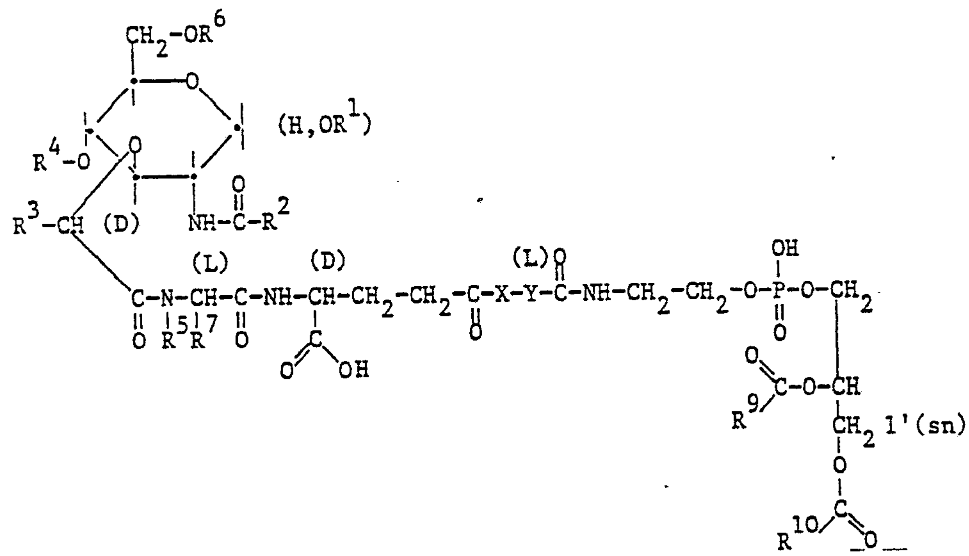

(IX)

dans laquelle les symboles ont les significations indiquées ci-dessus, les groupes fonctionnels libres présents dans un composé de formule IX, à l'exception du groupe participant à la réaction, étant éventuellement à l'état protégé, ou un dérivé d'acide carboxylique réactif d'un composé de formule IX, avec un alcanol inférieur $R^8$—OH, dans lequel $R^8$ représente un groupe alkyle en C1-C7, ou un dérivé réactif d'un tel alcanol, et on élimine les groupes protecteurs présents, ou bien

g) on oxyde à l'aide d'un agent oxydant un composé de formule X,

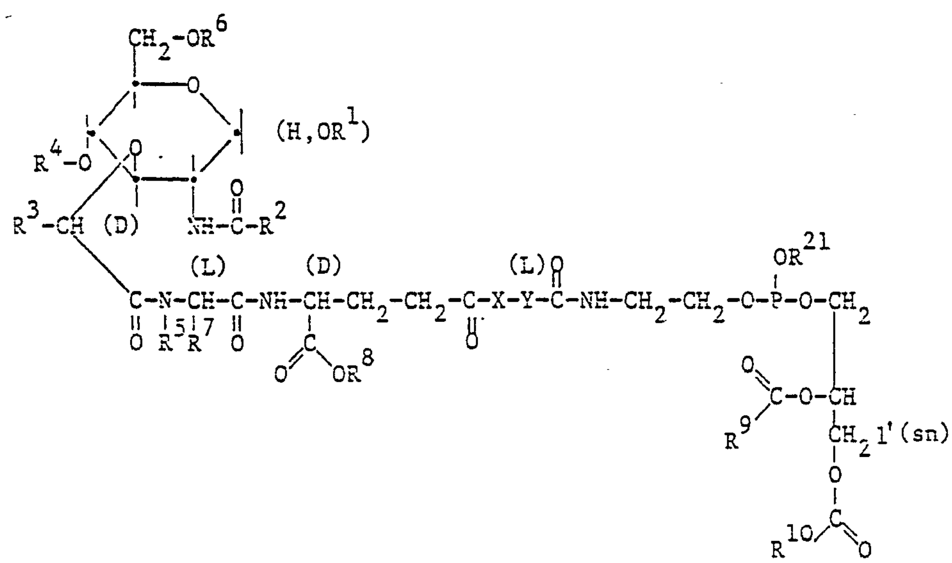

(X)

dans laquelle $R^{21}$ représente l'hydrogène ou un groupe protecteur et les autres symboles ont les significations indiquées ci-dessus, les groupes fonctionnels libres présents dans un composé de formule X, à l'exception du groupe participant à la réaction, étant éventuellement à l'état protégé, ou un tautomère de ce composé, et on élimine les groupes protecteurs présents, ou bien

h) dans un dérivé de furannose de formule XI,

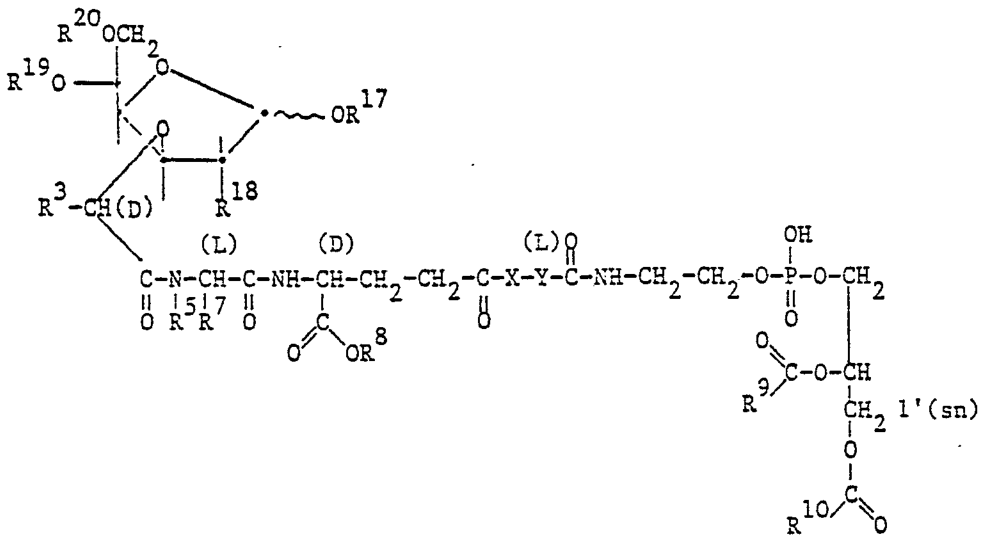

(XI)

dans laquelle $R^{17}$ représente l'hydrogène ou un groupe protecteur, facile à éliminer, du groupe hydroxy, et $R^{18}$ a les significations indiquées ci-dessus pour le reste

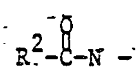

ou bien $R^{17}$ et $R^{18}$ forment ensemble un groupe protecteur bivalent de formule

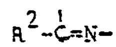

relié à l'oxygène par la valence libre de l'atome de carbone, $R^2$ ayant les significations indiquées ci-dessus, et $R^{19}$ et $R^{20}$ représentent l'hydrogène ou un groupe protecteur, facile à éliminer, du groupe hydroxy, ou bien $R^{19}$ et $R^{20}$ forment ensemble un groupe protecteur bivalent du groupe hydroxy, et les autres symboles ont les significations indiquées ci-dessus, on élimine les groupes protecteurs, ou bien

i) dans un composé de formule I dans laquelle l'un au moins des restes $R^1$, $R^2$, $R^4$, $R^6$, $R^7$ ou $R^8$ est à l'état protégé, on élimine le ou les groupes protecteurs, ou bien

j) on hydrolyse un composé de formule XII

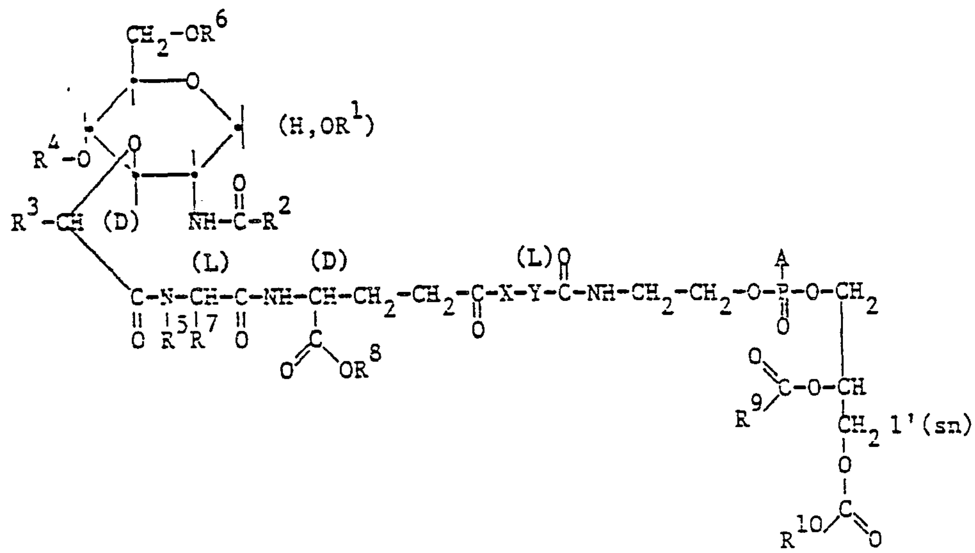

(XII)

dans laquelle A représente un atome d'halogène et les autres symboles ont les significations indiquées ci-dessus et, si on le désire, après mise en œuvre de l'un des procédés a) à j), on convertit un composé obtenu, répondant à la formule I, en un sel, ou un sel obtenu en un autre sel.

27. Les composés qu'on peut obtenir par les procédés de la rev. 26.

**Revendications** (pour l'Etat contractant AT)

1. Procédé de préparation de composés de formule I

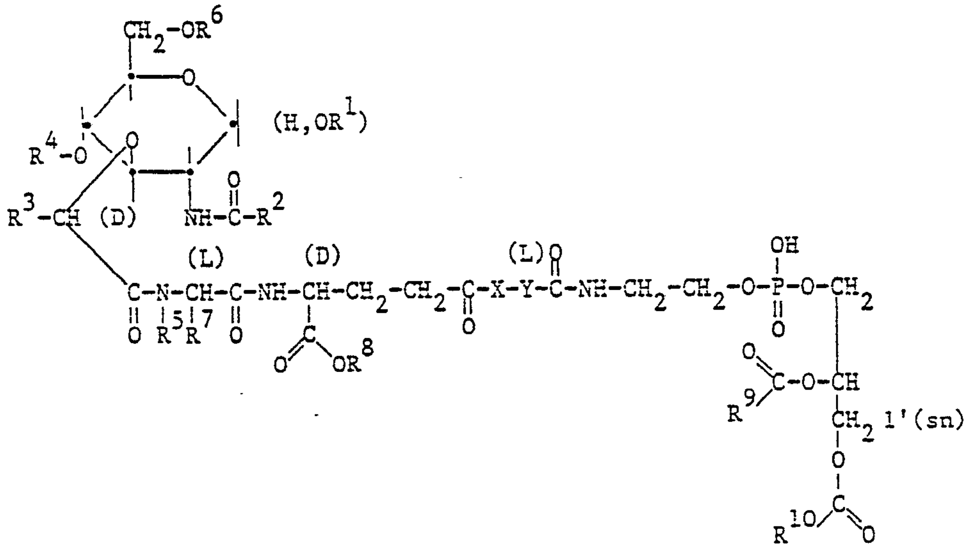

(I)

dans laquelle $R^1$, $R^4$ et $R^6$ représentent chacun, indépendamment les uns des autres, l'hydrogène ou un groupe alcanoyle inférieur, $R^2$ représente un groupe alkyle en C1-C4, hydroxyméthyle ou phényle, $R^3$ représente l'hydrogène ou un groupe méthyle, $R^5$ représente l'hydrogène ou un groupe alkyle en C1-C3, $R^7$ représente un groupe alkyle en C1-C3 non substitué ou substitué par des groupes hydroxy, mercapto ou méthylthio, $R^8$ représente l'hydrogène ou un groupe alkyle inférieur, X représente l'oxygène ou le groupe NH, Y représente un groupe alkylidène en C1-C4 et $R^9$ et $R^{10}$ représentent chacun, indépendamment l'un de l'autre, un groupe alkyle en C11-C17 ou alcényle en C11-C17, le qualificatif « inférieur » s'appliquant à des groupes contenant de 1 à 7 atomes de carbone inclus, et/ou de leurs sels, caractérisé en ce que

a) on acyle un composé de formule II

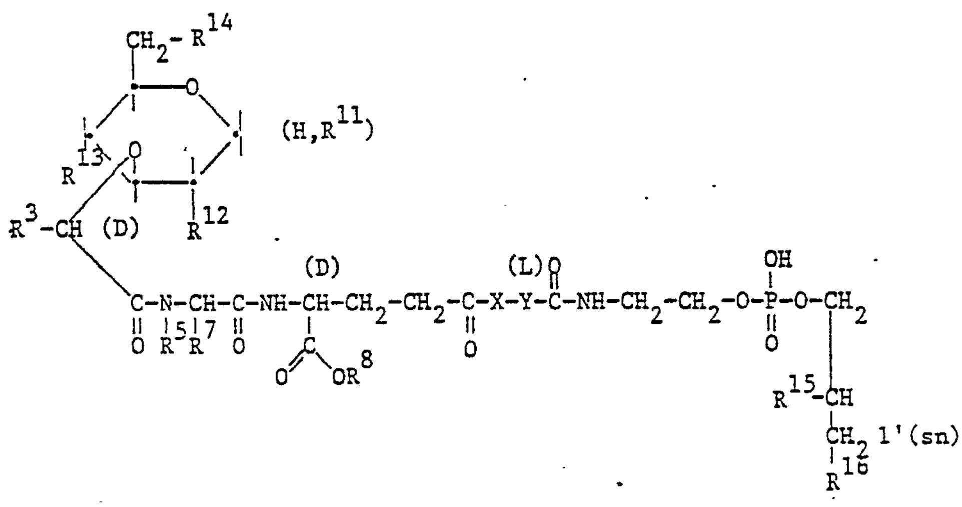

(II)

dans laquelle l'un au moins des symboles $R^{11}$, $R^{13}$, $R^{14}$, $R^{15}$ et $R^{16}$ représente un groupe hydroxy libre ou sous une forme réactive et/ou $R^{12}$ représente un groupe amino libre ou sous une forme réactive, et les autres symboles ont les significations indiquées ci-dessus, étant spécifié que les groupes fonctionnels présents dans un composé de formule II, à l'exception du/ou des groupes participant à la réaction, sont

éventuellement à l'état protégé, à l'aide d'un acide carboxylique ou d'un dérivé réactif d'un acide carboxylique, avec introduction d'au moins un groupe acyle $R^1$,

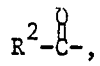

$R^4$, $R^6$,

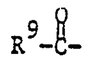

ou

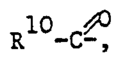

dans lequel les symboles ont les significations indiquées ci-dessus, et on élimine les groupes protecteurs éventuellement présents, ou bien

b) on fait réagir un composé de formule III,

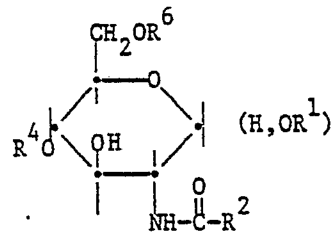

(III)

dans laquelle les symboles ont les significations indiquées ci-dessus et les groupes fonctionnels présents, à l'exception du groupe hydroxy libre en position 3, sont éventuellement à l'état protégé, le groupe 3-hydroxy étant éventuellement sous une forme réactive, avec un composé de formule IV,

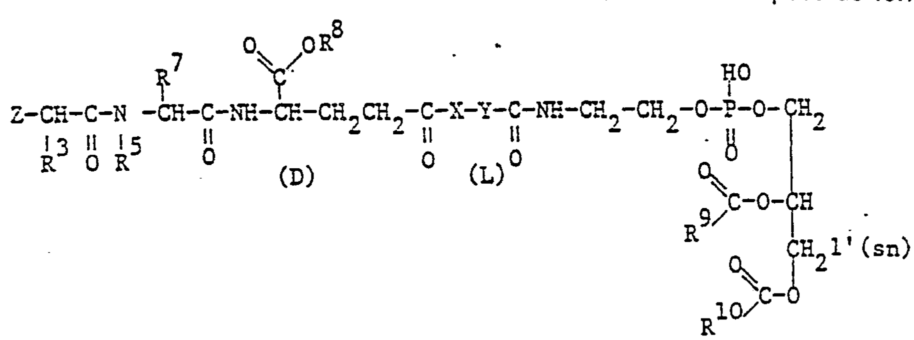

(IV)

dans laquelle Z représente un groupe hydroxy éventuellement sous une forme réactive et les autres symboles ont les significations indiquées ci-dessus, étant spécifié que les groupes fonctionnels présents dans un composé de formule IV, à l'exception de Z, sont éventuellement à l'état protégé, et on élimine les groupes protecteurs présents, ou bien

c) on fait réagir un composé de formule V,

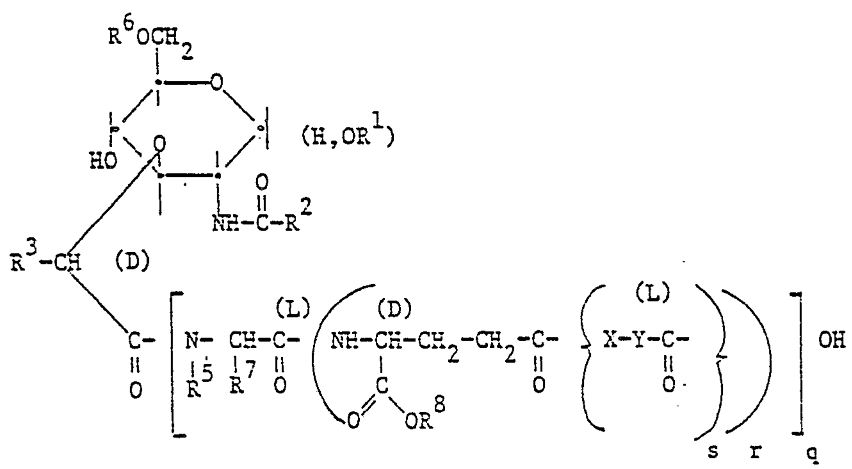

(V)

dans laquelle s, r et q sont égaux chacun, indépendamment les uns des autres, à 0 ou 1 et $R^8$ représente un groupe alkyle inférieur ou un groupe protecteur du groupe carboxyle, les autres substituants ayant les significations indiquées ci-dessus, étant spécifié que les groupes fonctionnels libres présents dans un composé de formule V, à l'exception du groupe participant à la réaction, sont éventuellement à l'état protégé, ou un dérivé d'acide carboxylique réactif d'un tel composé, avec un composé de formule VI,

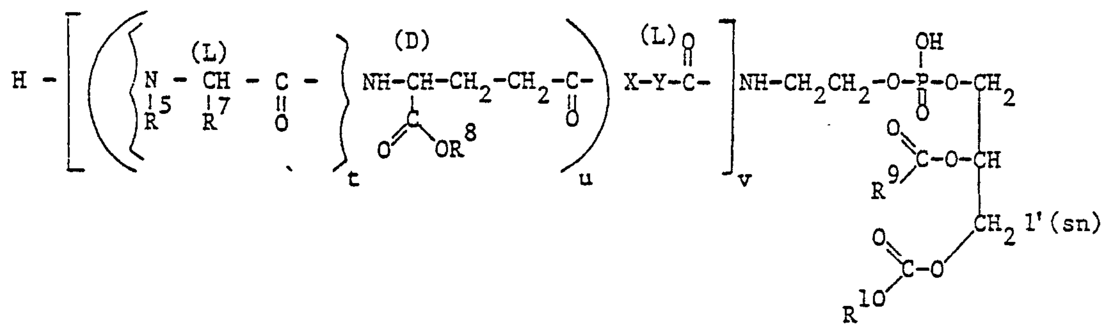

(VI)

dans laquelle t, u et v sont égaux chacun, indépendamment les uns des autres, à 0 ou 1 et les symboles ont les significations indiquées ci-dessus, étant spécifié que t, u et v sont égaux à 1 lorsque, dans l'autre composant de la réaction répondant à la formule V, q est égal à 0, ou que t est égal à 0, u à 1 et v à 1 lorsque q est égal à 1 et r à 0, ou bien que q est égal à 0 et v à 1 lorsque q est égal à 1, r à 1 et s à 0, ou que v est égal à 0 lorsque q, r et s sont égaux à 1, ou avec un dérivé réactif d'un tel composé, et on élimine les groupes protecteurs éventuellement présents, ou bien

d) on fait réagir un composé de formule VII,

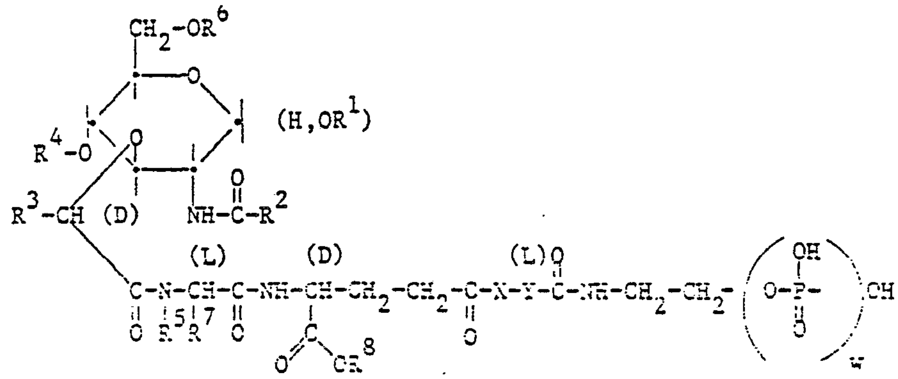

(VII)

dans laquelle w est égal à 0 ou 1 et les symboles ont les significations indiquées ci-dessus, étant spécifié que les groupes fonctionnels libres, à l'exception du groupe participant à la réaction, sont éventuellement à l'état protégé, ou un dérivé phosphorique réactif d'un tel acide de formule VII dans laquelle w est égal à 1, avec un composé de formule VIII,

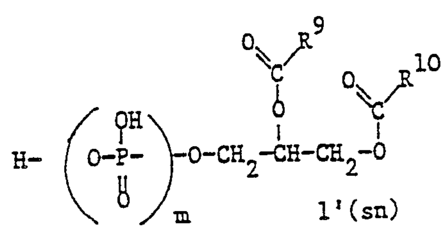

(VIII)

dans laquelle les symboles ont les significations indiquées ci-dessus et m est égal à 0 ou 1, étant spécifié que m est égal à 1 lorsque, dans l'autre composant de la réaction répondant à la formule VII, w est égal à 0, ou que m est égal à 0 lorsque w est égal à 1, ou avec un dérivé phosphorique réactif d'un acide de formule VIII dans laquelle m est égal à 1, et on élimine les groupes protecteurs présents, ou bien

e) on fait réagir le dérivé réactif d'un composé de formule VII,

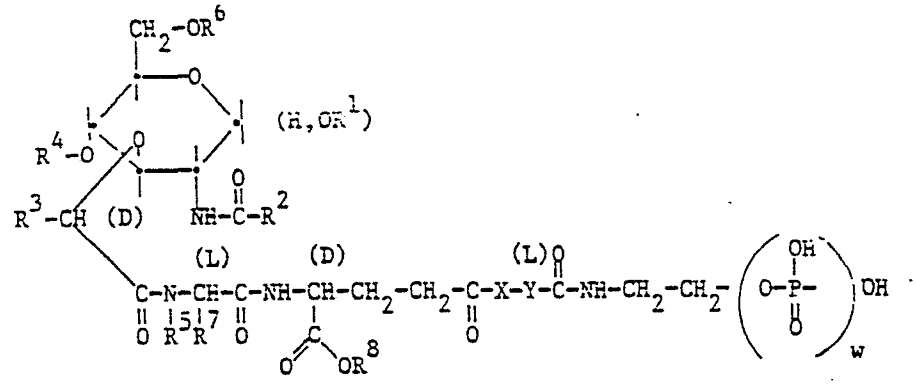

(VII)

dans laquelle w est égal à 0 ou 1 et les symboles ont les significations indiquées ci-dessus, étant spécifié que les groupes fonctionnels libres présents dans un composé de formule VII, à l'exception du groupe participant à la réaction, sont éventuellement protégés par des groupes protecteurs faciles à éliminer, et également que le dérivé réactif d'un composé de formule VII dans laquelle w est égal à 0 est un dérivé portant un groupe hydroxy terminal estérifié réactif et également que le dérivé réactif d'un composé de formule VII dans laquelle w est égal à 1 est un sel, avec le dérivé réactif d'un composé de formule VIII,

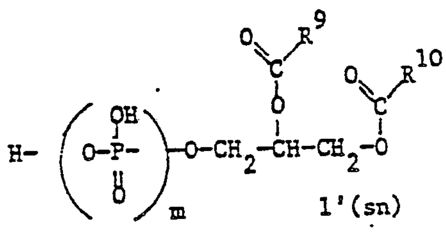

(VIII)

dans laquelle les symboles ont les significations indiquées ci-dessus et m est égal à 0 ou 1, étant spécifié que m est égal à 1 lorsque, dans le composant de réaction répondant à la formule VII, w est égal à 0, le dérivé réactif d'un composé de formule VIII étant alors un sel, ou que m est égal à 0 lorsque w est égal à 1, le dérivé réactif d'un composé de formule VIII étant alors un dérivé portant un groupe hydroxy terminal estérifié réactif, et on élimine les groupes protecteurs présents, ou bien

f) on fait réagir un composé de formule IX,

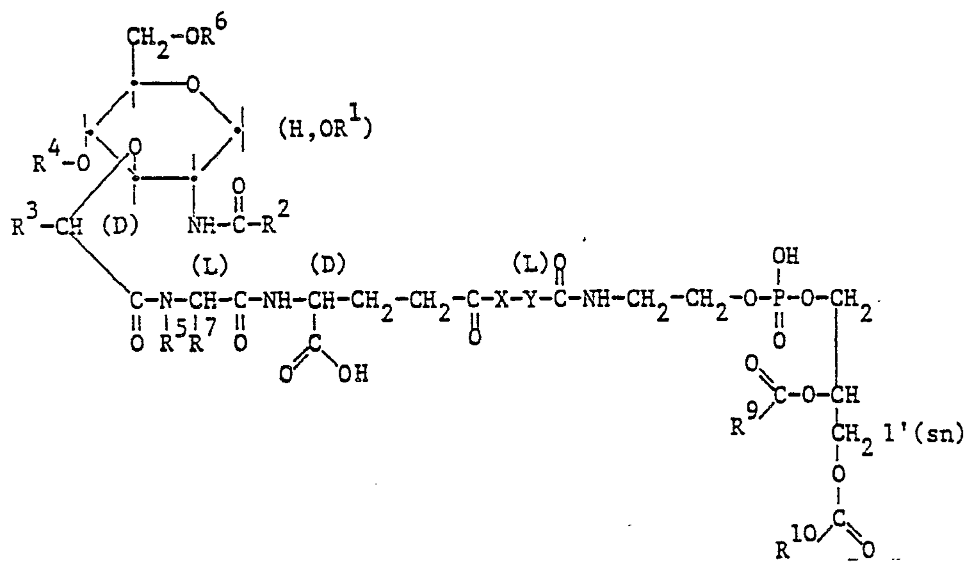

(IX)

dans laquelle les symboles ont les significations indiquées ci-dessus, les groupes fonctionnels libres présents dans un composé de formule IX, à l'exception du groupe participant à la réaction, étant éventuellement à l'état protégé, ou un dérivé d'acide carboxylique réactif d'un composé de formule IX, avec un alcanol inférieur $R^8$—OH dans lequel $R^8$ représente un groupe alkyle inférieur, ou un dérivé

réactif d'un tel alcanol, et on élimine les groupes protecteurs présents, ou bien

g) on oxyde un composé de formule X

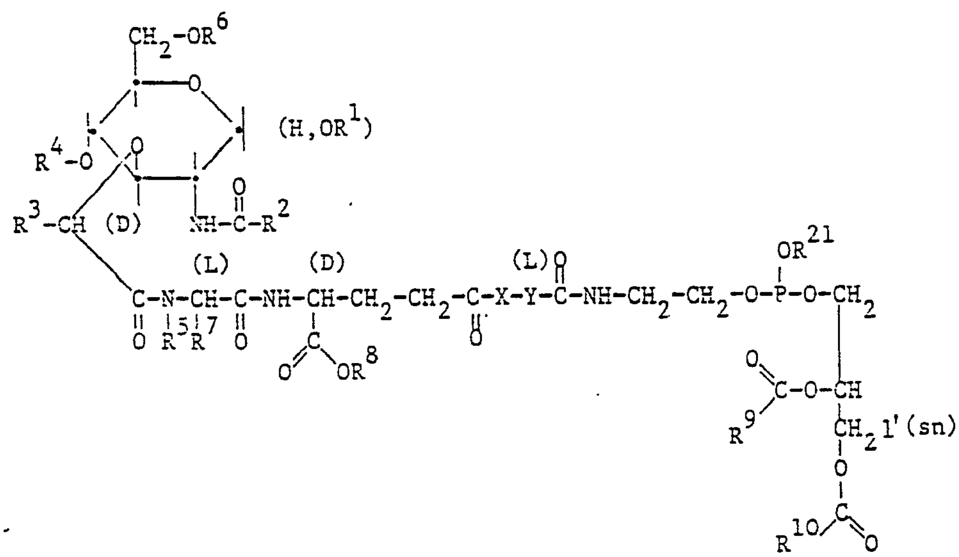

(X)

dans laquelle $R^{21}$ représente l'hydrogène ou un groupe protecteur et les autres symboles ont les significations indiquées ci-dessus, les groupes fonctionnels libres présents dans un composé de formule X, à l'exception du groupe participant à la réaction, étant éventuellement à l'état protégé, ou un tautomère de ce composé, à l'aide d'un agent oxydant, et on élimine les groupes protecteurs présents, ou bien

h) dans un dérivé de furannose de formule XI,

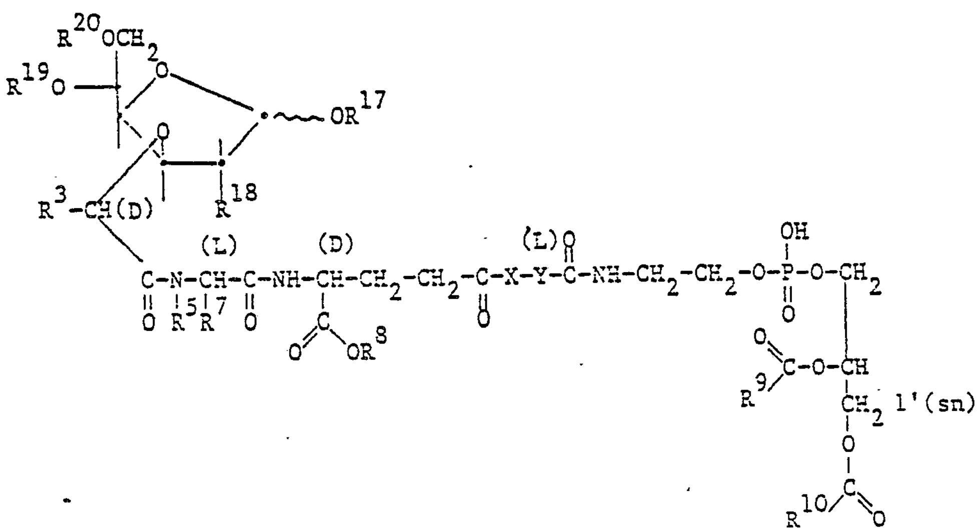

(XI)

dans laquelle $R^{17}$ représente l'hydrogène ou un groupe protecteur facile à éliminer du groupe hydroxy et $R^{18}$ a les significations indiquées ci-dessus pour le reste

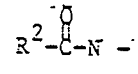

ou bien $R^{17}$ et $R^{18}$ forment ensemble un groupe protecteur bivalent de formule

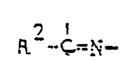

relié à l'oxygène par la valence libre de l'atome de carbone, $R^2$ ayant les significations indiquées ci-dessus, et $R^{19}$ et $R^{20}$ représentent l'hydrogène ou un groupe protecteur facile à éliminer du groupe hydroxy, ou bien $R^{19}$ et $R^{20}$ forment ensemble un groupe protecteur bivalent du groupe hydroxy, et les

autres substituants ont les significations indiquées ci-dessus, on élimine les groupes protecteurs, ou bien

i) dans un composé de formule I dans laquelle l'un au moins des restes $R^1$, $R^2$, $R^4$, $R^6$, $R^7$ ou $R^8$ est à l'état protégé, on élimine le ou les groupes protecteurs, ou bien

j) on hydrolyse un composé de formule XII,

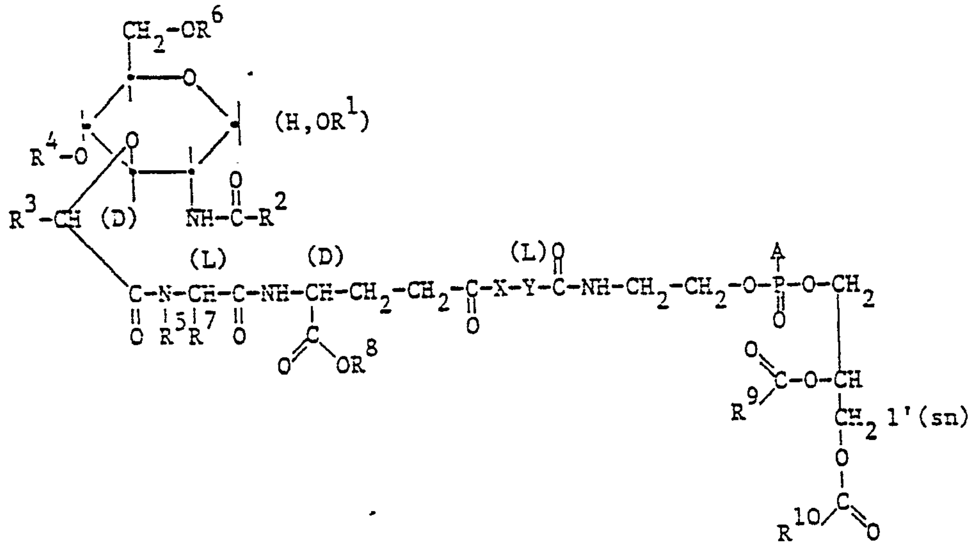

(XII)

dans laquelle A représente un atome d'halogène et les autres symboles ont les significations indiquées ci-dessus et, si on le désire, après mise en œuvre de l'un des procédés a) à j), on convertit un composé obtenu répondant à la formule I en un sel ou un sel obtenu en un autre sel.

2. Procédé selon la revendication 1, caractérisé en ce que l'on choisit les composants de départ de manière à obtenir des composés de formule I dans laquelle $R^1$, $R^4$ et $R^6$ représentent chacun, indépendamment les uns des autres, l'hydrogène ou un groupe alcanoyle en C1-C3, $R^2$ représente un groupe alkyle en C1-C2 ou hydroxyméthyle, $R^3$ représente l'hydrogène ou un groupe méthyle, $R^5$ représente l'hydrogène ou un groupe méthyle, $R^7$ représente un groupe alkyle en C1-C3 ou hydroxyméthyle, $R^8$ représente l'hydrogène ou un groupe alkyle en C1-C4, X représente l'oxygène ou le groupe NH, Y représente un groupe alkylidène en C1-C3 et $R^9$ et $R^{10}$ représentent chacun, indépendamment l'un de l'autre, un groupe alkyle en C11-C17 à chaîne droite ou le groupe descarbonyl-oléoyle et/ou leurs sels.

3. Procédé selon la revendication 2, caractérisé en ce que l'on choisit les composants de départ de manière à obtenir des composés de formule I dans laquelle $R^1$, $R^4$ et $R^6$ représentent chacun, indépendamment les uns des autres, l'hydrogène ou un groupe acétyle, $R^2$ représente un groupe méthyle ou éthyle, $R^3$ représente l'hydrogène ou un groupe méthyle, $R^5$ représente l'hydrogène ou un groupe méthyle, $R^7$ représente un groupe alkyle en C1-C3, $R^8$ représente l'hydrogène ou un groupe alkyle en C1-C4, X représente l'oxygène ou le groupe NH, Y représente un groupe alkylidène en C1-C2 et $R^9$ et $R^{10}$ représentent chacun, indépendamment l'un de l'autre, un groupe alkyle à chaîne droite en C11-C17 à nombre impair d'atomes de carbone ou un groupe descarbonbyl-oléoyle, et/ou leurs sels.

4. Procédé selon l'une des revendications 1 à 3, caractérisé en ce que l'on choisit les composants de départ de manière à obtenir des composés de formule I dans laquelle $R^8$ représente le groupe alkyle mentionné dans ces revendications, et/ou leurs sels.

5. Procédé selon l'une des revendications 1 à 4, caractérisé en ce que l'on choisit les composants de départ de manière à obtenir des composés de formule I dans laquelle l'un au moins des symboles $R^1$, $R^4$ et $R^6$ représente un groupe alcanoyle inférieur, et/ou leurs sels.

6. Procédé selon l'une des revendications 1 à 4, caractérisé en ce que l'on choisit les composants de départ de manière à obtenir des composés de formule I dans laquelle $R^1$, $R^4$ et $R^6$ représentent l'hydrogène, et/ou leurs sels.

7. Procédé selon la revendication 3, caractérisé en ce que l'on choisit les composants de départ de manière à obtenir des composés de formule I dans laquelle $R^1$, $R^4$ et $R^6$ représentent l'hydrogène, $R^2$ représente un groupe méthyle ou éthyle, $R^3$ représente l'hydrogène lorsque $R^2$ représente un groupe éthyle, ou un groupe méthyle lorsque $R^2$ représente un groupe méthyle, $R^5$ représente l'hydrogène ou un groupe méthyle, $R^7$ représente un groupe alkyle en C1-C2, $R^8$ représente un groupe alkyle à chaîne droite en C1-C4, X représente l'oxygène ou le groupe NH, Y représente un groupe éthylidène et $R^9$ et $R^{10}$ représentent chacun, indépendamment l'un de l'autre, un groupe alkyle à chaîne droite en C15-C17 à nombre impair d'atomes de carbone, et/ou leurs sels.

8. Procédé selon l'une des revendications 1 à 7, caractérisé en ce que l'on choisit les composants de départ de manière à obtenir des composés de formule I dans laquelle $R^3$ représente un groupe méthyle, et/ou leurs sels.

9. Procédé selon l'une des revendications 1 à 8, caractérisé en ce que l'on choisit les composés de départ de manière à obtenir des composés de formule I dans laquelle $R^5$ représente l'hydrogène, et/ou leurs sels.

10. Procédé selon l'une des revendications 1 à 9, caractérisé en ce que l'on choisit les composants de départ de manière à obtenir des composés de formule I dans laquelle $R^8$ représente le groupe n-butyle, et/ou leurs sels.

11. Procédé selon l'une des revendications 1 à 10, caractérisé en ce que l'on choisit les composants de départ de manière à obtenir des composés de formule I dans laquelle X représente le groupe NH, et/ou leurs sels.

12. Procédé selon l'une des revendications 1 à 11, caractérisé en ce que l'on choisit les composants de départ de manière à obtenir des composés de formule I dans laquelle $R^9$ et $R^{10}$ représentent des groupes n-pentadécyle, et/ou leurs sels.

13. Procédé selon la revendication 1, caractérisé en ce que l'on choisit les composants de départ de manière à obtenir le N-acétylmuramyl-L-alanyl-D-glutamyl-($C_\alpha$)-(ester n-butylique)-($C_\gamma$)-L-alanine-2-(1,2-dipalmitoyl-sn-glycéro-3-hydroxyphosphoryloxy)-éthylamide, et/ou un sel de ce composé.

14. Procédé selon la revendication 1, caractérisé en ce que l'on choisit les composants de départ de manière à obtenir le N-propionyl-desméthylmuramyl-L-alanyl-D-glutamyl-($C_\alpha$)-(ester n-butylique)-($C_\gamma$)-L-alanine-2-(1,2-dipalmitoyl-sn-glycéro-3-hydroxyphosphoryloxy)-éthylamide, et/ou un sel de ce composé.

15. Procédé selon la revendication 1, caractérisé en ce que l'on choisit les composants de départ de manière à obtenir le N-acétylmuramyl-L-alanyl-D-glutamyl-($C_\alpha$)-(ester méthylique)-($C_\gamma$)-L-alanine-2-(1,2-dipalmitoyl-sn-glycéro-3-hydroxyphosphoryloxy)-éthylamide, et/ou un sel de ce composé.

16. Procédé selon la revendication 1, caractérisé en ce que l'on choisit les composants de départ de manière à obtenir le N-propionyl-desméthylmuramyl-L-alanyl-D-glutamyl)-($C_\alpha$)-(ester méthylique)-($C_\gamma$)-L-alanine-2-(1,2-dipalmitoyl-sn-glycéro-3-hydroxyphosphoryloxy)-éthylamide, et/ou un sel de ce composé.

17. Procédé selon la revendication 1, caractérisé en ce que l'on choisit les composants de départ de manière à obtenir le ($C_\gamma$)-L-alanine-2-(1,2-dipalmitoyl-sn-glycéro-3-hydroxyphosphoryloxy)-éthylamide de l'acide N-acétylmuramyl-L-alanyl-D-glutamique, et/ou un sel de ce composé.

18. Procédé selon l'une des revendications 1 à 17, caractérisé en ce que l'on choisit les composants de départ de manière à obtenir des sels acceptables pour l'usage pharmaceutique.

19. Procédé selon la revendication 18, caractérisé en ce que l'on choisit les composants de départ de manière à obtenir des sels de sodium.